# EUROPEAN PATENT APPLICATION

(11) **EP 2 818 867 A1**
(43) Date of publication of application: **31.12.2014**
(21) Application number: 13305900.6
(22) Date of filing: 27.06.2013
(51) Int. Cl.: G01N 33/532, A61K 39/00, G01N 33/58

(54) **Antibodies conjugated to at least one nucleic acid molecule and their use in multiplex immuno-detection assays**

(71) Applicant: INSERM (Institut National de la Santé et de la Recherche Médicale), 75013 Paris (FR); Université d'Aix-Marseille, 13284 Marseille Cedex 07 (FR); Centre National de la Recherche Scientifique (CNRS), 75016 Paris (FR); Innate Pharma, 13009 Marseille (FR)
(72) Inventor: Vivier, Eric, 13288 Marseille Cedex 09 (FR); Imbert, Jean, 13009 Marseille (FR); Ugolini, Sophie, 13288 Marseille Cedex 09 (FR); Romagne, François, 13009 Marseille (FR); Bregeon, Delphine, 13009 Marseille (FR); Belmant, Christian, 13009 Marseille (FR)
(74) Representative: Hirsch, Denise Marie

(57) **Abstract**

The present invention relates to antibodies conjugated to at least one nucleic acid molecule and their use in multiplex immuno-detection assays. In particular, the present invention relates to an antibody conjugated to at least one nucleic acid molecule comprising an enzymatic cleavable sequence (CL) and a DNA barcode sequence N1N2N3N4Nn wherein N represents a nucleotide and n an integer number superior to 4.

## Description

### FIELD OF THE INVENTION:

The present invention relates to antibodies conjugated to at least one nucleic acid molecule and their use in multiplex immuno-detection assays.

### BACKGROUND OF THE INVENTION:

The detection of antigen is important for both molecular biology research and medical applications. Antibody-based reactions are widely used to diagnose diseases. For example enzyme-linked immunosorbent assay (ELISA), Western blotting, and indirect fluorescent antibody tests are extremely useful for identifying single target proteins. Rapid and simultaneous sample screening for the presence of multiple antibodies would be beneficial in both research and clinical applications. However, it is difficult, expensive, and time-consuming to simultaneously detect several protein structures under assay conditions using the aforementioned related protocols. Polymerase chain reaction (PCR) and other forms of target amplification have enabled rapid advances in the development of powerful tools for detecting and quantifying DNA targets of interest. The development of comparable target amplification methods for proteins could dramatically improve methods for detecting and quantifying antigens.

### SUMMARY OF THE INVENTION:

The present invention relates to antibodies conjugated to at least one nucleic acid molecule and their use in multiplex immuno-detection assays.

### DETAILED DESCRIPTION OF THE INVENTION:

The present invention relates to specific antibodies (named DNAbs) that utilize DNA barcodes for detecting multiple antigens in a sample. The invention takes advantage of recognition capabilities of antibodies and relies on the general idea that each of said antibodyantibodies can be associated with a different oligonucleotide DNA barcode allowing to reveal and quantify a plurality of antigens in a multiplex immuno-detection assay.

Accordingly an object of the present invention relates to an antibody conjugated to at least one nucleic acid molecule comprising an enzymatic cleavable sequence (CL) and a DNA barcode sequence N₁N₂N₃N₄Nₙ wherein N represents a nucleotide and n an integer number superior to 4.

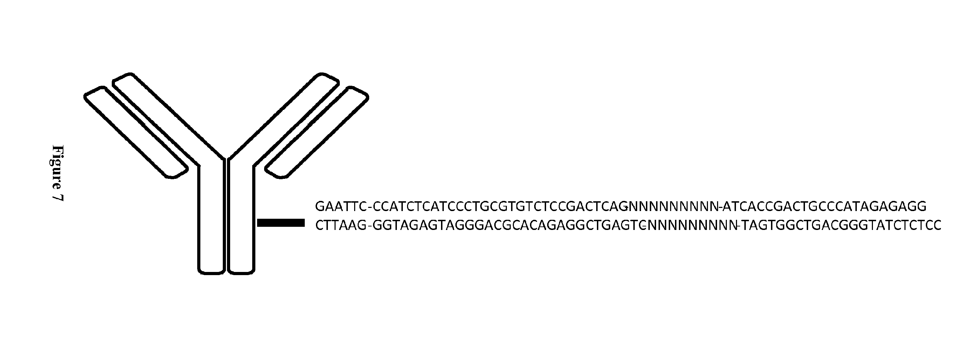

According to the present invention such a conjugated antibody is named "DNAb". A typical DNAb according to the invention is depicted in figure 7.

The term "antibody" is thus used to refer to any antibody-like molecule that has an antigen binding region, and this term includes antibody fragments that comprise an antigen binding domain such as Fab', Fab, F(ab')2, single domain antibodies (DABs or VHH), TandAbs dimer, Fv, scFv (single chain Fv), dsFv, ds-scFv, Fd, linear antibodies, minibodies, diabodies, bispecific antibody fragments, bibody, tribody (scFv-Fab fusions, bispecific or trispecific, respectively); sc-diabody; kappa(lamda) bodies (scFv-CL fusions); DVD-Ig (dual variable domain antibody, bispecific format); SIP (small immunoprotein, a kind of minibody); SMIP ("small modular immunopharmaceutical" scFv-Fc dimer; DART (ds-stabilized diabody "Dual Affmity ReTargeting"); small antibody mimetics comprising one or more CDRs and the like. The techniques for preparing and using various antibody-based constructs and fragments are well known in the art.

In one embodiment, the antibody is a monoclonal antibody. Monoclonal antibodies may be generated using the method of Kohler and Milstein (Nature, 256:495, 1975). To prepare monoclonal antibodies useful in the invention, a mouse or other appropriate host animal is immunized at suitable intervals (e.g., twice-weekly, weekly, twice-monthly or monthly) with the antigenic form of interest. The animal may be administered a final "boost" of antigen within one week of sacrifice. It is often desirable to use an immunologic adjuvant during immunization. Suitable immunologic adjuvants include Freund's complete adjuvant, Freund's incomplete adjuvant, alum, Ribi adjuvant, Hunter's Titermax, saponin adjuvants such as QS21 or Quil A, or CpG-containing immunostimulatory oligonucleotides. Other suitable adjuvants are well-known in the field. The animals may be immunized by subcutaneous, intraperitoneal, intramuscular, intravenous, intranasal or other routes. A given animal may be immunized with multiple forms of the antigen by multiple routes. Briefly, the recombinant antigen of interest may be provided by expression with recombinant cell lines. Antigen of interest may be provided in the form of human cells expressing antigen of interest at their surface. Recombinant forms of antigen of interest may be provided using any previously described method. Following the immunization regimen, lymphocytes are isolated from the spleen, lymph node or other organ of the animal and fused with a suitable myeloma cell line using an agent such as polyethylene glycol to form a hydridoma. Following fusion, cells are placed in media permissive for growth of hybridomas but not the fusion partners using standard methods, as described (Coding, Monoclonal Antibodies: Principles and Practice: Production and Application of Monoclonal Antibodies in Cell Biology, Biochemistry and Immunology, 3rd edition, Academic Press, New York, 1996). Following culture of the hybridomas, cell supernatants are analyzed for the presence of antibodies of the desired specificity, i.e., that selectively bind the antigen. Suitable analytical techniques include ELISA, flow cytometry, immunoprecipitation, and western blotting. Other screening techniques are well-known in the field. Preferred techniques are those that confirm binding of antibodies to conformationally intact, natively folded antigen, such as non-denaturing ELISA, flow cytometry, and immunoprecipitation.

Significantly, as is well-known in the art, only a small portion of an antibody molecule, the paratope, is involved in the binding of the antibody to its epitope (see, in general, Clark, W. R. (1986) The Experimental Foundations of Modern Immunology Wiley & Sons, Inc., New York; Roitt, I. (1991) Essential Immunology, 7th Ed., Blackwell Scientific Publications, Oxford). The Fc' and Fc regions, for example, are effectors of the complement cascade but are not involved in antigen binding. An antibody from which the pFc' region has been enzymatically cleaved, or which has been produced without the pFc' region, designated an F(ab')2 fragment, retains both of the antigen binding sites of an intact antibody. Similarly, an antibody from which the Fc region has been enzymatically cleaved, or which has been produced without the Fc region, designated an Fab fragment, retains one of the antigen binding sites of an intact antibody molecule. Proceeding further, Fab fragments consist of a covalently bound antibody light chain and a portion of the antibody heavy chain denoted Fd. The Fd fragments are the major determinant of antibody specificity (a single Fd fragment may be associated with up to ten different light chains without altering antibody specificity) and Fd fragments retain epitope-binding ability in isolation.

Thus, as will be apparent to one of ordinary skill in the art, the present invention also provides for F(ab') 2 Fab, Fv and Fd fragments. Antibodies can be indeed fragmented using conventional techniques. For example, F(ab')2 fragments can be generated by treating the antibody with pepsin. The resulting F(ab')2 fragment can be treated to reduce disulfide bridges to produce Fab' fragments. Papain digestion can lead to the formation of Fab fragments. Fab, Fab' and F(ab')2, scFv, Fv, dsFv, Fd, dAbs, TandAbs, ds-scFv, dimers, minibodies, diabodies, bispecific antibody fragments and other fragments can also be synthesized by recombinant techniques or can be chemically synthesized. Techniques for producing antibody fragments are well known and described in the art. For example, each of Beckman et al., 2006; Holliger & Hudson, 2005; Le Gall et al., 2004; Reff & Heard, 2001 ; Reiter et al., 1996; and Young et al., 1995 further describe and enable the production of effective antibody fragments. The various antibody molecules and fragments may derive from any of the commonly known immunoglobulin classes, including but not limited to IgA, secretory IgA, IgE, IgG and IgM. IgG subclasses are also well known to those in the art and include but are not limited to human IgG1, IgG2, IgG3 and IgG4.

The present invention also includes so-called single chain antibodies. The term "single domain antibody" (sdAb) or "VHH" refers to the single heavy chain variable domain of antibodies of the type that can be found in Camelid mammals which are naturally devoid of light chains. Such VHH are also called "nanobody®". According to the invention, sdAb can particularly be llama sdAb.

Typically, the antibody may be directed against any antigen. The antigen refers to the compound any macromolecule but is typically a polypeptide. The antigen can be a part of a cell such as a cell bearing the antigen, or a microorganism e.g., bacterium, fungus, protozoan, or virus. A wide variety of proteins may be considered as antigens. Such proteins include, for example, immunoglobulins, cytokines, enzymes, hormones, cancer antigens, nutritional markers, tissue specific antigens, etc.

In particular, the antibody may be specific for an immune cell regulatory molecule such as CD3, CD4, CD8, CD25, CD28, CD26, CTLA-4, ICOS, or CD11a. Other suitable antigens include but are not limited to those associated with immune cells including T cell-associated molecules, such as TCR/CD3 or CD2; NK cell-associated targets such as NKG2D, FcγRIIIa (CD16), CD38, CD44, CD56, or CD69; granulocyte-associated targets such as FcγRI (CD64), FcαRI (CD89), and CR3 (CD11b/CD18); monocyte/macrophage-associated targets (such as FcγRI (CD64), FcαRI (CD89), CD3 (CD11b/CD18), or mannose receptor; dendritic cell-associated targets such as FcγRI (CD64) or mannose receptor; and erythrocyte-associated targets such as CRI (CD35).

Alternatively, the antibody according to the invention may be directed against a cancer antigen. Known cancer antigens include, without limitation, c-erbB-2 (erbB-2 is also known as c-neu or HER-2), which is particularly associated with breast, ovarian, and colon tumor cells, as well as neuroblastoma, lung cancer, thyroid cancer, pancreatic cancer, prostate cancer, renal cancer and cancers of the digestive tract. Another class of cancer antigens is oncofetal proteins of nonenzymatic function. These antigens are found in a variety of neoplasms, and are often referred to as "tumor-associated antigens." Carcinoembryonic antigen (CEA), and α-fetoprotein (AFP) are two examples of such cancer antigens. AFP levels rise in patients with hepatocellular carcinoma: 69% of patients with liver cancer express high levels of AFP in their serum. CEA is a serum glycoprotein of 200 kDa found in adenocarcinoma of colon, as well as cancers of the lung and genitourinary tract. Yet another class of cancer antigens is those antigens unique to a particular tumor, referred to sometimes as "tumor specific antigens," such as heat shock proteins (e.g., hsp70 or hsp90 proteins) from a particular type of tumor. Other targets include the MICA/B ligands of NKG2D. These molecules are expressed on many types of tumors, but not normally on healthy cells.

Additional specific examples of cancer antigens include epithelial cell adhesion molecule (Ep-CAM/TACSTD1), mesothelin, tumor-associated glycoprotein 72 (TAG-72), gp100, Melan-A, MART-1, KDR, RCAS1, MDA7, cancer-associated viral vaccines (e.g., human papillomavirus antigens), prostate specific antigen (PSA, PSMA), RAGE (renal antigen), CAMEL (CTL-recognized antigen on melanoma), CT antigens (such as MAGE-B5, -B6, -C2, -C3, and D; Mage-12; CT10; NY-ESO-1, SSX-2, GAGE, BAGE, MAGE, and SAGE), mucin antigens (e.g., MUC1, mucin-CA125, etc.), cancer-associated ganglioside antigens, tyrosinase, gp75, C-myc, Mart1, MelanA, MUM-1, MUM-2, MUM-3, HLA-B7, Ep-CAM, tumor-derived heat shock proteins, and the like (see also, e.g., Acres et al., Curr Opin Mol Ther 2004 February, 6:40-7; Taylor-Papadimitriou et al., Biochim Biophys Acta. 1999 Oct. 8; 1455(2-3):301-13; Emens et al., Cancer Biol Ther. 2003 July-August; 2(4 Suppl 1):S161-8; and Ohshima et al., Int J Cancer. 2001 Jul. 1; 93(1):91-6). Other exemplary cancer antigen targets include CA 195 tumor-associated antigen-like antigen (see, e.g., U.S. Pat. No. 5,324,822) and female urine squamous cell carcinoma-like antigens (see, e.g., U.S. Pat. No. 5,306,811), and the breast cell cancer antigens described in U.S. Pat. No. 4,960,716.

The antibody according to the invention may target protein antigens, carbohydrate antigens, or glycosylated proteins. For example, the antibody can target glycosylation groups of antigens that are preferentially produced by transformed (neoplastic or cancerous) cells, infected cells, and the like (cells associated with other immune system-related disorders). In one aspect, the antigen is a tumor-associated antigen. In an exemplary aspect, the antigen is O-acetylated-GD2 or glypican-3. In another particular aspect, the antigen is one of the Thomsen-Friedenreich (TF) antigens (TFAs).

The antibody according to the invention can also exhibit specificity for a cancer-associated protein. Such proteins can include any protein associated with cancer progression. Examples of such proteins include angiogenesis factors associated with tumor growth, such as vascular endothelial growth factors (VEGFs), fibroblast growth factors (FGFs), tissue factor (TF), epidermal growth factors (EGFs), and receptors thereof; factors associated with tumor invasiveness; and other receptors associated with cancer progression (e.g., one of the HER1-HER4 receptors).

Alternatively the antibody according to the invention can be specific for a virus, a bacteria or parasite associated target. For example, the antibody may be specific for a virus-associated target such as an HIV protein (e.g., gp120 or gp41), CMV or other viruses, such as hepatitis C virus (HCV).

In one embodiment, the antibody is able to discriminate a protein (e.g; a surface receptor) in its active form or in its inactive form. Thus use of such antibody may be particularly relevant for determining whether said protein (e.g. a receptor) is activated or not. Thus the DNAb may be used a sensor and may find various application for screening drugs (agonists or antagonists, allosteric or not) or for screening patients eligible or not for a treatment.

In one embodiment, the antibody is specific for a CD (cluster of differentiation) molecule. Examples of CD molecules are listed in Table A.

**Table A: list of CD molecules**

| **CD molecule** | **Alternate name** |
|---|---|
| CD1a | R4; HTA1 |
| CD1b | R1 |
| CD1c | M241; R7 |
| CD1d | R3 |
| CD1e | R2 |
| CD2 | CD2R; E-rosette receptor; T11; LFA-2 |
| CD3delta | CD3d |
| CD3epsilon | CD3e |
| CD3gamma | CD3g |
| CD4 | L3T4; W3/25 |
| CD5 | Leu-1; Ly-1; T1; Tp67 |
| CD6 | T12 |
| CD7 | gp40 |
| CD8alpha | Leu2; Lyt2; T cell co-receptor; T8 |
| CD8beta | Leu2; CD8; Lyt3 |
| CD9 | DRAP-27; MRP-1; p24 |
| CD10 | EC 3.4.24.11; neprilysin; CALLA; enkephalinase; gp100; NEP |
| CD11a | AlphaL integrin chain; LFA-1alpha |
| CD11b | AlphaM integrin chain; AlphaM-beta2; C3biR; CR3; Mac-1; Mo 1 |
| CD11c | AlphaX integrin chain; Axb2; CR4; leukocyte surface antigen p150,95 |
| CDw12 | p90-120 |
| CD13 | APN; EC 3.4.11.2; gp150 |
| CD14 | LPS-R |
| CD15u | Sulphated CD15 |
| CD16a | FCRIIIA |
| CD16b | FCRIIIB |
| CDw17 | LacCer |
| CD18 | CD11a beta subunit; CD11b beta subunit; CD11c beta subunit; beta-2 integrin chain |
| CD19 | B4 |
| CD20 | B1; Bp35 |
| CD21 | C3d receptor; CR2; EBV-R |
| CD22 | BL-CAM; Lyb8 |
| CD23 | B6; BLAST-2; FceRII; Leu-20; Low affinity IgE receptor |
| CD24 | BA-1; HSA |
| CD25 | IL-2R alpha chain; IL-2R; Tac antigen |
| CD26 | EC 3.4.14.5; ADA-binding protein; DPP IV ectoenzyme |
| CD27 | S152; T14 |
| CD28 | T44;Tp44 |
| CD29 | Platelet GPIIa; VLA-beta chain; beta-1 integrin chain |
| CD30 | Ber-H2 antigen; Ki-1 antigen |
| CD31 | GPiia'; endocam; PECAM-1 |
| CD32 | FCR II; Fc gamma RII |
| CD33 | gp67;p67 |
| CD34 | gp105-120 |
| CD35 | C3bR; C4bR; CR1; Immune Adherence Receptor |
| CD36 | GPIIIb; GPIV; OKM5-antigen; PASIV |
| CD37 | gp52-40 |
| CD38 | T10; cyclic ADP-ribose hydrolase |
| CD39 | |
| CD40 | Bp50 |
| CD41 | GPIIb; alpha IIb integrin chain |
| CD42a | GPIX |
| CD42b | GPIbalpha; Glycocalicin |
| CD42c | GPIb-beta |
| CD42d | GPV |
| CD43 | gpL115; leukocyte sialoglycoprotein; leukosialin; sialophorin |
| CD44 | ECMR III; H-CAM; HUTCH-1; Hermes; Lu, In-related; Pgp-1; gp85 |
| CD44R | CD44v; CD44v9 |
| CD45 | B220; CD45R; CD45RA; CD45RB; CD45RC; CD45RO; EC 3.1.3.4; LCA; T200; Ly5 |
| CD46 | MCP |
| CD47R | Rh-associated protein; gp42; IAP; neurophilin; OA3; MEM-133; formerly CDw149 |
| CD48 | BCM1; Blast-1; Hu Lym3; OX-45 |
| CD49a | Alpha-1 integrin chain; VLA-1 alpha chain |
| CD49b | Alpha-2 integrin chain; GPIa; VLA-2 alpha chain |
| CD49c | Alpha-3 integrin chain; VLA-3 alpha chain |
| CD49d | Alpha-4 integrin chain; VLA-4 alpha chain |
| CD49e | Alpha-5 integrin chain; FNR alpha chain; VLA-5 alpha chain |
| CD49f | Alpha-6 integrin chain; Platelet gpI; VLA-6 alpha chain |
| CD50 | ICAM-3 |
| CD51 | VNR-alpha chain; alpha V integrin chain; vitronectin receptor |
| CD52 | |
| CD53 | |
| CD54 | ICAM-1 |
| CD55 | DAF |
| CD56 | Leu-19; NKH1; NCAM |
| CD57 | HNK1; Leu-7 |
| CD58 | LFA-3 |
| CD59 | 1F-5Ag; H19; HRF20; MACIF; MIRL; P-18; Protectin |
| CD60a | GD3 |
| CD60b | 9-O-acetyl-GD3 |
| CD60c | 7-O-acetyl-GD3 |
| CD61 | CD61A; GPIIb/IIIa; beta 3 integrin chain |
| CD62E | E-selectin; ELAM-1; LECAM-2 |
| CD62L | L-selectin; LAM-1; LECAM-1; Leu-8; MEL-14; TQ-1 |
| CD62P | P-selectin; GMP-140; PADGEM |
| CD63 | LIMP; MLA1; PTLGP40; gp55; granulophysin; LAMP-3; ME491; NGA |
| CD64 | FC gammaRI; FCR I |
| CD65 | Ceramide-dodecasaccharide; VIM-2 |
| CD65s | Sialylated-CD65; VIM2 |
| CD66a | NCA-160; BGP |
| CD66b | CD67; CGM6; NCA-95 |
| CD66c | NCA; NCA-50/90 |
| CD66d | CGM1 |
| CD66e | CEA |
| CD66f | Pregnancy specific b1 glycoprotein; SP-1; PSG |
| CD68 | gp110; macrosialin |
| CD69 | AIM; EA 1; MLR3; gp34/28; VEA |
| CD70 | CD27-ligand; Ki-24 antigen |
| CD71 | T9; transferrin receptor |
| CD72 | Ly-19.2; Ly-32.2; Lyb-2 |
| CD73 | Ecto-5'-nucleotidase |
| CD74 | Class II-specific chaperone; Ii; Invariant chain |
| CD75 | Lactosamines |
| CD75s | Alpha-2,6-sialylated lactosamines (formerly CDw75 and CDw76) |
| CD77 | Pk blood group antigen; BLA; CTH; Gb3 |
| CD79a | Ig alpha; MB1 |
| CD79b | B29; Ig beta |
| CD80 | B7; BB1 |
| CD81 | TAPA-1 |
| CD82 | 4F9;C33;IA4;KAI1; R2 |
| CD83 | HB15 |
| CD84 | |
| CD85 | ILT/LIR family |
| CD86 | B7-2;B70 |
| CD87 | uPAR |
| CD88 | C5aR |
| CD89 | Fcalpha-R; IgA Fc receptor;IgA receptor |
| CD90 | Thy-1 |
| CD91 | ALPHA2M-R; LRP |
| CD92 | CTL1; formerly CDw92 |
| CDw93 | |
| CD94 | Kp43 |
| CD95 | APO-1; Fas; TNFRSF6; APT1 |
| CD96 | TACTILE |
| CD97 | |
| CD98 | 4F2; FRP-1; RL-388 |
| CD99 | CD99R; E2; MIC2 gene product |
| CD100 | SEMA4D |
| CD101 | IGSF2; P126; V7 |
| CD102 | ICAM-2 |
| CD103 | ITGAE; HML-1; integrin alphaE chain |
| CD104 | beta 4 integrin chain; TSP-1180; beta 4 |
| CD105 | endoglin |
| CD106 | INCAM-110; VCAM-1 |
| CD107a | LAMP-1 |
| CD107b | LAMP-2 |
| CD108 | SEMA7A; JMH human blood group antigen; formerly CDw108 |
| CD109 | 8A3; E123; 7D1 |
| CD110 | MPL; TPO-R; C-MPL |
| CD111 | PVRL1; PRR1; HevC; nectin-1; HIgR |
| CD112 | HVEB; PRR2; PVRL2; nectin 2 |
| CDw113 | PVRL3, Nectin3; poliovirus receptor-related 3; nectin-3 |
| CD114 | CSF3R; HG-CSFR; G-CSFR |
| CD115 | c-fms; CSF-1R; M-CSFR |
| CD116 | GM-CSF receptor alpha chain |
| CD117 | c-KIT; SCFR |
| CD118 | LIFR; leukemia inhibitory factor receptor |
| CDw119 | IFNgR; IFNgRa |
| CD120a | TNFRI; p55 |
| CD120b | TNFRII; p75; TNFR p80 |
| CD121a | IL-1R; type 1 IL-1R |
| CDw121b | IL-1R, type 2 |
| CD122 | IL-2Rbeta |
| CD123 | IL-3Ralpha |
| CD124 | IL-4R |
| CDw125 | IL-5Ralpha |
| CD126 | IL-6R |
| CD127 | IL-7R; IL-7R alpha; p90 I17 R |
| CDw128a | CXCR1; IL-8RA |
| CDw128b | CXCR2; IL-8RB |
| CD129 | Reserved |
| CD130 | gp130 |
| CD131 | common beta subunit |
| CD132 | IL2RG; common cytokine receptor gamma chain; common gamma chain |
| CD133 | PROML1; AC133; hematopoietic stem cell antigen; prominin-like 1 |
| CD134 | OX40 |
| CD135 | flt3; Flk-2; STK-1 |
| CDw136 | msp receptor; ron; p158-ron |
| CDw137 | 4-1BB; ILA |
| CD138 | heparan sulfate proteoglycan; syndecan-1 |
| CD139 | |
| CD140a | PDGF-R; PDGFRa |
| CD140b | PDGFRb |
| CD141 | fetomodulin; TM |
| CD142 | F3; coagulation Factor III; thromboplastin; TF |
| CD143 | EC 3.4.15.1; ACE; kininase II; peptidyl dipeptidase A |
| CD144 | cadherin-5; VE-Cadherin |
| CDw145 | |
| CD146 | MCAM; A32; MUC18; Mel-CAM; S-endo |
| CD147 | 5A11; Basigin; CE9; HT7; M6; Neurothelin; OX-47; EMMPRIN; gp42 |
| CD148 | HPTP-eta; DEP-1; p260 |
| CDw149 | new designation is CD47R |
| CD150 | SLAM; IPO-3; fomerly CDw150 |
| CD151 | PETA-3; SFA-1 |
| CD152 | CTLA-4 |
| CD153 | CD30L |
| CD154 | CD40L; T-BAM; TRAP; gp39 |
| CD155 | PVR |
| CD156a | ADAM8; MS2 human; fomerly CD156 |
| CD156b | ADAM17; TACE; cSVP |
| CDw156C | ADAM10; a disintegrin and metalloproteinase domain 10 |
| CD157 | BP-3/IF-7; BST-1; Mo5 |
| CD158 | KIR family |
| CD159a | NKG2A |
| CD159c | NKG2C; killer cell lectin-like receptor subfamily C, member 2 |
| CD160 | BY55 antigen; NK1; NK28 |
| CD161 | KLRB1; NKR-P1A; killer cell lectin-like receptor subfamily B, member 1 |
| CD162 | PSGL-1, PSGL |
| CD162R | PEN5 (a post-translational modification of PSGL-1) |
| CD163 | GHI/61; M130; RM3/1 |
| CD164 | MUC-24; MGC-24v |
| CD165 | AD2; gp37 |
| CD166 | BEN; DM-GRASP; KG-CAM; Neurolin; SC-1; ALCAM |
| CD167a | trkE; trk6; cak; eddr1; DDR1; MCK10; RTK6; NTRK4 |
| CD168 | HMMR; IHABP; RHAMM |
| CD169 | sialoadhesin; siglec-1 |
| CD170 | Siglec-5 |
| CD171 | L1; L1CAM; N-CAM L1 |
| CD172a | SIRP alpha |
| CD172b | SIRPbeta; signal-regulatory protein beta 1 |
| CD172g | SIRPgamma; signal-regulatory protein beta 2 |
| CD173 | Blood group H type 2 |
| CD174 | Lewis y |
| CD175 | Tn |
| CD175s | Sialyl-Tn |
| CD176 | TF |
| CD177 | NB1 |
| CD178 | fas-L; TNFSF6; APT1LG1; CD95-L |
| CD179a | VpreB; VPREB1; IGVPB |
| CD179b | IGLL1; lambda5; immunoglobulin omega polypeptide; IGVPB; 14.1 chain |
| CD180 | LY64; RP105 |
| CD181 | CXCR1; (was CDw128A), IL8Ralpha |
| CD182 | CXCR2; (was CDw128B), IL8Rbeta |
| CD183 | CXCR3; GPR9; CKR-L2; IP10-R; Mig-R |
| CD184 | CXCR4; fusin; LESTR; NPY3R; HM89; FB22 |
| CD185 | CXCR5; Chemokine (C-X-C motif) Receptor 5, Burkitt lymphoma receptor 1 |
| CDw186 | CXCR6; Chemokine (C-X-C motif) Receptor 6 |
| CD191 | CCR1; Chemokine (C-C motif) Receptor 1, RANTES Receptor |
| CD192 | CCR2; Chemokine (C-C motif) Receptor 2, MCP-1 receptor |
| CD193 | CCR3; Chemokine (C-C motif) Receptor 3, eosinophil eotaxin receptor |
| CD195 | CCR5 |
| CD196 | CCR6; Chemokine (C-C motif) Receptor 6 |
| CD197 | CCR7; (was CDw197) Chemokine (C-C motif) Receptor 7 |
| CDw198 | CCR8; Chemokine (C-C motif) Receptor 8 |
| CDw199 | CCR9; Chemokine (C-C motif) Receptor 9 |
| CDw197 | CCR7 |
| CD200 | OX2 |
| CD201 | EPC R |
| CD202b | tie2; tek |
| CD203c | NPP3; PDNP3; PD-Ibeta; B10; gp 130RB13-6; ENPP3; bovine intestinal phosphodiesterase |
| CD204 | macrophage scavenger R |
| CD205 | DEC205 |
| CD206 | MRC1; MMR |
| CD207 | Langerin |
| CD208 | DC-LAMP |
| CD209 | DC-SIGN |
| CDw210 | IL-10 R |
| CD212 | IL-12 R |
| CD213a1 | IL-13 R alpha 1 |
| CD213a2 | IL-13 R alpha 2 |
| CDw217 | IL-17 R |
| CDw218a | IL18Ralpha; IL18Ralpha |
| CDw218b | IL18Rbeta; IL18Rbeta |
| CD220 | Insulin R |
| CD221 | IGF1 R |
| CD222 | Mannose-6-phosphate/IGF2 R |
| CD223 | LAG-3 |
| CD224 | GGT; EC2.3.2.2 |
| CD225 | Leu13 |
| CD226 | DNAM-1; PTA1; TLiSA1 |
| CD227 | MUC1; episialin; PUM; PEM; EMA; DF3 antigen; H23 antigen |
| CD228 | melanotransferrin |
| CD229 | Ly9 |
| CD230 | Prion protein |
| CD231 | TM4SF2; A15; TALLA-1; MXS1; CCG-B7; TALLA |
| CD232 | VESP R |
| CD233 | band 3; erythrocyte membrane protein band 3;AE1; SLC4A1; Diego blood group; EPB3 |
| CD234 | Fy-glycoprotein; Duffy antigen |
| CD235a | Glycophorin A |
| CD235b | Glycophorin B |
| CD235ab | Glycophorin A/B crossreactive mabs |
| CD236 | Glycophorin C/D |
| CD236R | Glycophorin C |
| CD238 | Kell |
| CD239 | B-CAM |
| CD240CE | Rh30CE |
| CD240D | Rh30D |
| CD240DCE | Rh30D/CE crossreactive mabs |
| CD241 | RhAg |
| CD242 | ICAM-4 |
| CD243 | MDR-1 |
| CD244 | 2B4; NAIL; p38 |
| CD245 | p220/240 |
| CD246 | Anaplastic lymphoma kinase |
| CD247 | Zeta chain |
| CD248 | TEM1, Endosialin; CD164 sialomucin-like 1, tumor endothelial marker 1 |
| CD249 | Aminopeptidase A; APA, gp160 |
| CD252 | OX40L; TNF (ligand) superfamily member 4, CD134 ligand |
| CD253 | TRAIL; TNF (ligand) superfamily member 10, AP02L |
| CD254 | TRANCE; TNF (ligand) superfamily member 11, RANKL |
| CD256 | APRIL; TNF (ligand) superfamily member 13, TALL2 |
| CD257 | BLYS; TNF (ligand) superfamily, member 13b, TALL1, BAFF |
| CD258 | LIGHT; TNF (ligand) superfamily, member 14 |
| CD261 | TRAIL-R1; TNFR superfamily, member 10a, DR4, AP02 |
| CD262 | TRAIL-R2; TNFR superfamily, member 10b, DR5 |
| CD263 | TRAIL-R3; TNFR superfamily, member 10c, DCR1 |
| CD264 | TRAIL-R4; TNFR superfamily, member 10d, DCR2 |
| CD265 | TRANCE-R; TNFR superfamily, member 11a, RANK |
| CD266 | TWEAK-R; TNFR superfamily, member 12A, type I transmembrane protein Fn14 |
| CD267 | TACI; TNFR superfamily, member 13B, transmembrane activator and CAML interactor |
| CD268 | BAFFR; TNFR superfamily, member 13C, B cell-activating factor receptor |
| CD269 | BCMA; TNFR superfamily, member 17, B-cell maturation factor |
| CD271 | NGFR (p75); nerve growth factor receptor (TNFR superfamily, member 16) |
| CD272 | BTLA; B and T lymphocyte attenuator |
| CD273 | B7DC, PDL2; programmed cell death 1 ligand 2 |
| CD274 | B7H1, PDL1; programmed cell death 1 ligand 1 |
| CD275 | B7H2, ICOSL; inducible T-cell co-stimulator ligand (ICOSL) |
| CD276 | B7H3; B7 homolog 3 |
| CD277 | BT3.1; B7 family: butyrophilin, subfamily 3, member A1 |
| CD278 | ICOS; inducible T-cell co-stimulator |
| CD279 | PD1; programmed cell death 1 |
| CD280 | ENDO180; uPARAP, mannose receptor, C type 2, TEM22 |
| CD281 | TLR1; TOLL-like receptor 1 |
| CD282 | TLR2; TOLL-like receptor 2 |
| CD283 | TLR3; TOLL-like receptor 3 |
| CD284 | TLR4; TOLL-like receptor 4 |
| CD289 | TLR9; TOLL-like receptor 9 |
| CD292 | BMPR1A; Bone Morphogenetic Protein Receptor, type IA |
| CDw293 | BMPR1B; Bone Morphogenetic Protein Receptor, type IB |
| CD294 | CRTH2; PGRD2; G protein-coupled receptor 44, |
| CD295 | LEPR; Leptin Receptor |
| CD296 | ART1; ADP-ribosyltransferase 1 |
| CD297 | ART4; ADP-ribosyltransferase 4; Dombrock blood group glycoprotein |
| CD298 | ATP1B3; Na+/K+ -ATPase beta 3 subunit |
| CD299 | DCSIGN-related; CD209 antigen-like, DC-SIGN2, L-SIGN |
| CD300a | CMRF35 FAMILY; CMRF-35H |
| CD300c | CMRF35 FAMILY; CMRF-35A |
| CD300e | CMRF35 FAMILY; CMRF-35L1 |
| CD301 | MGL; CLECSF14, macrophage galactose-type C-type lectin |
| CD302 | DCL1; Type I transmembrane C-type lectin receptor DCL-1 |
| CD303 | BDCA2; C-type lectin, superfamily member 11 |
| CD304 | BDCA4; Neuropilin 1 |
| CD305 | LAIR1; Leukocyte-Associated Ig-like Receptor 1 |
| CD306 | LAIR2; Leukocyte-Associated Ig-like Receptor 2 |
| CD307 | IRTA2; Immunoglobulin superfamily Receptor Translocation Associated 2 |
| CD309 | VEGFR2; KDR (a type III receptor tyrosine kinase) |
| CD312 | EMR2 ; EGF-like module containing, mucin-like, hormone receptor-like 2 |
| CD314 | NKG2D; Killer cell lectin-like receptor subfamily K, member 1 |
| CD315 | CD9P1; Prostaglandin F2 receptor negative regulator |
| CD316 | EWI2; Immunoglobulin superfamily, member 8 |
| CD317 | BST2; Bone Marrow Stromal cell antigen 2 |
| CD318 | CDCP1; CUB domain-containing protein 1 |
| CD319 | CRACC; SLAM family member 7 |
| CD320 | 8D6; 8D6 Antigen; FDC |
| CD321 | JAM 1; F11 receptor |
| CD322 | JAM2; Junctional Adhesion Molecule 2 |
| CD324 | E-Cadherin; cadherin 1, type 1, E-cadherin (epithelial) |
| CDw325 | N-Cadherin; cadherin 2, type 1, N-cadherin (neuronal) |
| CD326 | Ep-CAM; tumor-associated calcium signal transducer 1 |
| CDw327 | siglec6; sialic acid binding Ig-like lectin 6 |
| CDw328 | siglec7; sialic acid binding Ig-like lectin 7 |
| CDw329 | siglec9; sialic acid binding Ig-like lectin 9 |
| CD331 | FGFR1; Fibroblast Growth Factor Receptor 1 |
| CD332 | FGFR2; Fibroblast Growth Factor Receptor 2 (keratinocyte growth factor receptor) |
| CD333 | FGFR3; Fibroblast Growth Factor Receptor 3 (achondroplasia, thanatophoric dwarfism) |
| CD334 | FGFR4; Fibroblast Growth Factor Receptor 4 |
| CD335 | NKp46; NCR1, (Ly94); natural cytotoxicity triggering receptor 1 |
| CD336 | NKp44; NCR2, (Ly95); natural cytotoxicity triggering receptor 2 |
| CD337 | NKp30; NCR3 |
| CDw338 | ABCG2; ATP-binding cassette, sub-family G (WHITE), member 2 |
| CD339 | Jagged-1; Jagged 1 (Alagille syndrome) |

As used herein, the term "nucleic acid" refers to a double stranded nucleic acid typically made of DNA (deoxyribonucleic acid). Accordingly, when a sequence is given (e.g. sequence CL, P1, P2, DNA barcode), the nucleic acid molecule comprises the complementary sequence.

As used herein the term "enzymatic cleavable sequence" refers to any nucleic acid sequence that can be cleaved by an enzyme. Typically the enzyme is a protease, an endonuclease but is preferably a restriction endonculease. The one skilled in the art can easily identify an enzymatic cleavable sequence. Restriction endonucleases (also called restriction enzymes) are capable of recognizing and cleaving a DNA molecule at a specific DNA cleavage site between predefined nucleotides. In contrast, some endonucleases such as for example Fokl comprise a cleavage domain that cleaves the DNA unspecifically at a certain position regardless of the nucleotides present at this position. Therefore, preferably the specific DNA cleavage site and the DNA recognition site of the restriction endonuclease are identical. Typically restriction endonucleases, particularly type II restriction endonucleases, bind as a homodimer to DNA regularly. Accordingly, the nuclease has a reduced capability of forming homodimers in the absence of the DNA recognition site, thereby preventing unspecific DNA binding. Therefore, a functional homodimer is only formed upon recruitment of nucleases monomers to the specific DNA recognition sites. Accordingly in some embodiments, the restriction endonuclease from which the cleavage module of the nuclease is derived is a type 11P restriction endonuclease. The preferably palindromic DNA recognition sites of these restriction endonucleases consist of at least four or up to eight contiguous nucleotides. Preferably, the type 11P restriction endonucleases cleave the DNA within the recognition site which occurs rather frequently in the genome, or immediately adjacent thereto, and have no or a reduced star activity. The type 11P restriction endonucleases as referred to herein are preferably selected from the group consisting of: Pvull, EcoRV, BamH1, Bcnl, BfaSORF1835P, BfiI, Bgll, Bglll, BpuJ1, Bse6341, BsoB1, BspD6I, BstYl, Cfr101, Ecl18k1, Eco01091, EcoRl, EcoRll, EcoRV, EcoR1241, EcoR12411, HinP11, Hincll, Hindlll, Hpy991, Hpy1881, Mspl, Munl, Mval, Nael, NgoMIV, Notl, OkrAl, Pabl, Pacl, PspGl, Sau3Al, Sdal, Sfil, SgrAl, Thal, VvuYORF266P, Ddel, Eco571, Haelll, Hhall, Hindll, and Ndel. Other nuclease for use in the present invention are disclosed in WO 2010/079430, WO2011072246, WO2013045480, Mussolino C, et al (Curr Opin Biotechnol. 2012 Oct;23(5):644-50) and Papaioannou I. et al (Expert Opinion on Biological Therapy, March 2012, Vol. 12, No. 3 : 329-342) all of which are herein incorporated by reference.

In one aspect of any of the embodiments herein, an enzymatic cleavable sequence CL is a nucleic acid sequence of 2, 3, 4, 5, 6, 7, 8, 9 or 10 nucleotides in length. In one embodiment CL comprises a sequence of at least 10 nucleotides. In one embodiment CL comprises a sequence of between 2 and 20 nucleotides. In one embodiment CL comprises a sequence of between 2 and 15 nucleotides. In one embodiment CL comprises a sequence of between 4 and 10 nucleotides. In one embodiment CL comprises a sequence of between 4 and 15 nucleotides.

Restriction endonucleases that recognize the same sequence are isoschizomers.

Neoschizomers are a subset of isoschizomers that recognize the same sequence, but cleave at different positions from the prototype. Thus, AatII (recognition sequence: GACGT ↓ C) and ZraI (recognition sequence: GAC ↓ GTC) are neoschizomers of one another, while HpaII (recognition sequence: C ↓ CGG) and MspI (recognition sequence: C ↓ CGG) are isoschizomers. Analogous designations are not appropriate for MTases, where the differences between enzymes are not so easily defined and usually have not been well characterized.

**Table B: Examples isoschizomers for commercially available restriction endonucleases.** Isoschizomers with alternative cleavage sites are indicated with a " ^ ". All recognition sequences are written 5' to 3' using the single letter code nomenclature with the point of cleavage indicated by a "/".Numbers in parentheses indicate point of cleavage for non-palindromic enzymes. For example, GGTCTC(1/5) indicates cleavage at: 5' ...GGTCTCN/...3' 3' ...CCAGAGNNNNN/...5'. B = C or G or T ; D = A or G or T; H = A or C or T; K = G or T; M = A or C; N = A or C or G or T; R = A or G;S = C or G; V = A or C or G; W = A or T and Y = C or T.

| **Enzyme** | **CL Sequence** | **Other Isoschizomers** |
|---|---|---|
| AanI | TTA/TAA | PsiI |
| AarI | CACCTGC(4/8) | |
| AasI | GACNNNN/NNGTC | DrdI, DseDI |
| AatI | AGG/CCT | Eco147I, PceI , SseBI, StuI |
| AatII | GACGT/C | ZraI^ |
| AauI | T/GTACA | Bsp 1407I, BsrGI, BstAUI, SspBI |
| AbaCIIIx | CTATCAV | |
| AbsI | CC/TCGAGG | |
| Acc113I | AGT/ACT | AssI , BmcAI^, ScaI, ZrmI |
| Acc16I | TGC/GCA | AviII , FspI, MstI, NsbI |
| Acc36I | ACCTGC(4/8) | BfuAI, BspMI, BveI |
| Acc65I | G/GTACC | Asp718I , KpnI^ |
| AccB1I | G/GYRCC | BanI, BshNI, BspT107I, HgiCI |
| AccB7I | CCANNNN/NTGG | BasI , PflBI , PflMI, Van91I |
| AccBSI | CCGCTC(-3/-3) | BsrBI, MbiI |
| AccI | GT/MKAC | FblI, XmiI |
| AccII | CG/CG | Bsh1236I, BspFNI^, BstFNI, BstUI, FnuDII^, MvnI, ThaI |
| AccIII | T/CCGGA | Aor13HI , BlfI , BseAI, BsiMI, Bsp13I, BspEI, BspMII, Kpn2I , MroI |
| AceIII x | CAGCTC(7/11) | |
| AciI | CCGC(-3/-1) | BspACI, SsiI |
| AclI | AA/CGTT | Psp1406I |
| AclWI | GGATC(4/5) | AlwI, BinI, BspPI, Nt.AlwI^ |
| Aco12261IIx | CCRGAG | |
| AcoI | Y/GGCCR | CfrI, EaeI |
| AcsI | R/AATTY | ApoI, XapI |
| AcuI | CTGAAG(16/14) | Eco57I |
| AcvI | CAC/GTG | BbrPI , Eco72I, PmaCI , PmlI, PspCI |
| AcyI | GR/CGYC | BsaHI, BssNI, BstACI, Hin1I, Hsp92I |
| AdeI | CACNNN/GTG | DraIII |
| AfaI | GT/AC | Csp6I^, CviQI^, RsaI |
| AfeI | AGC/GCT | Aor51HI , Eco47III, FunI |
| AfiI | CCNNNNN/NNGG | Bsc4I, BseLI, BsiYI, BslI |
| AflI | G/GWCC | AvaII, Bme18I, Eco47I, PspO3I^, SinI, Vpak11AI^ |
| AflII | C/TTAAG | BfrI , BspTI, Bst98I, BstAFI, MspCI, Vha464I |
| AflIII | A/CRYGT | |
| AgeI | A/CCGGT | AsiAI , BshTI, CspAI, PinAI |
| AgsI | TTS/AA | |
| AhaIII x | TTT/AAA | DraI^ |
| AhdI | GACNNN/NNGTC | AspEI , BmeRI, DriI, Eam1105I, EclHKI, NruGI |
| AhlI | A/CTAGT | BcuI , SpeI |
| AjiI | CACGTC(-3/-3) | BmgBI, BtrI^ |
| AjnI | /CCWGG | BciT130I^, BptI^, BseBI^, BsiLI^, Bst2UI^, BstNI^, BstOI^, EcoRII^, MvaI^, Psp6I ^, PspGI^ |
| AjuI | (7/12)GAANNNNNNNTTGG(11/6) | |
| AleI | CACNN/NNGTG | OliI |
| AlfI | (10/12)GCANNNNNNTGC(12/10) | |
| AloI | (7/12)GAACNNNNNNTCC(12/7) | |
| AluBI | AG/CT | |
| AluI | AG/CT | |
| Alw21I | GWGCW/C | AspHI , Bbv12I , BsiHKAI, HgiAI |
| Alw26I | GTCTC(1/5) | BcoDI, BsmAI, BsoMAI, BstMAI, Nt.BsmAI^ |
| Alw44I | G/TGCAC | ApaLI, VneI |
| AlwFI x | GAAAYNNNNNRTG | |
| AlwI | GGATC(4/5) | AclWI , BinI, BspPI, Nt.AlwI^ |
| AlwNI | CAGNNN/CTG | CaiI, PstNI |
| Ama87I | C/YCGRG | AvaI, BcoI , BmeT110I, BsiHKCI, BsoBI, Eco88I, NspIII |
| AocI | CC/TNAGG | AxyI , Bse21I, Bsu36I, CvnI, Eco81I, SauI |
| Aor13HI | T/CCGGA | AccIII, BlfI , BseAI, BsiMI, Bsp13I, BspEI, BspMII, Kpn2I , MroI |
| Aor51HI | AGC/GCT | AfeI, Eco47III, FunI |
| AoxIx | /GGCC | BshFI ^, BsnI^, BspANI^, BsuRI^, HaeIII, PalI ^, PhoI^ |
| ApaBIx | GCANNNNN/TGC | BstAPI^ |
| ApaI | GGGCC/C | Bsp120I^, PspOMI^ |
| ApaLI | G/TGCAC | Alw44I, VneI |
| ApeKI | G/CWGC | TseI |
| ApoI | R/AATTY | AcsI , XapI |
| ApyPIx | ATCGAC(20/18) | |
| AquIIx | GCCGNAC(20/18) | |
| AquIVx | GRGGAAG(19/17) | |
| ArsI | (8/13)GACNNNNNNTTYG(11/6) | |
| AscI | GG/CGCGCC | PalAI , SgsI |
| AseI | AT/TAAT | AsnI , PshBI , VspI |
| AsiAI | A/CCGGT | AgeI, BshTI, CspAI, PinAI |
| AsiGI | A/CCGGT | AgeI, AsiAI , BshTI, CspAI, PinAI |
| AsiSI | GCGAT/CGC | RgaI, SfaAI, SgfI |
| AsnI | AT/TAAT | AseI, PshBI , VspI |
| Asp700I | GAANN/NNTTC | MroXI, PdmI , XmnI |
| Asp718I | G/GTACC | Acc65I, KpnI^ |
| AspA2I | C/CTAGG | AvrII, BlnI , BspA2I, XmaJI |
| AspBHIx | YSCNS(8/12) | |
| AspCNI x | GCCGC | |
| AspEI | GACNNN/NNGTC | AhdI, BmeRI, DriI, Eam1105I, EclHKI, NruGI |
| AspHI | GWGCW/C | Alw21I , Bbv12I , BsiHKAI, HgiAI |
| AspI | GACN/NNGTC | PflFI, PsyI, TelI, Tth111I |
| AspLEI | GCG/C | BstHHI, CfoI, GlaI^, HhaI, R9529^, HinP1I^, HspAI ^ |
| AspS9I | G/GNCC | AsuI , BmgT120I^, BsiZI, BspLI^, Cfr13I, FmuI^, PspPI , Sau96I, UnbI^ |
| AssI | AGT/ACT | Acc113I , BmcAI^, ScaI, ZrmI |
| AsuC2I | CC/SGG | BcnI , BpuMI, CauII, EcoHI^, NciI |
| AsuHPI | GGTGA(8/7) | HphI |
| AsuI x | G/GNCC | AspS9I , BmgT120I^, BsiZI, BspLI^, Cfr13I, FmuI^, PspPI , Sau96I, UnbI^ |
| AsuII | TT/CGAA | Bpu14I, BsiCI, Bsp119I, BspT104I, BstBI, Csp45I, NspV , SfuI |
| AsuNHI | G/CTAGC | BmtI^, BspOI^, NheI |
| AvaI | C/YCGRG | Ama87I , BcoI , BmeT110I, BsiHKCI, BsoBI, Eco88I, NspIII |
| AvaII | G/GWCC | AflI , Bme18I, Eco47I, PspO3I^, SinI, Vpak11AI^ |
| AvaIII x | ATGCAT | BsrB1^, EcoT22I^, Mph1103I^, NSiI^, Zsp2I^ |
| AviII | TGC/GCA | Acc16I , FspI, MstI, NsbI |
| AvrII | C/CTAGG | AspA2I , BlnI , BspA2I, XmaJI |
| AxyI | CC/TNAGG | AocI , Bse21I, Bsu36I, CvnI, Eco81I, SauI |
| BaeGI | GKGCM/C | BseSI, BstSLI |
| BaeI | (10/15)ACNNNNGTAYC(12/7) | |
| BalI | TGG/CCA | MlsI, MluNI, MscI, Msp20I |
| BamHI | G/GATCC | |
| BanI | G/GYRCC | AccB1I , BshNI, BspT107I, HgiCI |
| BanII | GRGCY/C | Eco24I, EcoT38I, FriOI, HgiJII |
| BanIII | AT/CGAT | Bsa29I, BseCI, BshVI, BsiXI, Bsp106I, BspDI, BspXI, Bsu15I, BsuTUI, ClaI, ZhoI |
| BarI | (7/12)GAAGNNNNNNTAC(12/7) | |
| BasI | CCANNNN/NTGG | AccB7I , PflBI , PflMI, Van91I |
| BauI | CACGAG(-5/-1) | BsiI, BssSI, Bst2BI |
| BbeI | GGCGC/C | DinI^, EgeI^, KasI^, Mly113I^, NarI^, PluTI, SfoI^, SspDI^ |
| BbrPI | CAC/GTG | AcvI , Eco72I, PmaCI , PmlI, PspCI |
| BbsI | GAAGAC(2/6) | BbvII , BpiI, BpuAI, BstV2I |
| BbuI | GCATG/C | PaeI , SpaHI, SphI-HF® |
| Bbv12I | GWGCW/C | Alw21I , AspHI , BsiHKAI, HgiAI |
| BbvCI | CCTCAGC(-5/-2) | Nb.BbvCI^, Nt.BbvCI^ |
| BbvI | GCAGC(8/12) | BseXI, Bst71I, BstV1I, R9896 |
| BbvII x | GAAGAC(2/6) | BbsI, BpiI, BpuAI, BstV2I |
| BccI | CCATC(4/5) | |
| Bce83I x | CTTGAG(16/14) | BpuEI |
| BceAI | ACGGC(12/14) | |
| BcefI x | ACGGC(12/13) | |
| BcgI | (10/12)CGANNNNNNTGC(12/10) | |
| BciT130I | CC/WGG | AjnI ^, BptI, BseBI, BsiLI, Bst2UI, BstNI, BstOI, EcoRII^, MvaI, Psp6I ^, PspGI^ |
| BciVI | GTATCC(6/5) | BfuI , BsuI |
| BclI | T/GATCA | BsiQI, FbaI, Ksp22I |
| BcnI | CC/SGG | AsuC2I , BpuMI, CauII, EcoHI^, NciI |
| BcoDI | GTCTC(1/5) | Alw26I , BsmAI, BsoMAI, BstMAI, Nt.BsmAI^ |
| BcoI | C/YCGRG | Ama87I , AvaI, BmeT110I, BsiHKCI, BsoBI, Eco88I, NspIII |
| BcuI | A/CTAGT | AhlI, SpeI |
| BdaIx | (10/12)TGANNNNNNTCA(12/10) | |
| BetI x | W/CCGGW | BsaWI |
| BfaI | C/TAG | FspBI, MaeI, XspI |
| BfiI x | ACTGGG(5/4) | BmrI, BmuI |
| BfmI | C/TRYAG | BpcI, BstSFI, SfeI |
| BfoI | RGCGC/Y | Bsp143II, BstH2I, HaeII |
| BfrBlx | ATGCA/T | AvaIII ^, EcoT22I, Mph1103I, NsiI, Zsp2I |
| BfrI | C/TTAAG | AflII, BspTI, Bst98I, BstAFI, MspCI, Vha464I |
| BfuAI | ACCTGC(4/8) | Acc36I , BspMI, BveI |
| BfuCI | /GATC | BscFI, Bsp143I, BssMI, BstENII, BstKTI^, BstMBI, DpnI^, Kzo9I, MalI^, MboI, NdeII^, Sau3AI |
| BfuI | GTATCC(6/5) | BciVI, BsuI |
| BolI | GCCNNNN/NGGC | |
| BglII | A/GATCT | |
| BinIx | GGATC(4/5) | AclWI , AlwI, BspPI, Nt.AlwI^ |
| BisI | GC/NGC | BlsI ^, BthCI^, Fnu4HI, Fsp4HI, GluI, ItaI , SatI |
| BlfI | T/CCGGA | AccIII, Aor13HI , BseAI, BsiMI, Bsp13I, BspEI, BspMII, Kpn2I , MroI |
| BlnI | C/CTAGG | AspA2I , AvrII, BspA2I, XmaJI |
| BlpI | GC/TNAGC | Bpu1102I, Bsp1720I, CelII, EspI |
| BlsI | GCN/GC | BisI^, BthCI^, Fnu4HI^, Fsp4HI^, GluI^, ItaI^, SatI^ |
| BmcAI | AGT/ACT | Acc113I^, AssI^, ScaI^, ZrmI^ |
| Bme1390I | CC/NGG | BmrFI, BssKI^, BstSCI^, MspR9I, ScrFI, StyD4I^ |
| Bme18I | G/GWCC | AflI, AvaII, Eco47I, PspO3I^, SinI, Vpak11AI^ |
| BmeRI | GACNNN/NNGTC | AhdI, AspEI, DriI, Eam1105I, EclHKI, NruGI |
| BmeT110I | C/YCGRG | Ama87I, AvaI, BcoI, BsiHKCI, BsoBI, Eco88I, NspIII |
| BmgBI | CACGTC(-3/-3) | AjiI, BtrI^ |
| BmgIx | GKGCCC | |
| BmgT120I | GG/NCC | AspS9I^, AsuI^, BsiZI^, BspLI^, Cfr13I^, FmuI^, PspPI^, Sau96I^, UnbI^ |
| BmiI | GGN/NCC | BscBI, NlaIV, PspN4I |
| BmrFI | CC/NGG | Bme1390I, BssKI^, BstSCI^, MspR9I, ScrFI, StyD4I^ |
| BmrI | ACTGGG(5/4) | BfiI , BmuI |
| BmsI | GCATC(5/9) | LweI, SfaNI |
| BmtI | GCTAG/C | AsuNHI^, BspOI, NheI^ |
| BmuI | ACTGGG(5/4) | BfiI , BmrI |
| BmyI | GDGCH/C | Bsp1286I, MhlI, SduI |
| BoxI | GACNN/NNGTC | BstPAI, PshAI |
| BpcI | C/TRYAG | BfmI , BstSFI, SfcI |
| Bpil | GAAGAC(2/6) | BbsI, BbvII , BpuAI, BstV2I |
| BplI | (8/13)GAGNNNNNCTC(13/8) | |
| BpmI | CTGGAG(16/14) | GsuI |
| BptI | CC/WGG | AjnI^, BciT130I, BseBI, BsiLI, Bst2UI, BstNI, BstOI, EcoRII^, MvaI, Psp6I^, PspGI^ |
| Bpu10I | CCTNAGC(-5/-2) | |
| Bpu1102I | GC/TNAGC | BlpI, Bsp1720I, CelII, EspI |
| Bpu14I | TT/CGAA | AsuII , BsiCI, Bsp119I, BspT104I, BstBI, Csp45I, NspV , SfuI |
| BpuAI | GAAGAC(2/6) | BbsI, BbvII , BpiI, BstV2I |
| BpuEI | CTTGAG(16/14) | Bce83I |
| BpuMI | CC/SGG | AsuC2I , BcnI , CauII, EcoHI^, NciI |
| BpvUI | CGAT/CG | BspCI, MvrI, Ple19I , PvuI |
| Bsa29I | AT/CGAT | BanIII , BseCI, BshVI, BsiXI, Bsp106I, BspDI, BspXI, Bsu15I, BsuTUI, ClaI, ZhoI |
| BsaAI | YAC/GTR | BstBAI, Ppu21I |
| BsaBI | GATNN/NNATC | Bse8I, BseJI, BsiBI, BsrBRI, MamI |
| BsaHI | GR/CGYC | AcyI , BssNI, BstACI, Hin1I, Hsp92I |
| BsaI | GGTCTC(1/5) | Bso31I, BspTNI, Eco31I |
| BsaJI | C/CNNGG | BseDI, BssECI, SecI |
| BsaMI | GAATGC(1/-1) | BscCI, BsmI, Mva1269I, Nb.BsmI^, PctI |
| BsaOI | CGRY/CG | Bsh1285I , BsiEI, BstMCI, McrI |
| BsaWI | W/CCGGW | BetI |
| BsaXI | (9/12)ACNNNNNCTCC(10/7) | |
| BsbIx | CAACAC(21/19) | |
| Bsc4I | CCNNNNN/NNGG | AfiI , BseLI, BsiYI, BslI |
| BscBI | GGN/NCC | BmiI, NlaIV, PspN4I |
| BscCI | GAATGC(1/-1) | BsaMI, BsmI, Mva1269I, Nb.BsmI^, PctI |
| BscFI | /GATC | BfuCI, Bsp143I, BssMI, BstENII, BstKTI^, BstMBI, DpnI^, Kzo9I, MalI^, MboI, NdeII^, Sau3AI |
| BscGIx | CCCGT | |
| Bse118I | R/CCGGY | BsrFI, BssAI, Cfr10I |
| Bse1I | ACTGG(1/-1) | BseNI, BsrI |
| Bse21I | CC/TNAGG | AocI , AxyI , Bsu36I, CvnI, Eco81I, SauI |
| Bse3DI | GCAATG(2/0) | BseMI, BsrDI, Nb.BsrDI^ |
| Bse8I | GATNN/NNATC | BsaBI, BseJI, BsiBI, BsrBRI, MamI |
| BseAI | T/CCGGA | AccIII, Aor13HI, BlfI , BsiMI, Bsp13I, BspEI, BspMII, Kpn2I , MroI |
| BseBI | CC/WGG | AjnI^, BciT130I, BptI, BsiLI, Bst2UI, BstNI, BstOI, EcoRII^, MvaI, Psp6I^, PspGI^ |
| BseCI | AT/CGAT | BanIII , Bsa29I, BshVI, BsiXI, Bsp106I, BspDI, BspXI, Bsu15I, BsuTUI, ClaI, ZhoI |
| BseDI | C/CNNGG | BsaJI, BssECI, SecI |
| BseGI | GGATG(2/0) | BstF5I, BtsCI, FokI^ |
| BseJI | GATNN/NNATC | BsaBI, Bse8I, BsiBI, BsrBRI, MamI |
| BseLI | CCNNNNN/NNGG | AfiI , Bsc4I, BsiYI, BslI |
| BseMI | GCAATG(2/0) | Bse3DI, BsrDI, Nb.BsrDI^ |
| BseMII | CTCAG(10/8) | BspCNI^ |
| BseNI | ACTGG(1/-1) | Bse1I, BsrI |
| BsePI | G/CGCGC | BssHII, PauI , PteI |
| BseRI | GAGGAG(10/8) | |
| BseSI | GKGCM/C | BaeGI, BstSLI |
| BseX3I | C/GGCCG | BstZI, EagI, EclXI, Eco52I, XmaIII |
| BseXI | GCAGC(8/12) | BbvI, Bst71I, BstV1I, R9896 |
| BseYI | CCCAGC(-5/-1) | GsaI^ |
| BsgI | GTGCAG(16/14) | |
| Bsh1236I | CG/CG | AccII , BspFNI^, BstFNI, BstUI, FnuDII^, MvnI, ThaI |
| Bsh1285I | CGRY/CG | BsaOI, BsiEI, BstMCI, McrI |
| BshFI | GG/CC | AoxI^, BsnI, BspANI, BsuRI, HaeIII, PalI , PhoI |
| BshI | GG/CC | AoxI^, BshFI , BsnI, BspANI, BsuRI, HaeIII, PalI , PhoI |
| BshNI | G/GYRCC | AccB1I , BanI, BspT107I, HgiCI |
| BshTI | A/CCGGT | AgeI, AsiAI, CspAI, PinAI |
| BshVI | AT/CGAT | BanIII , Bsa29I, BseCI, BsiXI, Bsp106I, BspDI, BspXI, Bsu15I, BsuTUI, ClaI, ZhoI |
| BsiBI | GATNN/NNATC | BsaBI, Bse8I, BseJI, BsrBRI, MamI |
| BsiCI | TT/CGAA | AsuII , Bpu14I, Bsp119I, BspT104I, BstBI, Csp45I, NspV , SfuI |
| BsiEI | CGRY/CG | BsaOI, Bsh1285I , BstMCI, McrI |
| BsiHKAI | GWGCW/C | Alw211 , AspHI , Bbv12I , HgiAI |
| BsiHKCI | C/YCGRG | Ama87I, AvaI, BcoI, BmeT110I, BsoBI, Eco88I, NspIII |
| BsiIx | CACGAG(-5/-1) | BauI , BssSI, Bst2BI |
| BsiLI | CC/WGG | AjnI^, BciT130I, BptI, BseBI, Bst2UI, BstNI, BstOI, EcoRII^, MvaI, Psp6I^, PspGI^ |
| BsiMI | T/CCGGA | AccIII, Aor13HI, BlfI , BseAI, Bsp13I, BspEI, BspMII, Kpn2I , MroI |
| BsiQI | T/GATCA | BclI, FbaI, Ksp22I |
| BsiSI | C/CGG | HapII, HpaII, MspI |
| BsiWI | C/GTACG | Pfl23II , PspLI , SplI, SunI |
| BsiXI | AT/CGAT | BanIII , Bsa29I, BseCI, BshVI, Bsp106I, BspDI, BspXI, Bsu15I, BsuTUI, ClaI, ZhoI |
| BsiYIx | CCNNNNN/NNGG | AfiI , Bsc4I, BseLI, BslI |
| BsiZI | G/GNCC | AspS9I , AsuI , BmgT120I^, BspLI^, Cfr13I, FmuI^, PspPI , Sau96I, UnbI^ |
| BslFI | GGGAC(10/14) | BsmFI, FaqI, FinI^ |
| BslI | CCNNNNN/NNGG | AfiI , Bsc4I, BseLI, BsiYI |
| BsmAI | GTCTC(1/5) | Alw26I , BcoDI, BsoMAI, BstMAI, Nt. BsmAI^ |
| BsmBI | CGTCTC(1/5) | Esp3I |
| BsmFI | GGGAC(10/14) | BslFI, FaqI, FinI^ |
| BsmI | GAATGC(1/-1) | BsaMI, BscCI, Mva1269I, Nb.BsmI^, PctI |
| BsnI | GG/CC | AoxI^, BshFI , BspANI, BsuRI, HaeIII, PalI , PhoI |
| Bso31I | GGTCTC(1/5) | BsaI, BspTNI, Eco31I |
| BsoBI | C/YCGRG | Ama87I, AvaI, BcoI, BmeT110I, BsiHKCI, Eco88I, NspIII |
| BsoMAI | GTCTC(1/5) | Alw26I , BcoDI, BsmAI, BstMAI, Nt.BsmAI^ |
| Bsp106I | AT/CGAT | BanIII , Bsa29I, BseCI, BshVI, BsiXI, BspDI, BspXI, Bsu15I, BsuTUI, ClaI, ZhoI |
| Bsp119I | TT/CGAA | AsuII , Bpu14I, BsiCI, BspT104I, BstBI, Csp45I, NspV , SfuI |
| Bsp120I | G/GGCCC | ApaI^, PspOMI |
| Bsp1286I | GDGCH/C | BmyI, MhlI, SduI |
| Bsp13I | T/CCGGA | AccIII, Aor13HI, BlfI , BseAI, BsiMI, BspEI, BspMII, Kpn2I , MroI |
| Bsp 1407I | T/GTACA | AauI , BsrGI, BstAUI, SspBI |
| Bsp143I | /GATC | BfuCI, BscFI, BssMI, BstENII, BstKTI^, BstMBI, DpnI^, Kzo9I, MalI^, MboI, NdeII^, Sau3AI |
| Bsp143II | RGCGC/Y | BfoI, BstH2I, HaeII |
| Bsp1720I | GC/TNAGC | BlpI, Bpu1102I, CelII, EspI |
| Bsp19I | C/CATGG | NcoI |
| Bsp24Ix | (8/13)GACNNNNNNTGG(12/7) | |
| Bsp68I | TCG/CGA | BtuMI, NruI, RruI^ |
| BspA2I | C/CTAGG | AspA2I , AvrII, BlnI , XmaJI |
| BspACI | CCGC(-3/-1) | AciI, SsiI |
| BspANI | GG/CC | AoxI^, BshFI , BsnI, BsuRI, HaeIII, PalI , PhoI |
| BspCI | CGAT/CG | BpvUI, MvrI, Ple19I , PvuI |
| BspCNI | CTCAG(9/7) | BseMII^ |
| BspDI | AT/CGAT | BanIII , Bsa29I, BseCI, BshVI, BsiXI, Bsp106I, BspXI, Bsu15I, BsuTUI, ClaI, ZhoI |
| BspEI | T/CCGGA | AccIII, Aor13HI, BlfI , BseAI, BsiMI, Bsp13I, BspMII, Kpn2I , MroI |
| BspFNI | CG/CG | AccII^, Bsh1236I^, BstFNI^, BstUI^, FnuDII, MvnI^, ThaI^ |
| BspGIx | CTGGAC | |
| BspHI | T/CATGA | CciI, PagI , RcaI |
| BspLI | GGN/CC | AspS9I^, AsuI^, BmgT120I^, BsiZI^, Cfr13I^, FmuI^, PspPI^, Sau96I^, UnbI^ |
| BspLU11Ix | A/CATGT | PciI, PscI |
| BspMAI | CTGCA/G | PstI |
| BspMI | ACCTGC(4/8) | Acc36I , BfuAI, BveI |
| BspMIIx | T/CCGGA | AccIII, Aor13HI, BlfI , BseAI, BsiMI, Bsp13I, BspEI, Kpn2I , MroI |
| BspNCIx | CCAGA | |
| BspOI | GCTAG/C | AsuNHI^, BmtI, NheI^ |
| BspPI | GGATC(4/5) | AclWI , AlwI, BinI, Nt.AlwI^ |
| BspQI | GCTCTTC(1/4) | LguI, Nt.BspQI^, PciSI , SapI |
| BspT104I | TT/CGAA | AsuII , Bpu14I, BsiCI, Bsp119I, BstBI, Csp45I, NspV , SfuI |
| BspT107I | G/GYRCC | AccB1I , BanI, BshNI, HgiCI |
| BspTI | C/TTAAG | AflII, BfrI , Bst98I, BstAFI, MspCI, Vha464I |
| BspTNI | GGTCTC(1/5) | BsaI, Bso31I, Eco31I |
| BspUIx | GCSG/C | |
| BspXI | AT/CGAT | BanIII , Bsa29I, BseCI, BshVI, BsiXI, Bsp106I, BspDI, Bsu15I, BsuTUI, ClaI, ZhoI |
| BsrBI | CCGCTC(-3/-3) | AccBSI , MbiI |
| BsrBRI | GATNN/NNATC | BsaBI, Bse8I, BseJI, BsiBI, MamI |
| BsrDI | GCAATG(2/0) | Bse3DI, BseMI, Nb.BsrDI^ |
| BsrFI | R/CCGGY | Bse118I, BssAI, Cfr10I |
| BsrGI | T/GTACA | AauI , Bsp1407I, BstAUI, SspBI |
| BsrI | ACTGG(1/-1) | Bse1I, BseNI |
| BsrSI | ACTGG(1/-1) | Bse1I, BseNI, BsrI |
| BssAI | R/CCGGY | Bse118I, BsrFI, Cfr10I |
| BssECI | C/CNNGG | BsaJI, BseDI, SecI |
| BssHI | C/TCGAG | PaeR7I, Sfr274I, SlaI, StrI, TliI, XhoI |
| BssHII | G/CGCGC | BsePI, PauI , PteI |
| BssKI | /CCNGG | Bme1390I^, BmrFI^, BstSCI, MspR9I^, ScrFI^, StyD4I |
| BssMI | /GATC | BfuCI, BscFI, Bsp143I, BstENII, BstKTI^, BstMBI, DpnI^, Kzo9I, MalI^, MboI, NdeII^, Sau3AI |
| BssNAI | GTA/TAC | Bst1107I, BstZ17I, SnaI^ |
| BssNI | GR/CGYC | AcyI , BsaHI, BstACI, Hin1I, Hsp92I |
| BssSI | CACGAG(-5/-1) | BauI , BsiI, Bst2BI |
| BssT1I | C/CWWGG | Eco130I, EcoT14I, ErhI , Styl |
| Bst1107I | GTA/TAC | BssNAI, BstZ17I, SnaI^ |
| Bst2BI | CACGAG(-5/-1) | BauI , BsiI, BssSI |
| Bst2UI | CC/WGG | AjnI^, BciT130I, BptI, BseBI, BsiLI, BstNI, BstOI, EcoRII^, MvaI, Psp6I^, PspGI^ |
| Bst4CI | ACN/GT | HpyCH4III, TaaI, Tsp4CI |
| Bst6I | CTCTTC(1/4) | Eam1104I, EarI, Ksp632I |
| Bst71Ix | GCAGC(8/12) | BbvI, BseXI, BstV1I, R9896 |
| Bst98I | C/TTAAG | AflII, BfrI , BspTI, BstAFI, MspCI, Vha464I |
| BstACI | GR/CGYC | AcyI , BsaHI, BssNI, Hin1I, Hsp92I |
| BstAFI | C/TTAAG | AflII, BfrI , BspTI, Bst98I, MspCI, Vha464I |
| BstAPI | GCANNNN/NTGC | ApaBI^ |
| BstAUI | T/GTACA | AauI , Bsp1407I, BsrGI, SspBI |
| BstBAI | YAC/GTR | BsaAI, Ppu21I |
| BstBI | TT/CGAA | AsuII , Bpu14I, BsiCI, Bsp119I, BspT104I, Csp45I, NspV , SfuI |
| BstC8I | GCN/NGC | Cac8I |
| BstDEI | C/TNAG | DdeI, HpyF3I |
| BstDSI | C/CRYGG | BtgI, DsaI |
| BstEII | G/GTNACC | BstPI, Eco065I, Eco91I, EcoO65I, PspEI |
| BstENI | CCTNN/NNNAGG | EcoNI, XagI |
| BstENII | /GATC | BfuCI, BscFI, Bsp143I, BssMI, BstKTI^, BstMBI, DpnI^, Kzo9I, MalI^, MboI, NdeII^, Sau3AI |
| BstF5I | GGATG(2/0) | BseGI, BtsCI, FokI^ |
| BstFNI | CG/CG | AccII , Bsh1236I, BspFNI^, BstUI, FnuDII^, MvnI, ThaI |
| BstH2I | RGCGC/Y | Bfol, Bsp143II, HaeII |
| BstHHI | GCG/C | AspLEI , CfoI, GlaI^, HhaI, R9529^, HinP1I^, HspAI ^ |
| BstHPI | GTT/AAC | HpaI, KspAI |
| BstKTI | GAT/C | BfuCI^, BscFI^, Bsp143I^, BssMI^, BstENII^, BstMBI^, DpnI^, Kzo9I^, MalI^, MboI^, NdeII^, Sau3AI^ |
| BstMAI | GTCTC(1/5) | Alw26I , BcoDI, BsmAI, BsoMAI, Nt.BsmAI^ |
| BstMBI | /GATC | BfuCI, BscFI, Bsp143I, BssMI, BstENII, BstKTI^, DpnI^, Kzo9I, MalI^, MboI, NdeII^, Sau3AI |
| BstMCI | CGRY/CG | BsaOI, Bsh1285I , BsiEI, McrI |
| BstMWI | GCNNNNN/NNGC | HpyF10VI, MwoI |
| BstNI | CC/WGG | AjnI ^, BciT130I, BptI, BseBI, BsiLI, Bst2UI, BstOI, EcoRII^, MvaI, Psp6I ^, PspGI^ |
| BstNSI | RCATG/Y | NspI, XceI |
| BstOI | CC/WGG | AjnI ^, BciT130I, BptI, BseBI, BsiLI, Bst2UI, BstNI, EcoRII^, MvaI, Psp6I ^, PspGI^ |
| BstPAI | GACNN/NNGTC | BoxI, PshAI |
| BstPI | G/GTNACC | BstEII , Eco065I, Eco91I, Eco065I, PspEI |
| BstSCI | /CCNGG | Bme1390I^, BmrFI^, BssKI, MspR9I^, ScrFI^, StyD4I |
| BstSFI | C/TRYAG | BfmI, BpcI, SfcI |
| BstSLI | GKGCM/C | BaeGI, BseSI |
| BstSNI | TAC/GTA | Eco105I, SnaBI |
| BstUI | CG/CG | AccII , Bsh1236I, BspFNI^, BstFNI, FnuDII^, MvnI, ThaI |
| BstV1I | GCAGC(8/12) | BbvI, BseXI, Bst71I, R9896 |
| BstV2I | GAAGAC(2/6) | BbsI, BbvII , BpiI, BpuAI |
| BstX2I | R/GATCY | BstYI, MflI, PsuI, XhoII |
| BstXI | CCANNNNN/NTGG | |
| BstYI | R/GATCY | BstX2I, MflI, PsuI, XhoII |
| BstZ17I | GTA/TAC | BssNAI, Bst1107I, SnaI^ |
| BstZI | C/GGCCG | BseX3I , EagI, EclXI, Eco521, XmaIII |
| Bsu15I | AT/CGAT | BanIII , Bsa29I, BseCI, BshVI, BsiXI, Bsp106I, BspDI, BspXI, BsuTUI, ClaI, ZhoI |
| Bsu36I | CC/TNAGG | AocI , AxyI , Bse21I CvnI, Eco81I, SauI |
| BsuI | GTATCC(6/5) | BciVI, BfuI |
| BsuRI | GG/CC | AoxI^, BshFI , BsnI, BspANI, HaeIII, PalI , PhoI |
| BsuTUI | AT/CGAT | BanIII , Bsa29I, BseCI, BshVI, BsiXI, Bsp106I, BspDI, BspXI, Bsu15I, ClaI, ZhoI |
| BtgI | C/CRYGG | BstDSI, DsaI |
| BtgZI | GCGATG(10/14) | |
| BthCIx | GCNG/C | BisI ^, BlsI ^, Fnu4HI^, Fsp4HI^, GluI^, Ital ^, SatI^ |
| BtrI | CACGTC(-3/-3) | AjiI ^, BmgBI^ |
| BtsCI | GGATG(2/0) | BseGI, BstF5I, FokI^ |
| BtsI | GCAGTG(2/0) | Nb.BtsI^ |
| BtsIMutI | CAGTG(2/0) | |
| BtuMI | TCG/CGA | Bsp68I, NruI, RruI^ |
| BveI | ACCTGC(4/8) | Acc36I , BfuAI, BspMI |
| Cac8I | GCN/NGC | BstC8I |
| CaiI | CAGNNN/CTG | AlwNI, PstNI |
| CauIIx | CC/SGG | AsuC2I , BcnI , BpuMI, EcoHI^, NciI |
| CchIIIx | CCCAAG(20/18) | |
| CchIIx | GGARGA(11/9) | |
| CciI | T/CATGA | BspHI, PagI, RcaI |
| CciNI | GC/GGCCGC | NotI |
| CdiIx | CATCG(-1/-1) | |
| CdpI | GCGGAG(20/18) | |
| CelII | GC/TNAGC | BlpI, Bpu1102I, Bsp1720I, EspI |
| CfoI | GCG/C | AspLEI , BstHHI, GlaI^, HhaI, R9529^, HinP1I^, HspAI ^ |
| Cfr10I | R/CCGGY | Bse118I, BsrFI, BssAI |
| Cfr13I | G/GNCC | AspS9I , AsuI , BmgT120I^, BsiZI, BspLI^, FmuI^, PspPI , Sau96I, UnbI^ |
| Cfr42I | CCGC/GG | KspI, SacII, Sfr303I, SgrBI, SstII |
| Cfr9I | C/CCGGG | PspAI , SmaI^, TspMI, XmaCI |
| CfrIx | Y/GGCCR | AcoI , EaeI |
| Cgl13032IIx | ACGABGG | |
| Cgl13032Ix | GGCGCA | |
| CjeFIIIx | GCAAGG | |
| CjeFVx | GGRCA | |
| CjeIx | (8/14)CCANNNNNNGT(15/9) | |
| CjeNIIIx | GKAAYG(19/17) | |
| CjeNIIx | GAGNNNNNGT | |
| CjeP659IVx | CACNNNNNNNGAA | |
| CjePIx | (7/13)CCANNNNMNNTC(14/8) | |
| CjuIIx | CAYNNNNNCTC | |
| CjuIx | CAYNNNNNRTG | |
| ClaI | AT/CGAT | BanIII , Bsa29I, BseCI, BshVI, BsiXI, Bsp106I, BspDI, BspXI, Bsu15I, BsuTUI, ZhoI |
| CpoI | CG/GWCCG | |
| CseI | GACGC(5/10) | HgaI |
| CsiI | A/CCWGGT | MabI, SexAI |
| Csp45I | TT/CGAA | AsuII , Bpu14I, BsiCI, Bsp119I, BspT104I, BstBI, NspV , SfuI |
| Csp6I | G/TAC | AfaI ^, CviQI, RsaI^ |
| CspAI | A/CCGGT | AgeI, AsiAI , BshTI, PinAI |
| CspCI | (11/13)CAANNNNNGTGG(12/10) | |
| CspI | CG/GWCCG | |
| CstMIx | AAGGAG(20/18) | |
| CviAII | C/ATG | FaeI^, FatI^, Hin1II^, Hsp92II ^, NlaIII^ |
| CviJI | RG/CY | |
| CviKI-1 | RG/CY | CviJI^, CviTI^ |
| CviQI | G/TAC | AfaI ^, Csp6I, RsaI^ |
| CviRIx | TG/CA | HpyCH4V |
| CviTI | RG/CY | |
| CvnI | CC/TNAGG | AocI , AxyI , Bse21I Bsu36I, Eco81I, SauI |
| DdeI | C/TNAG | BstDEI, HpyF3I |
| DinI | GGC/GCC | BbeI ^, EgeI, KasI^, Mly113I^, NarI^, PluTI^, SfoI, SspDI^ |
| DpnI | GA/TC | BfuCI^, BscFI^, Bsp143I^, BssMI^, BstENII^, BstKTI^, BstMBI^, Kzo9I^, MalI^, MboI^, NdeII^, Sau3AI^ |
| DpnII | /GATC | BfuCI, BscFI, Bsp143I, BssMI, BstENII, BstKTI^, BstMBI, DpnI^, Kzo9I, MalI^, MboI, NdeII^, Sau3AI |
| DraI | TTT/AAA | AhaIII ^ |
| DraII | RG/GNCCY | EcoO109I, PssI^ |
| DraIII | CACNNN/GTG | AdeI |
| DraRIx | CAAGNAC(20/18) | |
| DrdI | GACNNNN/NNGTC | AasI , DseDI |
| DrdIIx | GAACCA | |
| DriI | GACNNN/NNGTC | AhdI, AspEI , BmeRI, Eam1105I, EclHKI, NruGI |
| DsaIx | C/CRYGG | BstDSI, BtgI |
| DseDI | GACNNNN/NNGTC | AasI , DrdI |
| DvuIIIx | CACNCAC | |
| EaeI | Y/GGCCR | AcoI , CfrI |
| EagI | C/GGCCG | BseX3I , BstZI, EclXI, Eco521, XmaIII |
| Eam1104I | CTCTTC(1/4) | Bst6I, EarI, Ksp632I |
| Eam1105I | GACNNN/NNGTC | AhdI, AspEI , BmeRI, DriI, EclHKI, NruGI |
| EarI | CTCTTC(1/4) | Bst6I, Eam1104I, Ksp632I |
| Ecil | GGCGGA(11/9) | |
| Ecl136II | GAG/CTC | Eco53kI, EcoICRI, Psp124BI ^, SacI^, SstI^ |
| EclHKI | GACNNN/NNGTC | AhdI, AspEI , BmeRI, DriI, Eam1105I, NruGI |
| EclXI | C/GGCCG | BseX3I , BstZI, EagI, Eco521, XmaIII |
| Eco065I | G/GTNACC | BstEII , BstPI, Eco9II, EcoO65I, PspEI |
| Eco105I | TAC/GTA | BstSNI, SnaBI |
| Eco130I | C/CWWGG | BssT1I, EcoT14I, ErhI , StyI |
| Eco147I | AGG/CCT | AatI , PceI , SseBI, StuI |
| Eco24I | GRGCY/C | BanII, EcoT38I, FriOI, HgiJII |
| Eco31I | GGTCTC(1/5) | BsaI, Bso31I, BspTNI |
| Eco32I | GAT/ATC | EcoRV |
| Eco47I | G/GWCC | AflI , AvaII, Bme18I, PspO3I^, SinI, Vpak11AI^ |
| Eco47III | AGC/GCT | AfeI, Aor51HI, FunI |
| Eco52I | C/GGCCG | BseX3I , BstZI, EagI, EclXI, XmaIII |
| Eco53kI | GAG/CTC | Ecl136II, EcoICRI, Psp124BI ^, SacI^, SstI^ |
| Eco57I | CTGAAG(16/14) | AcuI |
| Eco57MIx | CTGRAG(16/14) | |
| Eco72I | CAC/GTG | AcvI , BbrPI , PmaCI , PmlI, PspCI |
| Eco81I | CC/TNAGG | AocI , AxyI , Bse21I Bsu36I, CvnI, SauI |
| Eco88I | C/YCGRG | Ama87I , AvaI, BcoI , BmeT110I, BsiHKCI, BsoBI, NspIII |
| Eco91I | G/GTNACC | BstEII , BstPI, Eco065I, EcoO65I, PspEI |
| EcoHIx | /CCSGG | AsuC2I ^, BcnI ^, BpuMI^, CauII^, NciI^ |
| EcoICRI | GAG/CTC | Ecl136II, Eco53kI, Psp124BI ^, SacI^, SstI^ |
| EcoNI | CCTNN/NNNAGG | BstENI, XagI |
| EcoO109I | RG/GNCCY | DraII, PssI^ |
| EcoO65I | G/GTNACC | BstEII , BstPI, Eco065I, Eco9II, PspEI |
| EcoP15I | CAGCAG(25/27) | |
| EcoRI | G/AATTC | FunII |
| EcoRII | /CCWGG | AjnI ^, BciT130I^, BptI^, BseBI^, BsiLI^, Bst2UI^, BstNI^, BstOI^, MvaI^, Psp6I , PspGI |
| EcoRV | GAT/ATC | Eco32I |
| EcoT14I | C/CWWGG | BssT1I, Eco130I, ErhI , StyI |
| EcoT22I | ATGCA/T | AvaIII ^, BfrB1, Mph1103I, NsiI, Zsp2I |
| EcoT38I | GRGCY/C | BanII, Eco24I, FriOI, HgiJII |
| EgeI | GGC/GCC | BbeI ^, DinI, KasI^, Mly113I^, NarI^, PluTI^, SfoI, SspDI^ |
| EheI | GGC/GCC | BabeI ^, DinI, EgeI, KasI^, Mly113I^, NarI^, PluTI^, SfoI, SspDI^ |
| ErhI | C/CWWGG | BssT1I, Eco130I, EcoT14I, StyI |
| EsaSSIx | GACCAC | |
| Esp3I | CGTCTC(1/5) | BsmBI |
| EspIx | GC/TNAGC | BlpI, Bpu1102I, Bsp1720I, CelII |
| FaeI | CATG/ | CviAII^, FatI^, Hin1II, Hsp92II , NlaIII |
| FaiI | YA/TR | |
| FalI | (8/13)AAGNNNNNCTT(13/8) | |
| FaqI | GGGAC(10/14) | BslFI, BsmFI, FinI^ |
| Fatl | /CATG | CviAII^, FaeI^, Hin1II^, Hsp92II ^, NlaIII^ |
| FauI | CCCGC(4/6) | SmuI |
| FauNDI | CA/TATG | NdeI |
| FbaI | T/GATCA | BelI, BsiQI, Ksp22I |
| FblI | GT/MKAC | AccI, XmiI |
| FinIx | GGGAC | BslFI^, BsmFI^, FaqI^ |
| FmuIx | GGNC/C | AspS9I ^, AsuI ^, BmgT120I^, BsiZI^, BspLI^, Cfr13I^, PspPI ^, Sau96I^, UnbI^ |
| Fnu4HI | GC/NGC | BisI , BlsI ^, BthCI^, Fsp4HI, GluI, ItaI , SatI |
| FnuDIIx | CG/CG | AccII ^, Bsh1236I^, BspFNI, BstFNI^, BstUI^, MvnI^, ThaI^ |
| FokI | GGATG(9/13) | BseGI^, BstF5I^, BtsCI^ |
| FriOI | GRGCY/C | BanII, Eco24I, EcoT38I, HgiJII |
| FseI | GGCCGG/CC | RigI |
| Fsp4HI | GC/NGC | BisI , BlsI ^, BthCI^, Fnu4HI, GluI, ItaI , SatI |
| FspAI | RTGC/GCAY | |
| FspBI | C/TAG | BfaI, MaeI, XspI |
| FspEI | CC(12/16) | |
| FspI | TGC/GCA | Acc16I , AviII , MstI, NsbI |
| FunI | AGC/GCT | AfeI, Aor51HI , Eco47III |
| FunII | G/AATTC | EcoRI |
| GauT27Ix | CGCGCAGG | |
| GdiIIx | CGGCCR(-5/-1) | |
| GlaI | GC/GC | AspLEI ^, BstHHI^, CfoI^, HhaI^, R9529^, HinP1I^, HspAI ^ |
| GluI | GC/NGC | BisI , BlsI ^, BthCI^, Fnu4HI, Fsp4HI, ItaI , SatI |
| GsaI | CCCAGC(-1/-5) | BseYI^ |
| GsuI | CTGGAG(16/14) | BpmI |
| HaeII | RGCGC/Y | BfoI, Bsp143II, BstH2I |
| HaeIII | GG/CC | AoxI^, BshFI , BsnI, BspANI, BsuRI, PalI , PhoI |
| HaeIVx | (7/13)GAYNNNNNRTC(14/9) | |
| HaeIx | WGG/CCW | |
| HapII | C/CGG | BsiSI, HpaII, MspI |
| HgaI | GACGC(5/10) | CseI |
| HgiAIx | GWGCW/C | Alw21I , AspHI , Bbv12I , BsiHKAI |
| HgiCIx | G/GYRCC | AccB1I , BanI, BshNI, BspT107I |
| HgiEIIx | ACCNNNNNNGGT | |
| HgiJIIx | GRGCY/C | BanII, Eco24I, EcoT38I, FriOI |
| HhaI | GCG/C | AspLEI , BstHHI, CfoI, GlaI^, R9529^, HinP1I^, HspAI ^ |
| Hin1I | GR/CGYC | AcyI , BsaHI, BssNI, BstACI, Hsp92I |
| Hin1II | CATG/ | CviAII^, FaeI, FatI^, Hsp92II , NlaIII |
| Hin4IIx | CCTTC(6/5) | HpyAV |
| Hin4Ix | (8/13)GAYNNNNNVTC(13/8) | |
| Hin6I | G/CGC | AspLEI ^, BstHHI^, CfoI^, GlaI^, HhaI^, R9529, HinP1I, HspAI |
| HincII | GTY/RAC | |
| HindII | GTY/RAC | |
| HindIII | A/AGCTT | |
| HinfI | G/ANTC | |
| HinP1I | G/CGC | AspLEI ^, BstHHI^, CfoI^, GlaI^, HhaI^, R9529, HspAI |
| HpaI | GTT/AAC | BstHPI, KspAI |
| HpaII | C/CGG | BsiSI, HapII, MspI |
| HphI | GGTGA(8/7) | AsuHPI |
| Hpy166II | GTN/NAC | Hpy8I^, MjaIV^ |
| Hpy178IIIx | TC/NNGA | Hpy188III |
| Hpy188I | TCN/GA | |
| Hpy188III | TC/NNGA | Hpy178III |
| Hpy8I | GTN/NAC | Hpy166II^, MjaIV^ |
| Hpy99I | CGWCG/ | |
| Hpy99XIIIx | GCCTA | |
| HpyAV | CCTTC(6/5) | Hin4II |
| HpyCH4III | ACN/GT | Bst4CI, TaaI, Tsp4CI |
| HpyCH4IV | A/CGT | HpySE526I, MaeII, TaiI^, TscI^ |
| HpyCH4V | TG/CA | CviRI |
| HpyF10VI | GCNNNNN/NNGC | BstMWI, MwoI |
| HpyF3I | C/TNAG | BstDEI, DdeI |
| HpySE526I | A/CGT | HpyCH4IV, MaeII, TaiI^, TscI^ |
| Hsp92I | GR/CGYC | AcyI , BsaHI, BssNI, BstACI, Hin1I |
| Hsp92II | CATG/ | CviAII^, FaeI, FatI^, Hin1II, NlaIII |
| HspAI | G/CGC | AspLEI ^, BstHHI^, CfoI^, GlaI^, HhaI^, R9529, HinP1I |
| I-CeuI | TAACTATAACGGTCCTAAGGTAGCGAA(-9/- 13) | |
| I-SceI | TAGGGATAACAGGGTAAT(-9/-13) | |
| ItaI | GC/NGC | BisI , BlsI ^, BthCI^, Fnu4HI, Fsp4HI, GluI, SatI |
| KasI | G/GCGCC | BbeI ^, DinI^, EgeI^, Mly113I^, NarI^, PluTI^, SfoI^, SspDI |
| KflI | GG/GWCCC | |
| Kpn2I | T/CCGGA | AccIII, Aor13HI , BlfI , BseAI, BsiMI, Bsp13I, BspEI, BspMII, MroI |
| KpnI | GGTAC/C | Acc65I^, Asp7181 ^ |
| KroI | G/CCGGC | MroNI, NaeI^, NgoAIV, NgoMIV, PdiI ^ |
| Ksp22I | T/GATCA | BclI, BsiQI, FbaI |
| Ksp632Ix | CTCTTC(1/4) | Bst6I, Eam1104I, EarI |
| Ksp632Ix | CTCTTC(1/4) | Bst6I, Eam1104I, EarI, Ksp632I |
| KspAI | GTT/AAC | BstHPI, HpaI |
| KspI | CCGC/GG | Cfr42I, SacII, Sfr303I, SgrBI, SstII |
| Kzo9I | /GATC | BfuCI, BscFI, Bsp143I, BssMI, BstENII, BstKTI^, BstMBI, DpnI^, MalI^, MboI, NdeII^, Sau3AI |
| LguI | GCTCTTC(1/4) | BspQI, Nt.BspQI^, PciSI , SapI |
| LlaGIx | CTNGAYG | |
| LpnPI | CCDG(10/14) | |
| LweI | GCATC(5/9) | BmsI, SfaNI |
| MabI | A/CCWGGT | CsiI, SexAI |
| MaeI | C/TAG | BfaI, FspBI, XspI |
| MaeII | A/CGT | HpyCH4IV, HpySE526I, TaiI^, TscI^ |
| MaeIII | /GTNAC | |
| MalI | GA/TC | BfuCI^, BscFI^, Bsp143I^, BssMI^, BstENII^, BstKTI^, BstMBI^, DpnI^, Kzo9I^, MboI^, NdeII^, Sau3AI^ |
| MamI | GATNN/NNATC | BsaBI, Bse8I, BseJI, BsiBI, BsrBRI |
| MaqIx | CRTTGAC(21/19) | |
| MauBI | CG/CGCGCG | |
| NbiI | CCGCTC(-3/-3) | AccBSI, BsrBI |
| MboI | /GATC | BfuCI, BscFI, Bsp143I, BssMI, BstENII, BstKTI^, BstMBI, DpnI^, Kzo9I, MalI^, NdeII^, Sau3AI |
| MboII | GAAGA(8/7) | |
| McrIx | CGRY/CG | BsaOI, Bsh12851 , BsiEI, BstMCI |
| MfeI | C/AATTG | MunI |
| MflI | R/GATCY | BstX2I, BstYI, PsuI, XhoII |
| MhlI | GDGCH/C | BmyI, Bsp1286I, SduI |
| MjaIVx | GTNNAC | Hpy166II^, Hpy8I^ |
| MlsI | TGG/CCA | BalI , MluNI, MscI, Msp20I |
| MluCI | /AATT | Sse9I, TasI, Tsp509I, TspEI |
| MluI | A/CGCGT | |
| MluNI | TGG/CCA | BalI , MlsI, MscI, Msp20I |
| Mly113I | GG/CGCC | BbeI^, DinI^, EgeI^, KasI^, NarI, PluTI^, SfoI^, SspDI^ |
| MlyI | GAGTC(5/5) | Nt.BstNBI^, PleI^, PpsI ^, SchI |
| MmeI | TCCRAC(20/18) | |
| MnlI | CCTC(7/6) | |
| Mph1103I | ATGCA/T | AvaIII ^, BfrBl, EcoT22I, NsiI, Zsp2I |
| MreI | CG/CCGGCG | |
| MroI | T/CCGGA | AccIII, Aor13HI , BlfI , BseAI, BsiMI, Bsp13I, BspEI, BspMII, Kpn2I |
| MroNI | G/CCGGC | KroI, NaeI^, NgoAIV, NgoMIV, PdiI ^ |
| MroXI | GAANN/NNTTC | Asp700I , PdmI , XmnI |
| MscI | TGG/CCA | BalI , MlsI, MluNI, Msp20I |
| MseI | T/TAA | SaqAI, Tru1I |
| MslI | CAYNN/NNRTG | RseI^, SmiMI |
| Msp20I | TGG/CCA | BalI , MlsI, MluNI, MscI |
| MspA1I | CMG/CKG | NspBII |
| MspCI | C/TTAAG | AflII, BfrI , BspTI, Bst98I, BstAFI, Vha464I |
| MspI | C/CGG | BsiSI, HapII, HpaII |
| MspJI | CNNR(9/13) | |
| MspR9I | CC/NGG | Bme1390I, BmrFI, BssKI^, BstSCI^, ScrFI, StyD4I^ |
| MssI | GTTT/AAAC | PmeI |
| MstIx | TGC/GCA | Acc16I , AviII , FspI, NsbI |
| MunI | C/AATTG | MfeI |
| Mva1269I | GAATGC(1/-1) | BsaMI, BscCI, BsmI, Nb.BsmI^, PctI |
| MvaI | CC/WGG | AjnI^, BciT130I, BptI, BseBI, BsiLI, Bst2UI, BstNI, BstOI, EcoRII^, Psp6I ^, PspGI^ |
| MvnI | CG/CG | AccII , Bsh1236I, BspFNI^, BstFNI, BstUI, FnuDII^, ThaI |
| MvrI | CGAT/CG | BpvUI, BspCI, Ple19I , PvuI |
| MwoI | GCNNNNN/NNGC | BstMWI, HpyF10VI |
| NaeI | GCC/GGC | KroI^, MroNI^, NgoAIV^, NgoMIV^, PdiI |
| NarI | GG/CGCC | BbeI^, DinI^, EgeI^, KasI^, Mly113I, PluTI^, SfoI^, SspDI^ |
| Nb.BbvCI | CCTCAGC | BbvCI^, Nt.BbvCI^ |
| Nb.BsmI | GAATGC | BsaMI^, BscCI^, BsmI^, Mva1269I^, PctI ^ |
| Nb.BsrDI | GCAATG | Bse3DI^, BseMI^, BsrDI^ |
| Nb.BtsI | GCAGTG | BtsI^ |
| NciI | CC/SGG | AsuC2I , BcnI , BpuMI, CauII, EcoHI^ |
| NcoI | C/CATGG | Bsp19I |
| NdeI | CA/TATG | FauNDI |
| NdeII | /GATC | BfuCI^, BscFI^, Bsp143I^, BssMI^, BstENII^, BstKTI^, BstMBI^, DpnI^, Kzo9I^, MalI^, MboI^, Sau3AI^ |
| NgoAIV | G/CCGGC | KroI, MroNI, NaeI^, NgoMIV, PdiI ^ |
| NgoAVIIIx | (12/14)GACNNNNNTGA(13/11) | |
| NgoMIV | G/CCGGC | KroI, MroNI, NaeI^, NgoAIV, PdiI ^ |
| NhaXIx | CAAGRAG | |
| NheI | G/CTAGC | AsuNHI , BmtI^, BspOI^ |
| NlaCIx | CATCAC(19/17) | |
| NlaIII | CATG/ | CviAII^, FaeI, FatI^, Hin1II, Hsp92II |
| NlaIV | GGN/NCC | BmiI, BscBI, PspN4I |
| NmeAIII | GCCGAG(21/19) | |
| NmuCI | /GTSAC | TseFI^, Tsp45I |
| NotI | GC/GGCCGC | CciNI |
| NruGI | GACNNN/NNGTC | AhdI, AspEI , BmeRI, DriI, Eam1105I, EclHKI |
| NruI | TCG/CGA | Bsp68I, BtuMI, RruI^ |
| NsbI | TGC/GCA | Acc16I , AviII , FspI, MstI |
| NsiI | ATGCA/T | AvaIII ^, BfrBl, EcoT22I, Mph1103I, Zsp2I |
| NspBII x | CMG/CKG | MspA1I |
| NspI | RCATG/Y | BstNSI, XceI |
| NspIII | C/YCGRG | Ama87I , AvaI, BcoI , BmeT110I, BsiHKCI, BsoBI, Eco88I |
| NspV | TT/CGAA | AsuII , Bpu14I, BsiCI, Bsp119I, BspT104I, BstBI, Csp45I, SfuI |
| Nt.AlwI | GGATC(4/-5) | AclWI ^, AlwI^, BinI^, BspPI^ |
| Nt.BbvCI | CCTCAGC(-5/-7) | BbvCI^, Nb.BbvCI^ |
| Nt.BsmAI | GTCTC(1/-5) | Alw26I ^, BcoDI^, BsmAI^, BsoMAI^, BstMAI^ |
| Nt.BspQI | GCTCTTC(1/-7) | BspQI^, LguI^, PciSI ^, SapI^ |
| Nt.BstNBI | GAGTC(4/-5) | MlyI^, PleI^, PpsI ^, SchI^ |
| Nt. CviPII | (0/-1)CCD | |
| OliI | CACNN/NNGTG | AleI |
| PacI | TTAAT/TAA | |
| PaeI | GCATG/C | BbuI , SpaHI, SphI-HF® |
| PaeR7I | C/TCGAG | BssHI, Sfr274I, SlaI, StrI, TliI, XhoI |
| PagI | T/CATGA | BspHI, CciI, RcaI |
| PAlAI | GG/CGCGCC | AscI, SgsI |
| PalI | GG/CC | AoxI^, BshFI , BsnI, BspANI, BsuRI, HaeIII, PhoI |
| PasI | CC/CWGGG | |
| PauI | G/CGCGC | BsePI, BssHII, PteI |
| PceI | AGG/CCT | AatI , Eco147I, SseBI, StuI |
| PciI | A/CATGT | BspLU11I, PscI |
| PciSI | GCTCTTC(1/4) | BspQI, LguI, Nt.BspQI^, SapI |
| PcsI | WCGNNNN/NNNCGW | |
| PctI | GAATGC(1/-1) | BsaMI, BscCI, BsmI, Mva1269I, Nb.BsmI^ |
| PdiI | GCC/GGC | KroI^, MroNI^, NaeI, NgoAIV^, NgoMIV^ |
| PdmI | GAANN/NNTTC | Asp700I , MroXI, XmnI |
| PenIx | GCAGT | |
| PfeI | G/AWTC | TfiI |
| Pfl1108I x | TCGTAG | |
| Pfl23II | C/GTACG | BsiWI, PspLI , SplI, SunI |
| PflBI | CCANNNN/NTGG | AccB7I , BasI , PflMI, Van91I |
| PflFI | GACN/NNGTC | AspI , PsyI, TelI, Tth111I |
| PflMI | CCANNNN/NTGG | AccB7I , BasI , PflBI , Van91I |
| PfoI | T/CCNGGA | |
| PhoI | GG/CC | AoxI^, BshFI , BsnI, BspANI, BsuRI, HaeIII, PalI |
| PinAI | A/CCGGT | AgeI, AsiAI , BshTI, CspAI |
| PI-PspI | TGGCAAACAGCTATTATGGGTATTATGGGT(-13/-17) | |
| PI-SceI | ATCTATGTCGGGTGCGGAGAAAGAGGTAAT(-15/-19) | |
| PlaDIx | CATCAG(21/19) | |
| Ple19I | CGAT/CG | BpvUI, BspCI, MvrI, PvuI |
| PleI | GAGTC(4/5) | MlyI^, Nt.BstNBI^, PpsI , SchI^ |
| PluTI | GGCGC/C | BbeI , DinI^, EgeI^, KasI^, Mly113I^, NarI^, SfoI^, SspDI^ |
| PmaCI | CAC/GTG | AcvI , BbrPI , Eco72I, PmlI, PspCI |
| PmeI | GTTT/AAAC | MssI |
| PmlI | CAC/GTG | AcvI , BbrPI , Eco72I, PmaCI , PspCI |
| PpiI x | (7/12)GAACNNNNNCTC(13/8) | |
| PpsI | GAGTC(4/5) | MlyI^, Nt.BstNBI^, PleI, SchI^ |
| Ppu21I | YAC/GTR | BsaAI, BstBAI |
| PpuMI | RG/GWCCY | Psp5II |
| PpuXI | RG/GWCCY | PpuMI, Psp5II |
| PscI | A/CATGT | BspLU11I, PciI |
| PshAI | GACNN/NNGTC | BoxI, BstPAI |
| PshBI | AT/TAAT | AseI, AsnI , VspI |
| PsiI | TTA/TAA | AanI |
| Psp124BI | GAGCT/C | Ecl136II^, Eco53kI^, EcoICRI^, SacI, SstI |
| Psp1406I | AA/CGTT | AclI |
| Psp5II | RG/GWCCY | PpuMI |
| Psp6I | /CCWGG | AjnI ^, BciT130I^, BptI^, BseBI^, BsiLI^, Bst2UI^, BstNI^, BstOI^, EcoRII, MvaI^, PspGI |
| PspAI | C/CCGGG | Cfr9I, SmaI^, TspMI, XmaCI |
| PspCI | CAC/GTG | AcvI , BbrPI , Eco72I, PmaCI , PmlI |
| PspEI | G/GTNACC | BstEII , BstPI, Eco0651, Eco91I, EcoO65I |
| PspGI | /CCWGG | AjnI ^, BciT130I^, BptI^, BseBI^, BsiLI^, Bst2UI^, BstNI^, BstOI^, EcoRII, MvaI^, Psp6I |
| PspLI | C/GTACG | BsiWI, Pfl23II , SplI, SunI |
| PspN4I | GGN/NCC | BmiI, BscBI, NlaIV |
| PspO3Ix | GGWC/C | AflI ^, AvaII^, Bme18I^, Eco47I^, SinI^, Vpak11AI^ |
| PspOMI | G/GGCCC | ApaI^, Bsp120I |
| PspOMIIx | CGCCCAR(20/18) | |
| PspPI | G/GNCC | AspS9I , AsuI , BmgT120I^, BsiZI, BspLI^, Cfr13I, FmuI^, Sau96I, UnbI^ |
| PspPRIx | CCYCAG(15/13) | |
| PspXI | VC/TCGAGB | |
| PsrI | (7/12)GAACNNNNNNTAC(12/7) | |
| PssIx | RGGNC/CY | DraII^, EcoO109I^ |
| PstI | CTGCA/G | BspMAI |
| PstNI | CAGNNN/CTG | AlwNI, CaiI |
| PsuI | R/GATCY | BstX2I, BstYI, MflI, XhoII |
| PsyI | GACN/NNGTC | AspI , PflFI, TelI, Tth111I |
| PteI | G/CGCGC | BsePI, BssHII, PauI |
| PvuI | CGAT/CG | BpvUI, BspCI, MvrI, Ple19I |
| PvuII | CAG/CTG | |
| R9529 | G/CGC | AspLEI ^, BstHHI^, CfoI^, GlaI^, HhaI^, HinP1I, HspAI |
| R9896 | GCAGC(8/12) | BbvI, BseXI, Bst71I, BstV1I |
| RcaI | T/CATGA | BspHI, CciI, PagI |
| RceIx | CATCGAC(20/18) | |
| RdeGBIIIx | (9/11)TGRYCA(11/9) | |
| RdeGBIIx | ACCCAG(20/18) | |
| RdeGBIx | CCGCAG | |
| RflFIIIx | CGCCAG | |
| RgaI | GCGAT/CGC | AsiSI, SfaAI, SgfI |
| RigI | GGCCGG/CC | FseI |
| RlaIx | VCW | |
| RleAIx | CCCACA(12/9) | |
| RpaB5Ix | CGRGGAC(20/18) | |
| RpaBIx | CCCGCAG(20/18) | |
| RpaIx | GTYGGAG(11/9) | |
| RpaTIx | GRTGGAG | |
| RruI | TCG/CGA | Bsp68I^, BtuMI^, NruI^ |
| RsaI | GT/AC | AfaI , Csp6I^, CviQI^ |
| RsaNI | G/TAC | AfaI ^, Csp6I, CviQI, RsaI^ |
| RseI | CAYNN/NNRTG | MslI^, SmiMI^ |
| Rsr2I | CG/GWCCG | |
| RsrII | CG/GWCCG | CpoI, CspI, Rsr2I |
| SacI | GAGCT/C | Ecl136II^, Eco53kI^, EcoICRI^, Psp124BI , SstI |
| SacII | CCGC/GG | Cfr42I, KspI, Sfr303I, SgrBI, SstII |
| SalI | G/TCGAC | |
| SanDIx | GG/GWCCC | |
| SapI | GCTCTTC(1/4) | BspQI, LguI, Nt.BspQI^, PciSI |
| SaqAI | T/TAA | MseI, Tru1I |
| SatI | GC/NGC | BisI , BlsI ^, BthCI^, Fnu4HI, Fsp4HI, GluI, ItaI |
| Sau3AI | /GATC | BfuCI, BscFI, Bsp143I, BssMI, BstENII, BstKTI^, BstMBI, DpnI^, Kzo9I, MalI^, MboI, NdeII^ |
| Sau96I | G/GNCC | AspS9I , AsuI , BmgT120I^, BsiZI, BspLI^, Cfr13I, FmuI^, PspPI , UnbI^ |
| SauIx | CC/TNAGG | AocI , AxyI , Bse21I, Bsu36I, CvnI, Eco81I |
| SbfI | CCTGCA/GG | SdaI, Sse8387I |
| ScaI | AGT/ACT | Acc113I , AssI , BmcAI^, ZrmI |
| SchI | GAGTC(5/5) | MlyI, Nt.BstNBI^, PleI^, PpsI ^ |
| ScrFI | CC/NGG | Bme1390I, BmrFI, BssKI^, BstSCI^, MspR9I, StyD4I^ |
| SdaI | CCTGCA/GG | SbfI, Sse8387I |
| SdeAIx | CAGRAG(21/19) | |
| SdeOSIx | (11/-1)GACNNNNRTGA(12/-11) | |
| SduI | GDGCH/C | BmyI, Bsp1286I, MhlI |
| SecIx | C/CNNGG | BsaJI, BseDI, BssECI |
| SetI | ASST/ | |
| SexAI | A/CCWGGT | CsiI, MabI |
| SfaAI | GCGAT/CGC | AsiSI, RgaI, SgfI |
| SfaNI | GCATC(5/9) | BmsI, LweI |
| SfcI | C/TRYAG | BfmI, BpcI, BstSFI |
| SfeIx | C/TRYAG | BfmI , BpcI, BstSFI, SfcI |
| SfiI | GGCCNNNN/NGGCC | |
| SfoI | GGC/GCC | BabeI ^, DinI, EgeI, KasI^, M1y113I^, NarI^, PluTI^, SspDI^ |
| Sfr274I | C/TCGAG | BssHI, PaeR7I, SlaI, StrI, TliI, XhoI |
| Sfr303I | CCGC/GG | Cfr42I, KspI, SacII, SgrBI, SstII |
| SfuI | TT/CGAA | AsuII , Bpu14I, BsiCI, Bsp119I, BspT104I, BstBI, Csp45I, NspV |
| SgeI | CNNGNNNNNNNNN/NNNN | |
| SgfI | GCGAT/CGC | AsiSI, RgaI, SfaAI |
| SgrAI | CR/CCGGYG | |
| SgrBI | CCGC/GG | Cfr42I, KspI, SacII, Sfr303I, SstII |
| SgrDI | CG/TCGACG | |
| SgrTIx | CCDS(10/14) | |
| SgsI | GG/CGCGCC | AscI, PAlAI |
| SimIx | GGGTC(-3/0) | |
| SinI | G/GWCC | AflI , AvaII, Bme18I, Eco47I, PspO3I^, Vpak11AI^ |
| SlaI | C/TCGAG | BssHI, PaeR7I, Sfr274I, StrI, TliI, XhoI |
| SmaI | CCC/GGG | Cfr9I^, PspAI ^, TspMI^, XmaCI^ |
| SmiI | ATTT/AAAT | SwaI |
| SmiMI | CAYNN/NNRTG | MslI, RseI^ |
| SmlI | C/TYRAG | |
| SmoI | C/TYRAG | |
| SmuI | CCCGC(4/6) | FauI |
| SnaBI | TAC/GTA | BstSNI, Eco105I |
| SnaIx | GTATAC | BssNAI^, Bst1107I^, BstZ17I^ |
| Sno506Ix | GGCCGAG | |
| SpaHI | GCATG/C | BbuI , PaeI , SphI-HF® |
| SpeI | A/CTAGT | AhLlI , BcuI |
| SphI | GCATG/C | BbuI , PaeI , SpaHI, SphI-HF® |
| SplIx | C/GTACG | BsiWI, Pfl23II , PspLI , SunI |
| SpoDIx | GCGGRAG | |
| SrfIx | GCCC/GGGC | |
| Sse232Ix | CG/CCGGCG | |
| Sse8387I | CCTGCA/GG | SbfI, SdaI |
| Sse8647Ix | AG/GWCCT | |
| Sse9I | /AATT | MluCI, TasI, Tsp509I, TspEI |
| SseBI | AGG/CCT | AatI , Eco147I, PceI , StuI |
| SsiI | CCGC(-3/-1) | AciI, BspACI |
| SspBIx | T/GTACA | AauI , Bsp1407I, BsrGI, BstAUI |
| SspDI | G/GCGCC | BbeI^, DinI^, EgeI^, KasI, Mly113I^, NarI^, PluTI^, SfoI^ |
| SspI | AAT/ATT | |
| SstE37Ix | CGAAGAC(20/18) | |
| SstI | GAGCT/C | Ecl136II^, Eco53kI^, EcoICRI^, Psp124BI , SacI |
| SstII | CCGC/GG | Cfr42I, KspI, SacII, Sfr303I, SgrBI |
| Sth132Ix | CCCG(4/8) | |
| StrI | C/TCGAG | BssHI, PaeR7I, Sfr274I, SlaI, TliI, XhoI |
| StuI | AGG/CCT | AatI , Eco147I, PceI , SseBI |
| StyD4I | /CCNGG | Bme1390I^, BmrFI^, BssKI, BstSCI, MspR9I^, ScrFI^ |
| StyI | C/CWWGG | BssT1I, Eco130I, EcoT 14I, ErhI |
| SunI | C/GTACG | BsiWI, Pfl23II , PspLI , SplI |
| SwaI | ATTT/AAAT | SmiI |
| TaaI | ACN/GT | Bst4CI, HpyCH4III, Tsp4CI |
| TaiI | ACGT/ | HpyCH4IV^, HpySE526I^, MaeII^, TscI |
| TaqI | T/CGA | Taq^{α}I, TthHB8I |
| TaqII | GACCGA(11/9) | |
| Taq^{α}I | T/CGA | TthHB8I |
| TasI | /AATT | MluCI, Sse9I, Tsp509I, TspEI |
| TatI | W/GTACW | |
| TauI | GCSG/C | |
| TelI | GACN/NNGTC | AspI , PflFI, PsyI, Tth111I |
| TfiI | G/AWTC | PfeI |
| ThaI | CG/CG | AccII , Bsh1236I, BspFNI^, BstFNI, BstUI, FnuDII^, MvnI |
| TliI | C/TCGAG | BssHI, PaeR7I, Sfr274I, SlaI, StrI, XhoI |
| Tru1I | T/TAA | MseI, SaqAI |
| Tru9I | T/TAA | MseI, SaqAI, Tru1I |
| TscAI | NNCASTGNN/ | TspRI |
| TscI | ACGT/ | HpyCH4IV^, HpySE526I^, MaeII^, TaiI |
| TseFI | GTSAC/ | NmuCI^, Tsp45I^ |
| TseI | G/C WGC | ApeKI |
| TsoIx | TARCCA(11/9) | |
| Tsp45I | /GTSAC | NmuCI, TseFI^ |
| Tsp4CIx | ACN/GT | Bst4CI, HpyCH4III, TaaI |
| Tsp509I | /AATT | MluCI, Sse9I, TasI, TspEI |
| TspDTI | ATGAA(11/9) | |
| TspEIx | /AATT | MluCI, Sse9I, TasI, Tsp509I |
| TspEIx | /AATT | MluCI, Sse9I, TasI, Tsp509I, TspEI |
| TspGWI | ACGGA(11/9) | |
| TspMI | C/CCGGG | Cfr9I, PspAI , SmaI^, XmaCI |
| TspRI | NNCASTGNN/ | TscAI |
| TssIx | GAGNNNCTC | |
| TstIx | (8/13)CACNNNNNNTCC(12/7) | |
| TsuIk | GCGAC | |
| Tth111I | GACN/NNGTC | AspI , PflFI, PsyI, TelI |
| Tth111IIx | CAARCA(11/9) | |
| TthHB8I | T/CGA | Taq^{α}I |
| UbaF11Ix | TCGTA | |
| UbaF12Ix | CTACNNNGTC | |
| UbaF13Ix | GAGNNNNNNCTGG | |
| UbaF14Ix | CCANNNNNTCG | |
| UbaF9Ix | TACNNNNNRTGT | |
| UbaPIx | CGAACG | |
| UnbIx | /GGNCC | AspS9I^, AsuI^, BmgT120I^, BsiZI^, BspLI^, Cfr13I^, FmuI^, PspPI^, Sau96I^ |
| Van91I | CCANNNN/NTGG | AccB7I , BasI , PflBI , PflMI |
| Vha464I | C/TTAAG | AflII, BfrI , BspTI, Bst98I, BstAFI, MspCI |
| VneI | G/TGCAC | Alw44I, ApaLI |
| Vpak11AIx | /GGWCC | AflI^, AvaII^, Bme18I^, Eco47I^, PspO3I^, SinI^ |
| VpaK11BI | G/GWCC | AflI , AvaII, Bme18I, Eco47I, PspO3I^, SinI, Vpak11AI^ |
| VspI | AT/TAAT | AseI, AsnI , PshBI |
| WviIx | CACRAG(20/18) | |
| XagI | CCTNN/NNNAGG | BstENI, EcoNI |
| XapI | R/AATTY | AcsI , ApoI |
| XbaI | T/CTAGA | |
| XceI | RCATG/Y | BstNSI, NspI |
| XcmI | CCANNNNN/NNNNTGG | |
| XhoI | C/TCGAG | BssHI, PaeR7I, Sfr274I, SlaI, StrI, TliI |
| XhoII | R/GATCY | BstX2I, BstYI, MflI, PsuI |
| XmaCI | C/CCGGG | Cfr9I, PspAI , SmaI^, TspMI |
| XmaI | C/CCGGG | Cfr9I, PspAI , SmaI^, TspMI, XmaCI |
| XmaIIIx | C/GGCCG | BseX3I , BstZI, EagI, EclXI, Eco52I |
| XmaJI | C/CTAGG | AspA2I , AvrII, BlnI , BspA2I |
| XmiI | GT/MKAC | AccI, FblI |
| XmnI | GAANN/NNTTC | Asp700I , MroXI, PdmI |
| XspI | C/TAG | BfaI, FspBI, MaeI |
| ZhoI | AT/CGAT | BanIII , Bsa29I, BseCI, BshVI, BsiXI, Bsp106I, BspDI, BspXI, Bsu15I, BsuTUI, ClaI |
| ZraI | GAC/GTC | AatII^ |
| ZrmI | AGT/ACT | Acc113I , AssI , BmcAI^, ScaI |
| Zsp2I | ATGCA/T | AvaIII^, BfrBl, EcoT22I, Mph1103I, NsiI |

The nucleic acid sequence "N₁N₂N₃N₄Nₙ" that is used as a fingerprint for labelling the antibody of interest. The DNA barcode is thus an oligonucleotide of a predefined sequence. According to the invention, the DNA barcode comprises at least 4 nucleotides. DNA barcoding provides the advantage to allow creating an indefinite number of combinations that will lead to a very high specific detection of the antigen and finally will allow using a plurality of DNAbs in multiplex assays. For example if the DNA barcode comprises 9 nucleotides, a combination of 4^{9 (}= 262144) of markers is possible. Preferably, a barcode is made with a specific nucleotide sequence having 4 (i.e., a virtual sequence), 4, 5, 6, or more base pairs in length. The length of a barcode may be increased along with the maximum sequencing length of a sequencer.

In one aspect of any of the embodiments herein, n may be an integer from among the range of 5 to 50. In one embodiment n is an integer from among the range of 5 to 30. In one embodiment n is an integer from among the range of 5 to 20. In one embodiment n is an integer from among the range of 5 to 15. In one embodiment n is an integer from among the range of 7 to 50. In one embodiment n is an integer from among the range of 7 to 30. In one embodiment n is an integer from among the range of 7 to 15. Typically, the DNA barcode according to the invention comprises 4, 5, 6, 7, 8, 9, 10, 11, 12, 13, 14 or 15 nucleotides.

In one embodiment, the nucleic acid sequence further comprises a sequence P1 of at least 10 nucleotides. In one aspect of any of the embodiments herein P1 comprises a sequence of between 10 and 50 nucleotides. In one embodiment P1 comprises a sequence of between 10 and 30 nucleotides. In one embodiment P1 comprises a sequence of between 10 and 20 nucleotides. Ione embodiment P1 comprises a sequence of between 10 and 15 nucleotides.

For example, in a first embodiment, sequence P1 may represent a tail, moreover a poly(dN) tail (e.g. a poly(A), a poly(C), a poly(T) or a poly(G) tail) that can be useful for capturing the DNA barcode once released after cleavage of sequence CL. Said capture may be done with a complementary nucleic acid immobilized on a surface (e.g. a bead) that is capable of hybridization with sequence P1. Then after deep sequencing (as hereinafter described) may be carried out so as to read the DNA barcode. Typically sequence P1 flanks the DNA barcode at the 3' terminal end of the DNA barcode, and the enzymatic cleavable sequence (CL) flanks sequence P1 at the 3' terminal end of P1. Accordingly in one embodiment, the nucleic acid molecule has the general formula of: 3'- CL-P1-N₁N₂N₃N₄Nₙ-5'.

In a second embodiment, sequence P1 is capable of hybridization with a primer. In said embodiment, the nucleic acid molecule conjugated to the antibody further comprises a second sequence P2 that is able to hybridize with a second primer, wherein the sequences P1 and P2 respectively flank the DNA barcode at the 3' and 5' terminal ends of the DNA barcode. Thus sequences P1 and P2 may be used for amplifying the DNA barcode. Accordingly in one embodiment, the nucleic acid molecule has the general formula of: 3'-CL-P1-N₁N₂N₃N₄Nₙ-P2- 5' wherein:
- CL represents an enzymatic cleavable sequence
- P1 represents a first sequence which is able to hybridize to a first forward primer
- N₁N₂N₃N₄Nₙ represents a DNA barcode wherein N represents a nucleotide and n an integer number superior to 4
- P2 represents a second sequence which is able to hybridize to a second reverse primer

In one aspect of any of the embodiments herein, P2 is a nucleic acid sequence of at least 10 nucleotides in length. In one embodiment P2 comprises a sequence of between 10 and 50 nucleotides. In one embodiment P2 comprises a sequence of between 10 and 30 nucleotides. In one embodiment P2 comprises a sequence of between 10 and 20 nucleotides. In one embodiment P2 comprises a sequence of between 10 and 15 nucleotides.

As used herein, a primer is an oligonucleotide for amplification of a target sequence by extension of the oligonucleotide after hybridization to the target sequence (i.e. P1 or P2) or by ligation of multiple oligonucleotides which are adjacent when hybridized to the target sequence. At least a portion of the amplification primer hybridizes to the target (i.e. P1 or P2). This portion is referred to as the target binding sequence and it determines the target-specificity of the primer. The terms "primer" thus refers to the function of the oligonucleotide. A nucleic acid molecule is capable to hybridize to another nucleic acid molecule, when a single stranded form of the nucleic acid molecule can anneal to the other nucleic acid molecule under the appropriate conditions of temperature and solution ionic strength (see Sambrook et al., 1989).

In one embodiment, sequence P1 is CCATCTCATCCCTGCGTGTCTCCGACTCAG. In one embodiment, sequence P2 is ATCACCGACTGCCCATAGAGAGG.

One essential feature of the invention is that the enzyme and the cleavable sequence are selected in a manner that the cleavage will occur only in the CL sequence and not in P1 (nor P2) nor in the DNA barcode.

In one embodiment, the nucleic acid molecule comprises the sequence GAATTCCCATCTCATCCCTGCGTGTCTCCGACTCAGNNNNNNNNNATCACCGACT GCCCATAGAGAGG.

Techniques for conjugating molecule to antibodies, are well-known in the art (See, e.g., Arnon et al., "Monoclonal Antibodies For Immunotargeting Of Drugs In Cancer Therapy," in Monoclonal Antibodies And Cancer Therapy (Reisfeld et al. eds., Alan R. Liss, Inc., 1985); Hellstrom et al., "Antibodies For Drug Delivery," in Controlled Drug Delivery (Robinson et al. eds., Marcel Deiker, Inc., 2nd ed. 1987); Thorpe, "Antibody Carriers Of Cytotoxic Agents In Cancer Therapy: A Review," in Monoclonal Antibodies '84: Biological And Clinical Applications (Pinchera et al. eds., 1985); "Analysis, Results, and Future Prospective of the Therapeutic Use of Radiolabeled Antibody In Cancer Therapy," in Monoclonal Antibodies For Cancer Detection And Therapy (Baldwin et al. eds., Academic Press, 1985); and Thorpe et al., 1982, Immunol. Rev. 62:119-58. See also, e.g., PCT publication WO 89/12624.) Typically, the nucleic acid molecule is covalently attached to lysines or cysteines on the antibody, through N-hydroxysuccinimide ester or maleimide functionality respectively. Methods of conjugation using engineered cysteines or incorporation of unnatural amino acids have been reported to improve the homogeneity of the conjugate (Axup, J.Y., Bajjuri, K.M., Ritland, M., Hutchins, B.M., Kim, C.H., Kazane, S.A., Halder, R., Forsyth, J.S., Santidrian, A.F., Stafm, K., et al. (2012). Synthesis of site-specific antibody-drug conjugates using unnatural amino acids. Proc. Natl. Acad. Sci. USA 109, 16101-16106.; Junutula, J.R., Flagella, K.M., Graham, R.A., Parsons, K.L., Ha, E., Raab, H., Bhakta, S., Nguyen, T., Dugger, D.L., Li, G., et al. (2010). Engineered thio-trastuzumab-DM1 conjugate with an improved therapeutic index to target humanepidermal growth factor receptor 2-positive breast cancer. Clin. Cancer Res. 16, 4769-4778.). Junutula et al. (2008) developed cysteine-based site-specific conjugation called "THIOMABs" (TDCs) that are claimed to display an improved therapeutic index as compared to conventional conjugation methods. Conjugation to unnatural amino acids that have been incorporated into the antibody is also being explored for ADCs; however, the generality of this approach is yet to be established (Axup et al., 2012). In particular the one skilled in the art can also envisage Fc-containing polypeptide engineered with an acyl donor glutamine-containing tag (e.g., Gin-containing peptide tags or Q- tags) or an endogenous glutamine that are made reactive by polypeptide engineering (e.g., via amino acid deletion, insertion, substitution, or mutation on the polypeptide). Then a transglutaminase, can covalently crosslink with an amine donor agent (e.g., a small molecule comprising or attached to a reactive amine) to form a stable and homogenous population of an engineered Fc-containing polypeptide conjugate with the amine donor agent being site- specifically conjugated to the Fc-containing polypeptide through the acyl donor glutamine- containing tag or the accessible/exposed/reactive endogenous glutamine (WO 2012059882).

The term "transglutaminase", used interchangeably with "TGase" or "TG", refers to an enzyme capable of cross-linking proteins through an acyl-transfer reaction between the γ-carboxamide group of peptide-bound glutamine and the ε-amino group of a lysine or a structurally related primary amine such as amino pentyl group, e.g. a peptide-bound lysine, resulting in a ε-(γ-glutamyl)lysine isopeptide bond. TGases include, *inter alia,* bacterial transglutaminase (BTG) such as the enzyme having EC reference EC 2.3.2.13 (protein-glutamine-γ-glutamyltransferase).

TGase will be able to conjugate a linking reagent comprising a primary amine and a moiety of interest (e.g, a reactive group, a nucleic acid comprising an enzymatic cleavable sequence (CL) and a DNA barcode sequence N₁N₂N₃N₄Nₙ wherein N represents a nucleotide and n represents an integer number superior to 4) to an acceptor glutamine residue in an antibody.

The term "acceptor glutamine residue", when referring to a glutamine residue of an antibody, means a glutamine residue that is recognized by a TGase and can be cross-linked by a TGase through a reaction between the glutamine and a lysine or a structurally related primary amine such as amino pentyl group. Preferably the acceptor glutamine residue is a surface-exposed glutamine residue. Antibodies of human or murine subtypes will comprise an acceptor glutamine at residue 295 (EU numbering) in their heavy chain CH2 domains. Antibodies or antibody fragments can also be modified (e.g. by introducing or substitution one or more amino acid residues) so as to comprise an acceptor glutamine at a non-naturally occurring position. For example one or more amino acids (e.g. a peptide tag) can be modified or introduced so as to generate a TGase recognition tag, for example fused to the C-terminus of a heavy or light chain constant region.

The term "TGase recognition tag" refers to a sequence of amino acids comprising an acceptor glutamine residue and that when incorporated into (e.g. appended to) a polypeptide sequence, under suitable conditions, is recognized by a TGase and leads to cross-linking by the TGase through a reaction between an amino acid side chain within the sequence of amino acids and a reaction partner. The TGase recognition tag may be a peptide sequence that is not naturally present in the antibody.

The antibodies can thus be conjugated to the nucleic acid of the invention via a linking reagent that can be attached, by the action of a TGase, at a glutamine residue (Q) within the sequence of the antibody or antibody fragment. The linking reagent comprises a lysine derivative (Lys), or a functional equivalent thereof, that is connected to at least one a nucleic acid comprising a DNA barcode sequence (e.g. a nucleic acid comprising an enzymatic cleavable sequence (CL) and a DNA barcode sequence N₁N₂N₃N₄Nₙ wherein N represents a nucleotide and n represents an integer number superior to 4) . One example of such a linking reagent is a compound of Formula Ia. Such linker can then be brought into contact with an antibody comprising an acceptor glutamine, in the presence of TGase enzyme, under suitable conditions, such that the linking reagent is directly, in a single reaction step, conjugated to the antibody in a site-specific manner at the acceptor glutamine. The result is a composition comprising a plurality of antibodies having an acceptor glutamine (e.g. one or two acceptor glutamines on each heavy chain) functionalized with (covalently bound to) a linking reagent comprising a lysine derivative (Lys), or a functional equivalent thereof, and least one a nucleic acid comprising a DNA barcode sequence (e.g. a nucleic acid comprising an enzymatic cleavable sequence (CL) and a DNA barcode sequence N₁N₂N₃N₄Nₙ wherein N represents a nucleotide and n represents an integer number superior to 4).

In one embodiment, at least 70%, 80% or 90% of the antibodies in the composition will have exactly two functionalized acceptor glutamines per antibody. In one embodiment, at least 70%, 80% or 90% of the antibodies in the composition will have exactly four functionalized acceptor glutamines per antibody.

In one embodiment, a multi-step conjugation process can be used to produce antibody conjugates. Such process can have the advantage of achieving efficiencies in substrate use, achieving higher product homogeniety. Such process can also have the advantage of permitting the conjugation of large molecules (e.g. longer oligonucleotides) to obtain compositions having homogenous DNA barcode: antibody ratios, and in particular highly homogenous compositions having two or four DNA barcode conjugates per antibody. In a multi-step method, a linking reagent comprises a lysine derivative (Lys), or a functional equivalent thereof, and a reactive group (R) (e.g. a linking reagent of Formula Ib). The linking reagent can be attached, by the action of a TGase, at a glutamine residue (Q) within the sequence of the antibody or antibody fragment to create an intermediate antibody conjugate. The intermediate antibody conjugate (e.g. an antibody of Formula II) comprising a reactive group (R) can in turn be reacted with a compound comprising a complementary group R' and nucleic acid comprising a DNA barcode sequences (e.g. a nucleic acid comprising an enzymatic cleavable sequence (CL) and a DNA barcode sequence N₁N₂N₃N₄Nₙ wherein N represents a nucleotide and n represents an integer number superior to 4). The result is an antibody (e.g. a composition of a plurality of antibodies) in which a DNA barcode sequence (e.g. a nucleic acid comprising an enzymatic cleavable sequence (CL) and a DNA barcode sequence N₁N₂N₃N₄Nₙ wherein N represents a nucleotide and n represents an integer number superior to 4) is connected to the antibody via a linking reagent that comprises the reaction product of R and R' (the reaction produce is referred to herein as the (RR') group).

In one embodiment, at least 70%, 80% or 90% of the antibodies in a composition of antibodies obtained will have exactly two functionalized acceptor glutamines per antibody. In one embodiment, at least 70%, 80% or 90% of the antibodies in a composition of antibodies obtained will have exactly four functionalized acceptor glutamines per antibody.

The lysine derivative can be a 2 to 20 alkyl or heteroalkyl chain, or a functional equivalent thereof, with an H₂N, H₂NOCH₂, H₂NCH₂ (aminomethylene) group or a protected H₂N, H₂NOCH₂, H₂NCH₂ group positioned at one or more ends of the alkyl or heteroalkyl chain. The heteroalkyl chain can be a chain of 3 to 20 atoms where one or more non-terminal atoms can be other than carbon, for example oxygen, sulfur, nitrogen, or other atoms. The oxygen, sulfur, or nitrogen atom can be of an ether, ester, thioether, thioester, amino, alkylamino, amido or alkylamido functionality within the carbon chain.

The heteroalkyl chain can be an oligo (ethylene oxide) chain. The functionality within the alkyl or heteroalkyl chain can be included to couple the reactive group to the H₂N, H₂NOCH₂, H₂NCH₂ group or protected H₂N, H₂NOCH₂, H₂NCH₂ group. The alkyl or heteroalkyl chain can be substituted or unsubstituted. The substituents can be alkyl groups, aryl groups, alkyl aryl groups, carboxylic acid groups, amide groups, hydroxy groups, or any other groups that do not compete with the amino group for, or inhibit, conjugation with a glutamine residue of the protein. Typically, when a substituent is present, its presence is in a convenient starting material, such as the carboxylic acid group of lysine, from which the lysine derivative results. The H₂N, H₂NOCH₂, H₂NCH₂ end of an alkyl or heteroalkyl chain is necessarily included in the linking reagent.

Exemplary starting materials for the functional equivalent of lysine can be an α,ω-diaminoalkane, for example, 1,2-diaminoethane, 1,3-diaminopropane, 1,4-diaminobutane, 1,5-diaminopentane, 1,6-diaminohexane, 1,7-diaminoheptane, 1,8-diaminooctane, 1,9-diaminononane, 1,10-diaminodecane, 1,11-diaminoundecane, or 1,12-diaminododecane. Other starting materials for the functional equivalent of a lysine derivative can be α,ω-diamino oligo (ethylene oxide), for example, H₂N(CH₂CH₂O)ₓCH₂CH₂NH₂ where x is 1 to about 6. The α,ω-diamino oligo (ethylene oxide) can be a single oligomer or it can be a mixture of oligomers where x defines an average size. An exemplary protected H₂NCH₂ is the *tert*-butylcarbamate protected amine of *tert*-butyl N-(5-aminopentyl)carbamate (N-Boc-cadaverin)

A linking reagent, or an antibody-conjugated linking reagent for use in a multi-step method, can have the general Formula Ib:

G-NH-(C)-(R)q Formula Ib

wherein:
- G is a H, amine protecting group, or upon conjugation, an antibody or antibody fragment attached via an amide bond;
- C is a bond or a linking moiety;
- q is an integer selected from among 1, 2, 3 or 4; and
- R is a reactive moiety.

When such reactive group-containing linker is conjugated to an antibody, the antibody will have a functionalized acceptor glutamine of Formula II:

Q-NH-(C)-((R))q Formula II

wherein:
- Q is a glutamine residue present in the antibody;
- C is a bond or a linking moiety;
- q is an integer selected from among 1, 2, 3 or 4; and
- R is a reactive moiety.

A linking reagent, or an antibody-conjugated linking reagent for use in a one-step conjugation method, can have the general Formula Ia:

G-NH-C-(CL-(P¹-Z-P²)_{q} Formula Ia

wherein:
- G is a H, amine protecting group, or upon conjugation, an antibody or antibody fragment attached via an amide bond;
- C is a bond or a linking moiety;
- q is an integer selected from among 1, 2, 3 or 4;
- CL is an enzymatic cleavable nucleic acid sequence;
- P¹ is independently absent or a nucleic acid having at least 10 nucleotides;
- Z is a DNA barcode sequence N₁N₂N₃N₄Nₙ wherein N represents a nucleotide and n represents an integer number superior to 4; and
- P² is independently absent or a nucleic acid having at least 10 nucleotides.

In one embodiment, the linking moiety C is a -(C)ₙ-X-L- unit, e.g. the linking reagent with reactive group has a formula Ib variant as follows: G-NH-(C)ₙ-X-L-(CL-(P¹-Z-P²)_{q} or G-NH-(C)ₙ-X-L- (CL-(R)_{q}. The linking reagent for single step reactions has a formula Ia variant as follows: G-NH-(C)ₙ-X-L-(CL-(P¹-Z-P²)_{q}.

The (C)ₙ group may for example be a straight, branched and/or cyclic C₂₋₃₀ alkyl, C₂₋₃₀ alkenyl, C₂₋₃₀ alkynyl, C₂₋₃₀ heteroalkyl, C₂₋₃₀ heteroalkenyl, C₂₋₃₀ heteroalkynyl, optionally wherein one or more homocyclic aromatic compound radical or heterocyclic compound radical may be inserted; notably, any straight or branched C₁₋₅ alkyl, C₅₋₁₀ alkyl, C₁₁₋₂₀ alkyl, - O- C₁₋₅ alkyl, -O- C₅₋₁₀ alkyl, -O- C₁₁₋₂₀ alkyl, CH₂-(CH₂-O-CH₂)₁₋₁₂-CH₂ or (CH₂- CH₂-O-)₁₋₁₂, an amino acid, an oligopeptide, glycan, sulfate, phosphate or carboxylate.

In one example the (C)ₙ group is a carbon comprising framework substituted with one or more O atoms. In one embodiment, the carbon adjacent to the nitrogen is substituted with an O atom. In one embodiment, the carbon adjacent to the nitrogen is unsubstituted. In one embodiment, the (C)ₙ group is or comprises an ethylene oxide group, e.g. a CH₂-(CH₂-O-CH₂)ₙ-CH₂ group or an (CH₂- CH₂-O-)ₙ, where n is an integer from 1 to 10.

The L group can be a carbon comprising framework, where L is a linear hydrocarbon, a symmetrically or asymmetrically branched hydrocarbon, monosaccharide, disaccharide, linear or branched oligosaccharide (asymmetrically branched or symmetrically branched), an amino acid, a di-, tri-, tetra-, or oligopeptide, other natural oligomer, dimer, trimer, or higher oligomer (linear asymmetrically branched or symmetrically branched) resulting from any chain-growth or step-growth polymerization process. For example, L may comprise or be a straight, branched and/or cyclic C₂₋₃₀ alkyl, C₂₋₃₀ alkenyl, C₂₋₃₀ alkynyl, C₂₋₃₀ heteroalkyl, C₂₋₃₀ heteroalkenyl, C₂₋₃₀ heteroalkynyl, optionally wherein one or more homocyclic aromatic compound radical or heterocyclic compound radical may be inserted; notably, any straight or branched C₂₋₅ alkyl, C₅₋₁₀ alkyl, C₁₁₋₂₀ alkyl, -O- C₁₋₅ alkyl, -O- C₅₋₁₀ alkyl, -O- C₁₁₋₂₀ alkyl, CH₂-(CH₂-O-CH₂)₁₋₃₀-CH₂ or (CH₂- CH₂-O-)₁₋₃₀, e.g., (CH₂- CH₂-O-)₁₂, (CH₂- CH₂-O-)₁₋₂₄,an amino acid, an oligopeptide, glycan, sulfate, phosphate, carboxylate. Optionally, L is absent.

Linking moiety C or L can have q sites of attachment for the respective (CL-(P¹-Z-P²) or R groups, where q represents the degree of branching or polymerization. The sites of attachment can comprise a bond or comprise a functional group selected from an alkene, alkyne, ether, thioether, ester, thioester, amine, amide, alkylamide, or other functional group readily generated by a condensation or addition reaction.

In one example the carbon comprising framework of the L group is optionally substituted with one or more O atoms. In one embodiment, the L group comprises one or more ethylene oxide groups (CH₂-O-CH₂). Optionally, the L group comprises a carbon framework comprising a (CH₂-CH₂-O-)ₙ group, wherein n is an integer selected among the range of 1 to 10 (e.g., 1, 2, 3, 4, 5, 6, 7, 8, 9 or 10).

In Formulae Ia, Ib, II, IVa and IVb variants, the linking group L links the aminopeptidyl moiety -NH-(C)ₙ-X to the reactive group R or the nucleic acid unit (CL-(P¹-Z-P²). L may be a bond connecting R or (CL-(P¹-Z-P²) directly to the aminopeptidyl moiety. In another aspect, however, L is a linking group that functionally links or spaces the reactive moiety R or nucleic acid (CL-(P¹-Z-P²). In Formulae Ib, II and IVb, spacing improves efficiency and completion of BTGase coupling, make additionally the reactive moiety R more accessible to the reaction partner, for example when the reactive moiety is present on a lysine-based linker and coupled to the antibody and then brought into contact with a reaction partner. In Formulae Ia and IVa, the linking group L links the aminopeptidyl moiety -NH-(C)ₙ-X to the nucleic acid (CL-(P¹-Z-P²). L may be a bond connecting the nucleic acid unit (CL-(P¹-Z-P²)directly to the aminopeptidyl moiety. In another aspect, however, L is a linking group that functionally links or spaces the reactive group (R) or nucleic acid (CL-(P¹-Z-P²). In Formulae Ia and IVa, spacing improves efficiency and completion of TGase coupling, providing for highly homogenous antibody compositions. In one embodiment, the invention relates to antibody compositions wherein at least 80% or 90% of the antibodies in a composition have exactly two functionalized acceptor glutamines of formulae II, IVa or IVb (e.g., one acceptor glutamine on each heavy chain, for example at position Q295 (EU numbering)).

In another embodiment, the invention relates to antibody compositions wherein at least 80% or 90% of the antibodies in a composition have exactly four functionalized acceptor glutamines of formulae II, IVa or IVb (e.g., two acceptor glutamines on each heavy chain. Acceptor glutamines may be, for example, at positions 295 and 297 (EU numbering)), wherein an asparagine naturally present at position 297 has been substituted by a glutamine residue.

The linking group L may be a water-soluble moiety or contain one or more water-soluble moieties, such that L contributes to the water solubility of a compound of Formula (I) - (VI). An L may also be a moiety or contain one or more moieties that reduce(s) aggregation, which may or may not be a moiety/moieties that also increase(s) the water solubility.

L may be for example a linear linker or a branched linker. In one aspect, the L moiety is branched, optionally further a dendritic structure, so that it can be connected to at least two, three or four R moieties or nucleic acid units (CL-(P¹-Z-P²)). Each nucleic acid unit (CL-(P¹-Z-P²)) is however only attached once to an L moiety. Branching can occur at one or more branching atoms that may for example be carbon, nitrogen, silicon, or phosphorus.

The R group is a reactive moiety, for example a moiety comprising an unprotected or protected bioorthogonal-reaction compatible reactive group, for example an unprotected or protected thiol, epoxide, maleimide, haloacetamide, *o*-phoshenearomatic ester, azide, fulminate, sulfonate ester, alkyne, cyanide, amino-thiol, carbonyl, aldehyde, generally any group capable of oxime and hydrazine formation, 1,2,4,5-tetrazine, norbornene, other stained or otherwise electronically activated alkene, a substituted or unsubstituted cycloalkyne, generally any reactive groups which form via bioorthogonal cycloaddition reaction a 1,3- or 1,5-disubstituted triazole, any diene or strained alkene dienophile that can react via inverse electron demand Diels-Alder reaction , a protected or unprotected amine, a carboxylic acid, an aldehyde, or an oxyamine.

The reactive group of the linking reagent can for example chosen to undergo thio-maleimide (or haloacetamide) addition, Staudinger ligation, Huisgen 1,3-cycloaddition (click reaction), or Diels-Alder cycloaddition with a complementary reactive group attached to an agent comprising a therapeutic moiety, a diagnostic moiety, or any other moiety for a desired function.

In one embodiment, the reactive group is a haloacetamide, (e.g. bromo-acetamide, iodo-acetamide, chloro-acetamide). Such reactive groups will be more stable *in vivo* (and in serum) compared with maleimide groups.

In one embodiment, the reactive group is a reagent capable of undergoing a "click" reaction. For example a 1,3-dipole-functional compound can react with an alkyne in a cyclization reaction to form a heterocyclic compound, preferably in the substantial absence of added catalyst (e.g., Cu(I)). A variety compounds having at least one 1,3-dipole group attached thereto (having a three-atom pi-electron system containing 4 electrons delocalized over the three atoms) can be used to react with the alkynes disclosed herein. Exemplary 1,3-dipole groups include, but are not limited to, azides, nitrile oxides, nitrones, azoxy groups, and acyl diazo groups.

Examples include *o*-phosphenearomatic ester, an azide, a fulminate, an alkyne (including any strained cycloalkyne), a cyanide, an anthracene, a 1,2,4,5-tetrazine, or a norbornene (or other strained cycloalkene).

In one embodiment, R is a moiety having a terminal alkyne or azide; such moieties are described for example in U.S. patent no. 7,763,736, the disclosure of which is incorporated herein by reference. Suitable reaction conditions for use of copper (and other metal salt) as catalysts of click-reactions between terminal alkynes and azides are provided in U.S. patent no. 7,763,736.

In one embodiment, R is a substituted or unsubstituted cycloalkyne. Cycloalkynes, including specific compounds, are described for example in U.S. Patent No. 7,807,619, the disclosure of which is incorporated herein by reference.

In some embodiments, a cycloalkyne may be a compound of Formula A: 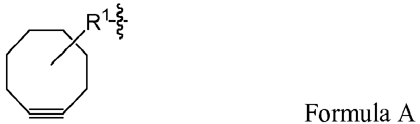 where:
- R¹ is selected from a carbonyl, an alkyl ester, an aryl ester, a substituted aryl ester, an aldehyde, an amide, an aryl amide, an alkyl halide, a thioester, a sulfonyl ester, an alkyl ketone, an aryl ketone, a substituted aryl ketone, and a halosulfonyl;
- R¹ can be at any position on the cyclooctyne group other than at the two carbons joined by the triple bond.

In some embodiments, the modified cycloalkyne is of Formula A, wherein one or more of the carbon atoms in the cyclooctyne ring, other than the two carbon atoms joined by a triple bond, is substituted with one or more electron-withdrawing groups, e.g., a halo (bromo, chloro, fluoro, iodo), a nitro group, a cyano group, a sulfone group, or a sulfonic acid group. Thus, e.g., in some embodiments, a subject modified cycloalkyne is of Formula B: 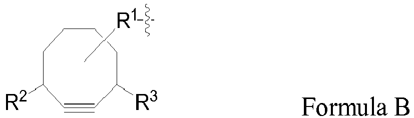 where:
- each of R² and R³ is independently: (a) H; (b) a halogen atom (e.g., bromo, chloro, fluoro, iodo); (c) -W-(CH₂)ₙ-Z (where: n is an integer from 1-4 (e.g., n=1, 2, 3, or 4); W, if present, is O, N, or S; and Z is nitro, cyano, sulfonic acid, or a halogen); (d) -(CH₂)ₙ-W-(CH₂)ₘ-R⁴ (where: n and m are each independently 1 or 2; W is O, N, S, or sulfonyl; if W is O, N, or S, then R⁴ is nitro, cyano, or halogen; and if W is sulfonyl, then R⁴ is H); or (e) -CH₂)ₙ- R⁴ (where: n is an integer from 1-4 (e.g., n=1, 2, 3, or 4); and R⁴ is nitro, cyano, sulfonic acid, or a halogen); and
- R¹ is selected from a carbonyl, an alkyl ester, an aryl ester, a substituted aryl ester, an aldehyde, an amide, an aryl amide, an alkyl halide, a thioester, a sulfonyl ester, an alkyl ketone, an aryl ketone, a substituted aryl ketone and a halosulfonyl. R¹ can be at any position on the cyclooctyne group other than at the two carbons linked by the triple bond.

In one embodiment, R is a substituted or unsubstituted heterocyclic strained alkyne. Cycloalkynes, including specific compounds, are described for example in U.S. Patent No. 8,133,515, the disclosure of which is incorporated herein by reference. In one embodiment, the alkyne is of the Formula C: 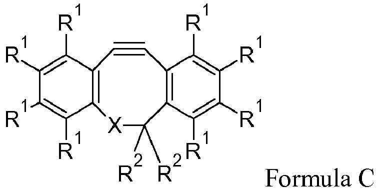 wherein:
- each R¹ is independently selected from the group consisting of hydrogen, halogen, hydroxy, alkoxy, nitrate, nitrite, sulfate, and a C₁-C₁₀ alkyl or heteroalkyl;
- each R² is independently selected from the group consisting of hydrogen, halogen, hydroxy, alkoxy, nitrate, nitrite, sulfate, and a C₁-C₁₀ organic group; X represents N-R³R⁴, NH-R⁴, CH-N-OR⁴, C-N-NR³R⁴, CHOR₄, or CHNHR₄; and each R³ represents hydrogen or an organic group and R⁴ represents linking moiety C (or (C)ₙ) of a linker of the invention. In one embodiment, R or R' is a DBCO (dibenzycyclooctyl) group below:

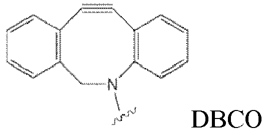

Alkynes such as those described herein above can be reacted with at least one 1,3-dipole-functional compound (e.g., embodied as an R' moiety in a compound of Formula III) in a cyclization reaction to form a heterocyclic compound, preferably in the substantial absence of added catalyst (e.g., Cu(I)). A wide variety compounds having at least one 1,3-dipole group attached thereto (having a three-atom pi-electron system containing 4 electrons delocalized over the three atoms) can be used to react with the alkynes disclosed herein. Exemplary 1,3-dipole groups include, but are not limited to, azides, nitrile oxides, nitrones, azoxy groups, and acyl diazo groups.

The reactive moiety R is connected to C, (C)ₙ, X or L, and is able to react with a suitable functional group (R') on a reaction partner, e.g. a complementary reagent of Formula III which undergoes a high conversion addition reaction when brought into contact with a reactive moiety R. When reactive moiety R is present on an antibody of Formula II, the reaction results in formation of a antibody of Formula IVb. In this reaction, the moieties R and R' are transformed into the moiety (RR'). Any R' moiety can be defined in the same way as aan R moiety, so long as R and R' are complementary when used in moieties that are to be reacted together.

The complementary reactive froup R' (e.g. in Formula III) is a reactive moiety that can be defined similary to reactive moiety R, so long as R' when unprotected is reactive with R (when R is unprotected). For example R' may be a moiety comprising an unprotected or protected bioorthogonal-reaction compatible reactive group, for example an unprotected or protected thiol, epoxide, maleimide, haloacetamide, *o*-phoshenearomatic ester, azide, fulminate, sulfonate ester, alkyne, cyanide, amino-thiol, carbonyl, aldehyde, generally any group capable of oxime and hydrazine formation, 1,2,4,5-tetrazine, norbornene, other stained or otherwise electronically activated alkene, a substituted or unsubstituted cycloalkyne, generally any reactive groups which form via bioorthogonal cycloaddition reaction a 1,3- or 1,5-disubstituted triazole, any diene or strained alkene dienophile that can react via inverse electron demand Diels-Alder reaction , a protected or unprotected amine, a carboxylic acid, an aldehyde, an oxyamine, so long as such group when unprotected is reactive with R (when R' is unprotected). In one embodiment, R' is a moiety having a terminal alkyne or azide. In one embodiment, R' is a substituted or unsubstituted heterocyclic strained alkyne of Formula A, B or C.

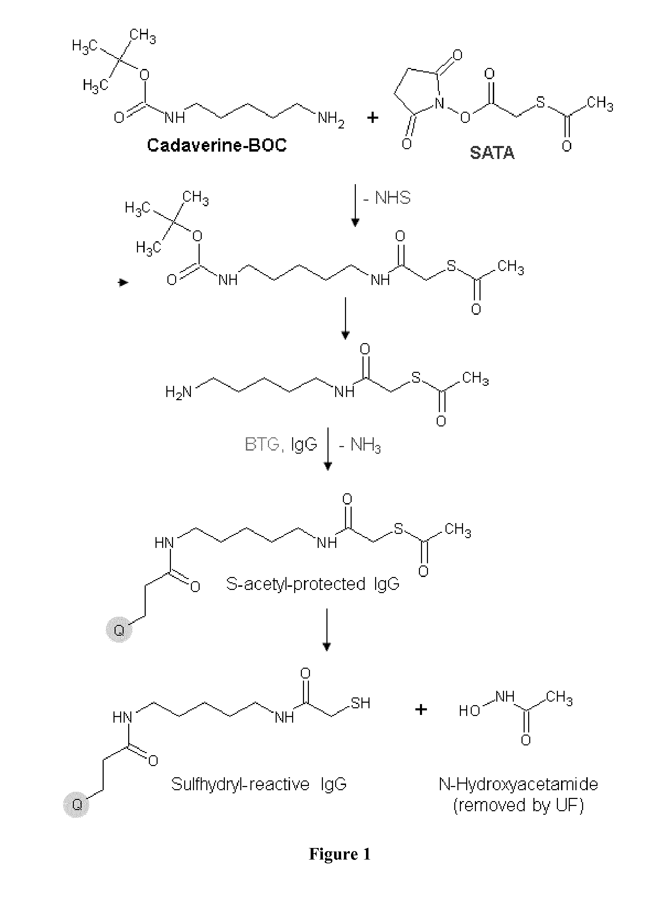

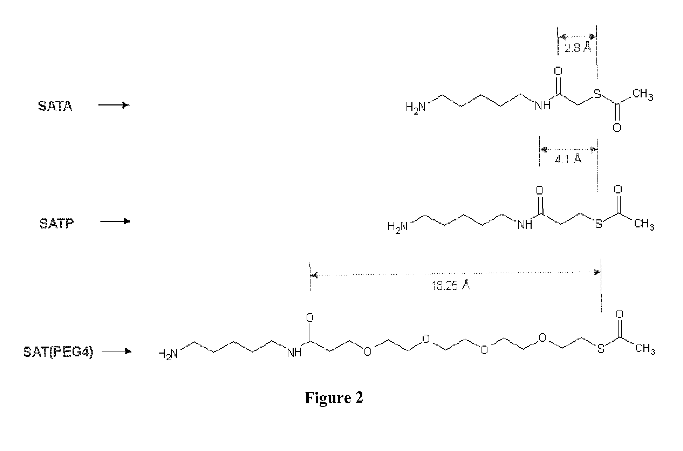

The preparation of an exemplary linking reagent and its conjugation with a protein is illustrated in Figure 1, where: R is a thiol (sulfhydryl) reactive group that is ultimately generated from the S-acetyl protected thiol, SC(O)CH₃; q is 1; L is the two carbon comprising framework C(O)CH₂; X is NH; (C)ₙ is (CH₂)₅; and G is transformed from the (H₃C)₃COC(0) protecting group to H and ultimately to the amide upon conjugation of a glutamine residue of a protein. Figure 2 illustrates the preparation of various exemplary linking reagents with a single S-acetyl protected thiol reactive group that can be prepared from an N-succinimidyl-S-acetylthioester reagent. In addition to S-acetyl, other S-protecting groups can be employed, includingp-hydroxyphenylacyl, 2-quinoline, or Hqm and Hgm groups that can be deprotected by the addition of hydrazine.

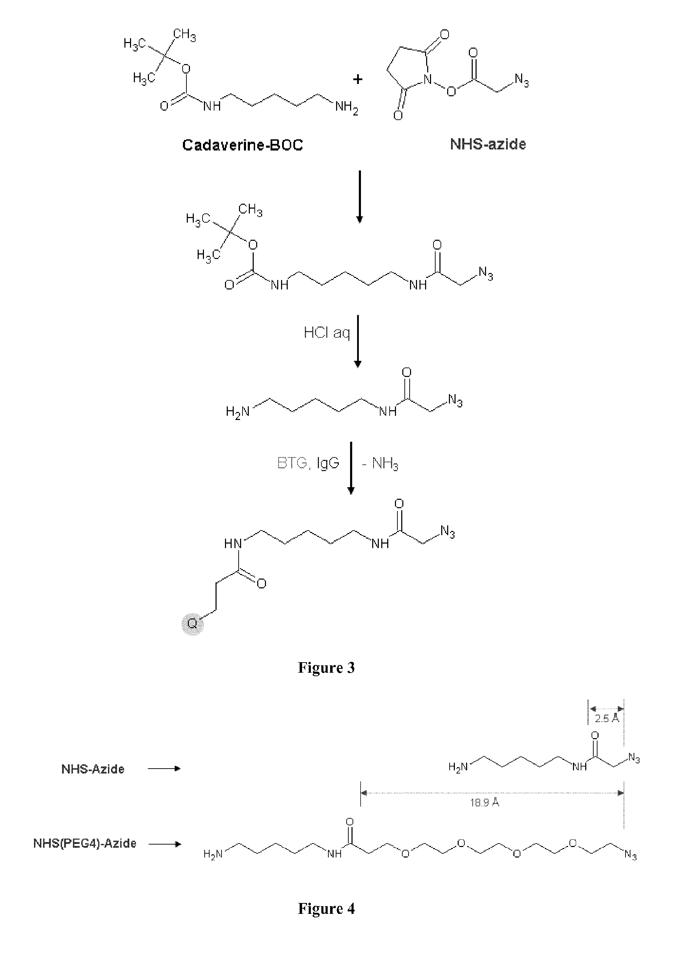

Figure 3 illustrates the preparation of an exemplary linking reagent, and its conjugation with a protein, where: R is an azide reactive group; q is 1; L is the two carbon comprising framework C(O)CH₂; X is NH; (C)ₙ is (CH₂)₅; and G is transformed from the (H₃C)₃COC(0) protecting group to H and ultimately to the amide upon conjugation of a glutamine residue of a protein. Figure 4 illustrates the preparation of various exemplary linking reagents with a single azide reactive group that can be prepared from an N-succinimidyl-azide reagent.

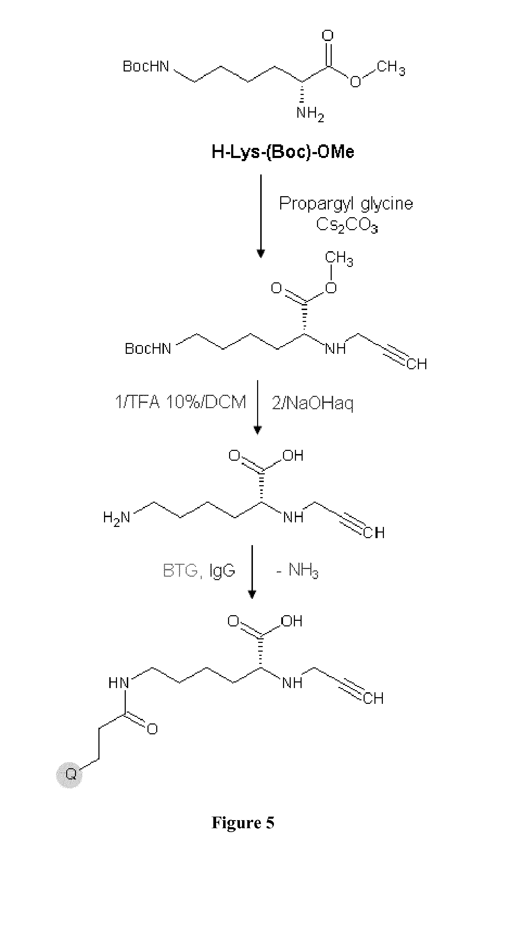

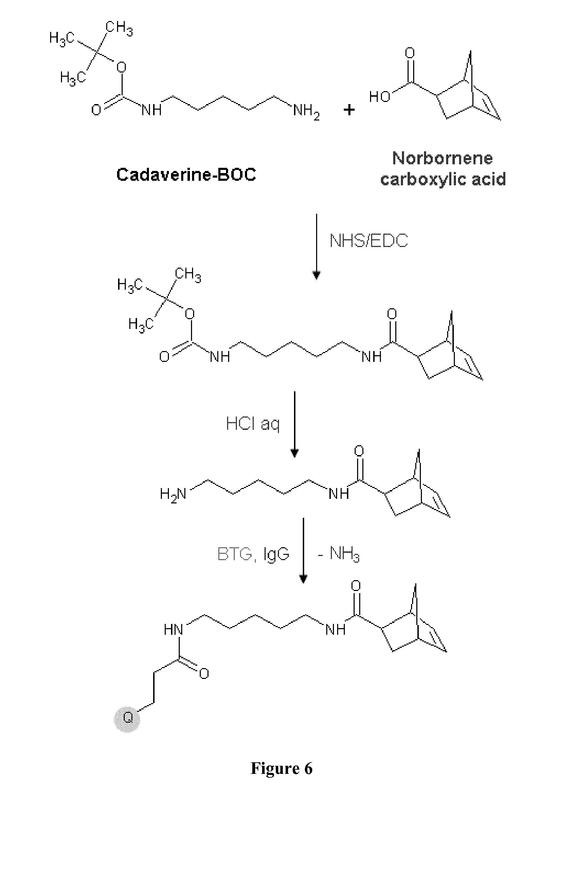

Figure 5 depicts the preparation of an exemplary linking reagent and its conjugation with a protein, where: R is an alkyne reactive group; q is 1; L is a one carbon comprising framework CH₂; X is NH; (C)ₙ is (CH₂)₄CH(CO₂H); and G is transformed from the (H₃C)₃COC(0) protecting group to H and ultimately to the amide upon conjugation of a glutamine residue of a protein. Figure 6 shows the preparation of an exemplary linking reagent and its conjugation with a protein, where: R is a norbornene reactive group; q is 1; L is the one carbon comprising framework C(O); X is NH; (C)ₙ is(CH₂)₄CH(CO₂H); and G is transformed from the (H₃C)₃COC(0) protecting group to H and ultimately to the amide upon conjugation of a glutamine residue of a protein.

The selective and very high conversion addition reaction that can be carried out with the linking reagents, according to this aspect of the invention, can be uncatalyzed or catalyzed reactions. For example, the 2+4 Diels-Alder cycloadditions, thio-maleimide (or haloacetamide) additions, and Staudinger ligations can be carried out without a catalyst. Other very high conversion addition reactions, for example any of the click reactions, can be catalyzed with metal salts, such as Cu, Ru, Ni, Pd, and Pt salts.

The linking group (RR') in M of compounds of Formula IVb represents the remainder of R when the reactive moiety R of Formula II has reacted with a reactive moiety R' in a compound of Formula III. This group (RR') then links the nucleic acid unit (CL-(P¹-Z-P²)) (e.g. comprised in the compound of formula IVb) with C (or L or (C)ₙ or X when present). The group that remains may optionally be a bond.

In one embodiment, R is an azide and R' is a substituted or unsubstituted cycloalkyne, optionally R' is a DBCO (dibenzycyclooctyl) group.

Enzymes of the TG-family catalyze covalent protein crosslinking by forming proteinase resistant isopeptide bonds between a lysine donor residue of one protein and an acceptor glutamine residue of another protein, that is accompanied by the release of ammonia. The antibodies that are to be conjugated to the lysine-based linker may or may not be free of N-linked glycosylation (e.g. an antibody which does not comprise glycosylation sites or a modified full-length antibody). For conjugation onto the acceptor glutamines within the CH2 domain, and particularly at residue Q295, antibodies will be free of N-linked glycosylation. Full-length wild-type IgG antibodies naturally comprise N-linked glycosylation at residue 297 of the heavy chain which interferes and prevents with TGase-mediated conjugation onto glutamine residues in the CH2 domain. Deglycosylation can be carried out according to any suitable method. For example, antibody in PBS buffer (PBS (10X): Weight 2.1 g KH₂PO₄, 90 g NaCl, 4.8 g Na₂HPO₄ x 2 H₂O is transfered to a 1 L glass bottle, to which is added water to a volume of 1 L. To get PBS 1X, use 100 mL PBS (10X) and add water to a volume of 900 mL. pH is adjusted to 7.2 and filled to 1 L with water), and incubated with 6 Units/mg protein ofN-glycosidase F (PNGase F) from *Flavobacterium meningosepticum* (Roche, Switzerland) overnight at 37°C. The enzyme is then removed by centrifugation-dialysis (Vivaspin MWCO 50 kDa, Vivascience, Winkel, Switzerland). The product can be analyzed by LC/MS. Alternatively, an antibody will be naturally free of N-linked glycosylation, for example as a result of an amino acid modification, e.g. at residues 297, 298 and/or 299 (EU numbering). For conjugation onto the acceptor glutamines within the CH3 domain (inculding on a TGase recognition tag fused to a CH3 domain) antibodies need not be free of (may comprise) N-linked glycosylation.

An acceptor glutamine can also advantageously be introduced at residue 297 (EU numbering) of the heavy chain, e.g. by substituting the asparagine naturally present in IgG antibodies by a glutamine (a N297Q variant). An antibody having a N297Q substitution and an acceptor glutamine naturally present at residue 295 will have two acceptor glutamines per heavy chain; when the antibody is a tetrameric, e.g. full-length, antibody, the antibody will have four acceptor gluamines per antibody.

Once antibody and lysine-based linker substrates are prepared they can be reacted by bringing them into contact with one another in a reaction vessel in the presence of a bacterial transglutaminase (BTG) (see, e.g. EC 2.3.2.13, protein-glutamine-γ-glutamyltransferase). The BTG will capable of causing, under suitable conditions, the formation of a covalent bond between the acceptor glutamine residue of the antibody and the linking reagent (at the primary amine of the linking reagent) In one embodiment, the TGase is from S. mobaraense. In another embodiment, the TGase is a mutant TGase having 1, 2, 3, 4, 5, 10 or more amino acid modifications (e.g. substitutions, insertions, deletions), optioanlly the TGase has at least 80% sequence identity with native TGase, e.g. a TGase from S. mobaraense. A preferred example is recombinant bacterial transglutaminase derived from *streptomyces mobaraensis* (available from Zedira, Darmstadt, Germany).

The TGase-catalyzed reaction can be carried out under mild conditions, from several hours to a day (e.g. overnight). Recombinant BTG (EC 2.3.2.13) from *streptomyces mobaraensis* (Zedira, Darmstadt, Germany) are typically used at a concentration of between 1 and 20 U/mL. The lysine-based linker substrates are reacted with antibody (1 mg/mL) at ligand concentrations between 400 and 600 mol/L, providing a 60 to 90-fold excess of the substrates over the antibody, or optionally at lower excess of substrates, e.g. 1- to 20-fold,or 10-20 fold excess over acceptor glutamines. The reactions are performed in potassium-free phosphate buffered saline (PBS; pH 8) at 37 °C. After 4 h to several days (depending on the antibody and the ligand), steady-state conditions are achieved. Excess ligand and enzyme are then removed using centrifugation-dialysis (Vivaspin MWCO 50 kDa, Vivascience, Winkel, Switzerland). The disclosure of U.S. Application No. 13/725,385 filed on December 21, 2012, and entitled "Enzymatic Conjugation of Polypeptides" is hereby incorporated by reference in its entirety.

An acceptor glutamine present on an antibody (e.g. part of the antibody's primary structure, including for example an antibody fragment with a peptide tag) will, under suitable conditions, be recognized by a TGase and covalently bound to a lysine-based linker (e.g., compound of Formula Ia or Ib). The result is an antibody of Formula IVa (the acceptor glutamine is functionalized with the compound of Formula Ia) or an antibody of Formula II (the acceptor glutamine is functionalized with the compound of Formula Ib). Resulting antibody conjugates can be analyzed using any suitable method. Preferably, the stoichiometry of the conjugated antibodies can be characterized by liquid chromatography mass spectrometry (LC/MS) using a top-down approach in order to assess the number of lysine-based linker and/or where applicable moieties-of-interest conjugated to antibodies, and in particular the homogeneity of the composition. Conjugates can be reduced before LC/MS analysis and light chains and heavy chains are measured separately.

Once a lysine-based linker (e.g., compound of Formula Ib) comprising a reactive moiety R is conjugated to an antibody (e.g., resulting in an antibody of Formula II) the antibody can be reacted with a compound comprising a DNA barcode sequence (e.g., a nucleic acid unit (CL-(P¹-Z-P²))) and a reactive group R' (e.g. a compound of Formula III), thereby forming an antibody- DNA barcode sequence conjugate (e.g. a compound of Formula IVb).

The antibodies resulting from the TGase-mediated conjugation can be characterized as having a functionalized acceptor glutamine or Formulae IVa or IVb. The result of direct conjugation of a linking reagent (e.g. of Formula Ia) to an antibody or antibody fragment, without requirement for a step of reaction involving reactive groups R and R', is an antibody or antibody fragment comprises a functionalized glutamine residue of Formula IVa. The result of multi-step conjugation of a linking reagent (e.g. of Formula Ib) to an antibody or antibody fragment involving reactive groups R and R', is an antibody or antibody fragment comprises a functionalized glutamine residue of Formula IVb. Reference to "Formulas I", "Formula II", "Formula III" or "Formula IV", unless the context clearly indicates otherwise, designates all compounds derived from such Formulas I to IV, including e.g., Formula I includes reference to Ia and/or Ib, Formula IV includes IVa and IVb.

In one embodiment, the antibody is conjugated to the at least one nucleic acid molecule in a stoechiometric manner (i.e. the one skilled in the art knows how many nucleic acid molecules are conjugated to the antibody).

The one skilled in the art can easily verify that after conjugation, the DNAb still efficiently recognizes the antigen of interest.

The present invention also relates to a method for detecting the presence of at least one antigen in a sample comprising the steps consisting of providing at least one DNAb according to the invention that is specific for the antigen, bringing into contact the sample with an amount of said at least one DNAb under conditions effective to allow for binding between the DNAdb and the antigen, washing the sample to remove unbound DNAbs, bringing the sample into contact with the enzyme that is able to cleave the sequence CL, isolating the released DNA barcodes, and sequencing the DNA barcode wherein the presence and the amount of the barcode indicate the presence and the amount of the antigen in a sample.

A "sample" encompasses a variety of sample types. Typically, the sample is a fluid sample comprising a plurality of soluble antigens and/or a plurality of cells bearing a plurality of antigens. Therefore, samples encompass clinical samples, cells in culture, cell supernatants, cell lysates, serum, plasma, biological fluid, and tissue samples. For example the samples can be, for example, urine, blood, plasma, serum, saliva, semen, stool, sputum, cerebral spinal fluid, tears, mucus, and the like. The sample is generally prepared from a subject and can be used for research, analysis or diagnostic purposes. In some particular embodiment, the sample can be examined directly or may be pre-treated to render the antigen more readily detectible (e.g. by lysing the cells).

In one embodiment, the antigens or the cells bearing the antigen are immobilized to a solid support. Typically, the antigen or the cells bearing the antigen is immobilizing with a capture binding agent (such as an aptamer or antibody). For example, a soluble antigen may be captured with an antibody which does not compete with the antibody of the DNAb. The cells bearing the antigen may be immobilized with at least one irrelevant antibody for the antigen; typically a antibody directed against an universal surface marker or preferably with at least one antibody directed a specific surface marker of the cells of interest (i.e. liable to express the antigen of interest at their surface) so that the cells of the sample which are not immobilized can be washed out. The term "solid support" refers to a material having a rigid or semi-rigid surface. Such materials will preferably take the form of small beads, pellets, disks, chips, or wafers, although other forms may be used. Such surfaces include, simply by way of example, surfaces of art-known supports such as beads, plates, cuvettes, filters, titer plates, and the like, that have avidin, streptavidin and/or any art known derivative of these agents linked or coated to the surface(s) of those supports. The supports are generally made of conventional materials, e.g., plastic polymers, cellulose, glass, ceramic, stainless steel alloy, and the like.

In one embodiment, a heterogeneous cell mixture, e.g. a tumor needle biopsy, inflammatory lesion biopsy, synovial fluid, spinal tap, etc., is divided randomly or in a certain order into spatially separated single cells, e.g. into a multiwell plate, microarray, microfluidic device, or slide. Then after the cells may be individually analyzed for expression of antigens of interest. The cells thus analyzed are classified according to the antigen signatures of individual cells. Such classification allows an accurate assessment of the cellular composition of the test sample, which assessment may find use, for example, in determining the identity and number of cancer cells in a tumor; in determining the identity and number of immune-associated cells such as the number and specificity of T cells, dendritic cells, B cells and the like.

In some embodiments, cells in a sample are separated on a microarray. For example, a highly integrated live-cell microarray system may utilize microwells each of which is just large enough to fit a single cell (see Tokimitsu et al. (2007) Cytometry Part A 71k 1003: 1010; and Yamamura et al. (2005) Analytical Chemistry 77:8050; each herein specifically incorporated by reference). Prior enrichment of cells of interest - such as by FACS or other sorting - is optional and in some embodiments, cells from a sample are divided into discrete locations without any prior sorting or enrichment. For example, cells from a sample (e.g., blood sample, biopsy, solid tumor) can be individually isolated into distinct positions. Typically, for solid tissue samples, the samples are mechanically, chemically, and/or enzymatically separated (e.g., by treatment with trypsin or sonication). Cells from a sample can be placed into any cell sorting device (e.g., a micro fluidic cell sorter) such that individual cells are isolated, such as at an addressable position on a planar surface. Planar surfaces can have indentations, barriers or other features ensuring isolation of individual cells. Isolated cells can then be analyzed according to the methods herein. Preferably, cells are separated into distinct positions wherein each position contains 1 or 0 cells.

Cells are optionally sorted, e.g. by flow cytometry, prior to the separation. For example, FACS sorting or size- differential sorting, can be used to increase the initial concentration of the cells of interest by at least 1 ,000, 10,000, 100,000, or more fold, according to one or more markers present on the cell surface. Such cells are optionally sorted according to the presence and/or absence of cell surface markers particularly markers of a population or subpopulation of interest.

Where the cells are isolated into distinct positions for analysis, the cells may be sorted with a microfluidic sorter, by flow cytometry, microscopy, etc. A microfabricated fluorescence-activated cell sorter is described by Fu et al. (1999) Nature Biotechnology 17: 1 109 and Fu et al. (2002) Anal. Chem. 74:2451-2457, each herein specifically incorporated by reference. A sample can be sorted with an integrated microfabricated cell sorter using multilayer soft lithography. This integrated cell sorter may incorporate various microfluidic functionalities, including peristaltic pumps, dampers, switch valves, and input and output wells, to perform cell sorting in a coordinated and automated fashion. The active volume of an actuated valve on this integrated cell sorter can be as small as 1 pL, and the volume of optical interrogation as small as 100 fL. Compared with conventional FACS machines, the microfluidic FACS provides higher sensitivity, no cross-contamination, and lower cost.

Devices for single cell isolation include a microfluidic cell sorter, which isolates live cells from cellular debris and sorts cells from a single cell suspension. Microfluidic devices can be used in combination with fluorescent signals (e.g., labeled antibodies to markers for a target population or subpopulation) from 1, 2, 3, 4, 5 or more different surface markers, and places them in individual bins for subsequent genetic studies. Other upstream steps such as digesting the tumor or cell culture to obtain a cell suspension and staining the cells with fluorescent surface markers may be incorporated in this system. The number of cells to be analyzed depends on the heterogeneity of the sample, and the expected frequency of cells of interest in the sample. Usually at least about 10² cells are analyzed, at least about 10³, at least 5 x 10³, at least about 10⁴, at least about 10⁵, at least about 10⁶, at least about 10⁷, at least about 10⁸, at least about 10⁹, at least about 10¹⁰, at least about 10¹¹, at least about 10¹², at least about 10¹³, at least about 10¹⁴, at least about 10¹⁵, or more cells are analyzed.

The method of the invention is particularly useful for implementing multiplex immuno-detection assays. Indeed the method of the invention allows the detection and quantification of a least 5, 10, 30, 50 or even more antigens in a single test. The DNA barcode in each type of antibody has a sequence that is different and that serves as an identifier for a particular antigen. The method of the invention thus combines the powerful ability of antibodies to specifically recognize antigens with the powerful ability of DNA barcode to constitute a unique reporter. Accordingly, at least 2; 3; 4; 5; 6; 7; 8; 9; 10; 11; 12; 13; 14; 15; 16; 17; 18; 19; 20; 21; 22; 23; 24; 25; 26; 27; 28; 29; 30; 31; 32; 33; 34; 35; 36; 37; 38; 39; 40; 41; 42; 43; 44; 45; 46; 47; 48; 49; or 50 DNAbs are provided ; each of said DNAb is conjugated to a unique barcode.

Typically the DNAbs are brought into contact with the sample in an excess manner (the amount of the DNAbs is significantly superior to the amount of antigens) so as to saturate the antigen with the binding of DNAbs.

Any suitable washing solution that removes unbound DNAbs after complex formation may be used. A representative example includes, without limitation, PBS (phosphate buffer solution).

Typically, the enzyme that is able to cleave the sequence CL (e.g. endonuclease) is added in an excess manner.

When several DNAbs are used, a single enzyme may be used (i.e. the DNAbs have the same CL sequences) or different enzymes may be used for releasing the DNA barcodes in the solution (i.e. the DNAbs have different CL sequences).

Selective cleavage by the enzyme (e.g. restriction endonuclease) may be achieved naturally or under predetermined conditions. Typically, the effects of metal ions, pH and temperature on restriction enzyme cleavage of the DNA can be tested to define the optimal conditions. Buffers that include any of metal ions such as Mg²⁺, Mn²⁺, Ca²⁺, Co²⁺, Ni²⁺ etc., pH or temperature can be tested. The skilled man in the art can easily select the appropriate conditions for the enzyme reaction.

The released barcodes may be isolated by any well known method in the art. For example, when the sample comprise a plurality of cells bearing the antigen of interest, a centrifugation may be used to eliminate the cells (and the DNAb binding the cells) in an appropriate solution (e.g. buffer) for recovering the released DNA barcodes.

Antigen quantification and detection occurs indirectly by ascertaining for the presence of the DNA barcode by any suitable means. In practicing this invention, amplification by the PCR method is preferred. PCR amplification (herein also referred to as RPCR) of the DNA barcode allows one to detect the antigen at nanomolar level. Once the DNA barcode is released, it is thus amplified by PCR amplification using a couple of primers that hybridize to P1 and P2 respectively. The amplification step increases the sensitivity of the assay, allowing for detection of small amounts of DNA barcodes. Accordingly the method of the invention further comprises a step consisting of amplifying the released DNA barcodes with a couple of primers specific for sequences P1 and P2 respectively.

Typically, the amplification takes place under standard hybrization conditions and stringency. The term "standard hybridization conditions" refers to a Tm of 55°C, and utilizes conditions as set forth above. In one embodiment, the Tm is 60°C. In a more preferred embodiment, the Tm is 65°C. In a specific embodiment, "high stringency" refers to hybridization and/or washing conditions at 68°C in 0.2 X SSC, at 42°C in 50 % formamide, 4 X SSC, or under conditions that afford levels of hybridization equivalent to those observed under either of these two conditions. The conditions of temperature and ionic strength determine the "stringency" of the hybridization. Typically appropriate stringency for hybridizing nucleic acids depends on the length of the nucleic acids and the degree of complementation, variables well known in the art. The greater the degree of similarity or homology between two nucleotide sequences, the greater the value of Tm for hybrids of nucleic acids having those sequences. The relative stability (corresponding to higher Tm) of nucleic acid hybridizations decreases in the following order: RNA:RNA, DNA:RNA, DNA:DNA. For hybrids of greater than 100 nucleotides in length, equations for calculating Tm have been derived (see Sambrook et al., 1989, 9.50-9.51). For hybridization with shorter nucleic acids, i.e., oligonucleotides, the position of mismatches becomes more important, and the length of the oligonucleotide determines its specificity (see Sambrook et al., 1989 I1.7-11.8). A minimum length for a hybridizable nucleic acid is at least about 10 nucleotides, preferably at least about 15 nucleotides, and more preferably the length is at least about 20 nucleotides.

When several DNAbs are used, a single couple of primers may be used for amplifying the released barcodes (i.e. the DNAbs have the same P1 and P2 sequences) or different couple of primers may be used for amplifying the released barcodes (i.e. the DNAbs have different P1 and P2 sequences).

Typically the DNA barcode is detected ("read") by any technique well known in the art, but preferably sequencing is used. The one skilled in the art is familiar with several methods for sequencing of polynucleotides. These include, but are not limited to, Sanger sequencing (also referred to as dideoxy sequencing) and various sequencing-by-synthesis (SBS) methods as reviewed by Metzger (Metzger ML 2005, Genome Research 1767), sequencing by hybridization, by ligation (for example, WO 2005/021786 ), by degradation (for example, U.S. Patent Nos. 5,622,824 and 6,140,053 ), nanopore sequencing. Preferably in a multiplex assay deep high throughput sequencing is preferred. The term "deep high throughput sequencing" refers to a method of sequencing a plurality of nucleic acids in parallel. See e.g., Bentley et al, Nature 2008, 456:53-59. The leading commercially available platforms produced by Roche/454 (Margulies et al., 2005a), Illumina/Solexa (Bentley et al., 2008), Life/APG (SOLiD) (McKernan et al., 2009), Ion Torrent PGM/Proton () and Pacific Biosciences (Eid et al., 2009) may be used for deep HT sequencing. For example, in the 454 method, the DNA to be sequenced is either fractionated and supplied with adaptors or segments of DNA can be PCR-amplified using primers containing the adaptors. The adaptors are nucleotide 25-mers required for binding to the DNA Capture Beads and for annealing the emulsion PCR Amplification Primers and the Sequencing Primer. The DNA fragments are made single stranded and are attached to DNA capture beads in a manner that allows only one DNA fragment to be attached to one bead. Next, the DNA containing beads are emulsified in a water- in-oil mixture resulting in microreactors containing just one bead. Within the microreactor, the fragment is PCR-amplified, resulting in a copy number of several million per bead. After PCR, the emulsion is broken and the beads are loaded onto a pico titer plate. Each well of the pico-titer plate can contain only one bead. Sequencing enzymes are added to the wells and nucleotides are flowed across the wells in a fixed order. The incorporation of a nucleotide results in the release of a pyrophosphate, which catalyzes a reaction leading to a chemiluminescent signal. This signal is recorded by a CCD camera and a software is used to translate the signals into a DNA sequence. In the lllumina method (Bentley (2008)), single stranded, adaptor-supplied fragments are attached to an optically transparent surface and subjected to "bridge amplification". This procedure results in several million clusters, each containing copies of a unique DNA fragment. DNA polymerase, primers and four labeled reversible terminator nucleotides are added and the surface is imaged by laser fluorescence to determine the location and nature of the labels. Protecting groups are then removed and the process is repeated for several cycles. The SOLiD process (Shendure (2005)) is similar to 454 sequencing, DNA fragments are amplified on the surface of beads. Sequencing involves cycles of ligation and detection of labeled probes. The Ion Torrent sequencers uses a post-light, semiconductor-based technology that dramatically reduce the sequencing cost. Several other techniques for high-throughput sequencing are currently being developed. Examples of such are The Helicos system (Harris (2008)), Complete Genomics (Drmanac (2010)) and Pacific Biosciences (Lundquist (2008)). For example, a PACIFIC BIOSCIENCES' SMRT™ (Single Molecule Real Time sequencing) sequencing platform may be used. Said technology uses a real time sequencing by synthesis and can produce reads of up to 1000 by in length as a result of not being limited by reversible terminators. Raw read throughput that is equivalent to one-fold coverage of a diploid human genome can be produced per day using this technology. As this is an extremely rapidly developing technical field, the applicability to the present invention of high throughput sequencing methods will be obvious to a person skilled in the art.

It is important to note that the quantity of the detected DNA barodes shall be reported to the number of DNA barcodes functionalized on the antibody. For example; when the antibody have exactly four conjugated DNA barcodes, the quantity of the DNA barcodes shall be divided by 4 to exactly determine the number of antigens.

The method of the invention may find various applications in very different fields such as biological research, forensic science, and clinical diagnostics. For example analyzing protein signatures at single-cell resolution would aid studies of the role of cellular heterogeneity in disease progression, stem cell differentiation, response to drugs, and other cellular signaling processes. Clinically, accurate molecular profiling and proteomic analysis of rare cells (e.g., circulating tumor cells) holds considerable promise for early disease detection and monitoring of treatment response. Thus, the sensitive, reliable, and multiplexable antigen detection technology of the invention may answer to a great demand.

The invention relates to a kit comprising at least one DNAb of the invention. In some embodiments, the kit may comprise the means for the performing the method of the invention and thus may comprise in addition to the DNAb, at least one couple of primer capable to hybridize with sequences P1 and P2 respectively and/or the enzyme that is able to cleave the CL sequence (e.g. a endonuclease) and/or all means for immobilizing the antigen (i.e. a solid support coated with a capture antibody) and/or all means for performing the DNA barcodes releasing and amplification (e.g. buffers) and/or all means for perfoming the sequencing of the DNA barcodes.

The invention will be further illustrated by the following figures and examples. However, these examples and figures should not be interpreted in any way as limiting the scope of the present invention.

### FIGURES:

**Figure 1** shows the preparation of an exemplary linking reagent and its conjugation with a protein, where: R is a thiol (sulfhydryl) reactive group that is ultimately generated from the S-acetyl protected thiol, SC(O)CH₃; q is 1; L is the two carbon comprising framework C(O)CH₂; X is NH; (C)ₙ is (CH₂)₅; and G is transformed from the (H₃C)₃COC(0) protecting group to H and ultimately to the amide upon conjugation of a glutamine residue of a protein.
**Figure 2** illustrates the preparation of various exemplary linking reagents with a single S-acetyl protected thiol reactive group that can be prepared from an N-succinimidyl-S-acetylthioester reagent.
**Figure 3** illustrates the preparation of an exemplary linking reagent and its conjugation with a protein, where: R is an azide reactive group; r is q is 1; L is the two carbon comprising framework C(O)CH₂; X is NH; (C)ₙ is (CH₂)₅; and G is transformed from the (H₃C)₃COC(0) protecting group to H and ultimately to the amide upon conjugation of a glutamine residue of a protein.
**Figure 4** illustrates the preparation of various exemplary linking reagents with a single azide reactive group that can be prepared from an N-succinimidyl-azide reagent.
**Figure 5** depicts the preparation of an exemplary linking reagent and its conjugation with a protein, where: R is an alkyne reactive group; q is 1; L is a one carbon comprising framework CH₂; X is NH; (C)ₙ is (CH₂)₄CH(CO₂H); and G is transformed from the (H₃C)₃COC(0) protecting group to H and ultimately to the amide upon conjugation of a glutamine residue of a protein.
**Figure 6** shows the preparation of an exemplary linking reagent and its conjugation with a protein, where: R is a norbornene reactive group; q is 1; L is the one carbon comprising framework C(O); X is NH; (C)ₙ is(CH₂)₄CH(CO₂H); and G is transformed from the (H₃C)₃COC(0) protecting group to H and ultimately to the amide upon conjugation of a glutamine residue of a protein.
**Figure 7** illustrates a typical DNAb format according to the invention.
**Figure 8** illustrates a method used for analysis a population of cells in a sample after cell sorting.

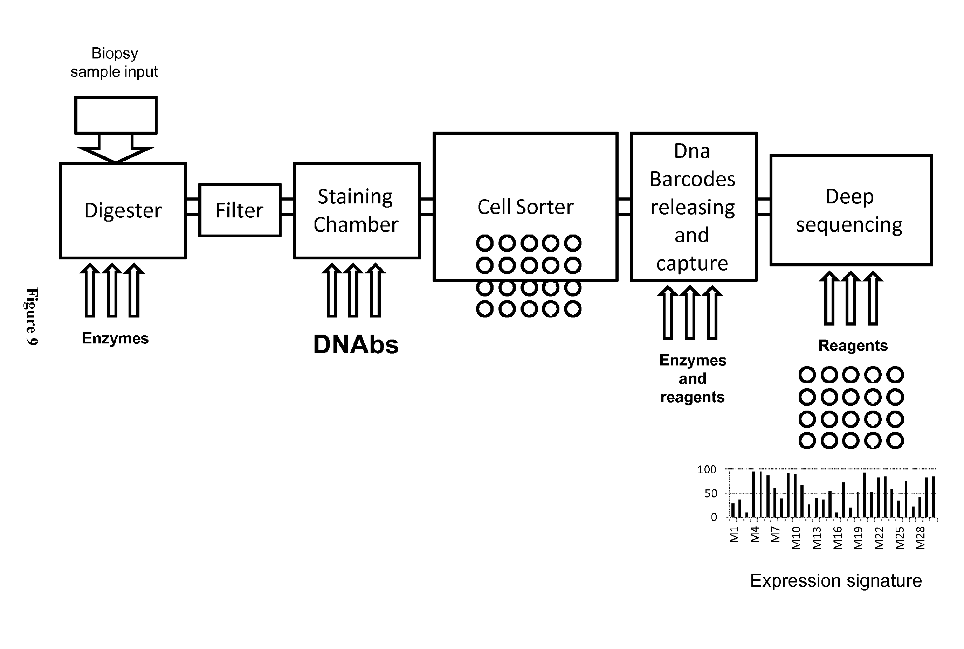

**Figure 9** depicts the workflow of an automated device to identify and quantify cells by the method of the invention.

### EXAMPLES:

### Example 1 : Multiplex assays in population of cells

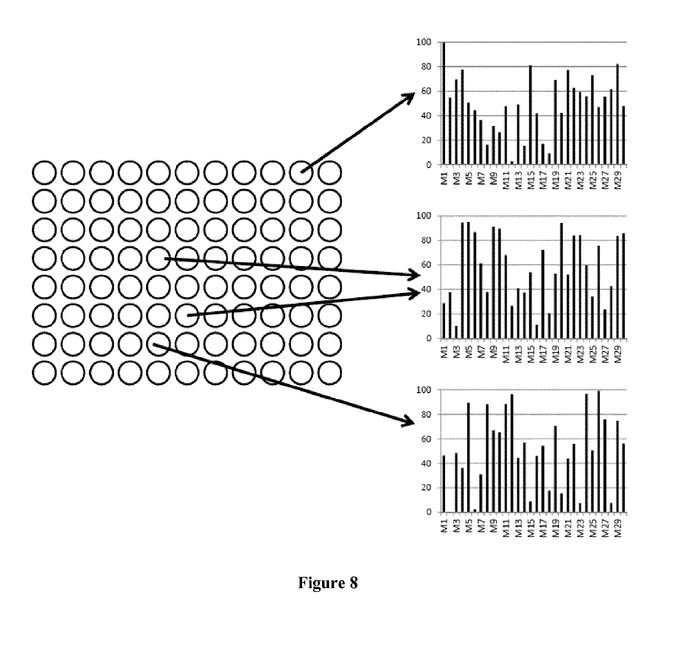

A method that can be used to identify and quantify different cells (e.g. cancer cells) in a heterogeneous population of cells is of particular interest. For example, a reliable method allowing detection and quantification of the cells may be particularly suitable for diagnosing purposes (e.g. for stratifying patients). It is also desirable to optimize the profiling of markers to identify particular cells, such as cancer cells. The method of the invention brings a solution to these technical problems. For example 30 cell surface markers (M1, M2,M3,...,Mn,...,M30) are selected and antibodies specific for the markers are provided. The antibodies are then conjugated to a specific DNA barcode. All the antibodies bear the same enzymatic cleavable sequence (e.g. for EcoRI) and the same sequences P1 and P2 for amplifying the DNA barcode. A sample comprising a population of cells (e.g normal and cancer cells) is provided and is brought into contact with an excess of the antibodies for the marker. The unbound antibodies are then washed out. A single cell sorting is then carried out, by using for example a micro fluidic cell sorter. Cells are thus separated into distinct positions wherein each position contains 1 cell. In every position the released of the barcode is carried out by bringing into contact the cell with an excess amount of the endonuclease (e.g. EcoRI). Optionally application of the DNA barcodes is carried out with the appropriate couple of primer for each position. DeepHT sequencing in then performed for detecting and quantifying the DNA barcodes. An immunoprofiling of the single cells is then generated and a comparison step between every position may be performed. The same profiling corresponding to the same type of cells may be counted and the number may be compared with different samples (e.g. samples corresponding to another patient or sample corresponding to another stage of the disease). How the method is used for analysis a population of cells is illustrated in Figure 8.

### Example 2: Engineering an automated device to identify and quantify cells

An automated device is designed to identify specific cells and calculate their frequency in a sample. This device will make a single cell suspension of cells from the sample, isolate the cell subpopulations and measure the cell content of the sample. Such a fully automated device will eliminate the labor-intensive steps currently needed for flow-cytometry sorting, and will allow a truly hands-off, bed-side diagnostic tool to identify and quantify cells (e.g. cancer cells). Automated operation, effectiveness and low cost associated with microfluidic chip technology will make individualized, rapid diagnosis possible. At the heart of this system is a microfluidic cell sorter. This device isolates live cells from the debris (necrotic cells and other particles), sorts out the cells from the single cell suspension and places them in individual bins for subsequent immunoprofiling analyses. Other upstream steps such as for example digesting the tumor or cell culture to obtain a cell suspension may be incorporated in this system. How the system is used for analysis is illustrated here. Once the biopsy is obtained, the physician will place the sample in the input port of this system. Utilizing a user friendly computer interface, the physician will set the necessary parameters in the sorting and immunoprofiling analysis such as the number and type of surface markers, the number of PCR cycles needed etc, and the machine will perform the rest of the steps without human intervention. A single cell analysis device (SCAD) can be modular (Figure 3) and will perform the following steps in an integrated, fully automated fashion: 1) Digestion of the tissue: The tissue is placed in the input port of the device. Appropriate enzymes are introduced in the device and flowed to perform the digestion of the extracellular matrix in order to obtain a cell suspension. 2) Separation of live cells from the debris: The suspension typically contains live cell with an average size of 10 to 15 micrometers, and debris material with an average size around 5 micrometers. The amount of dead material is sometimes relatively high compared to live cells, therefore it is critical to filter out the dead material for efficient isolation of cells. We accomplish this by flowing the digested tissue suspension through a micro fluidic "metamaterial," which allows splitting the fluidic flow according to the size of the particles. 3) Staining: The filtered single cell suspension is stained the using appropriate DNAbs specific for surface markers in a different compartment of the microfluidic device. 4) Sorting: The stained single-cell suspension is flowed into the next compartment of the microfluidic device to sort out the cancer cells from the rest of the cells. Flow based microfluidic cell sorter is typically used. The cells are flowed into the sorter array and are captured in appropriates baskets. After all the baskets are filled with a cell, the microfluidic valves are closed 5) Dna Barcodes releasing and capture: the DNA barcodes are then released with appropriate endonuclease, is optionally amplified transferred to a module for deep HT sequencing. An expression signatures in then generated for every single cell and may be compared between control samples.

### REFERENCES:

Throughout this application, various references describe the state of the art to which this invention pertains. The disclosures of these references are hereby incorporated by reference into the present disclosure.

## Claims

1. An antibody conjugated to at least one nucleic acid molecule comprising an enzymatic cleavable sequence (CL) and a DNA barcode sequence N₁N₂N₃N₄Nₙ wherein N represents a nucleotide and n represents an integer number superior to 4.

2. The antibody according to claim 1 which is selected from the group consisting of monoclonal antibodies, antibody fragments that comprise an antigen binding domain such as Fab', Fab, F(ab')2, single domain antibodies (DABs or VHH), TandAbs dimer, Fv, scFv (single chain Fv), dsFv, ds-scFv, Fd, linear antibodies, minibodies, diabodies, bispecific antibody fragments, bibody, tribody (scFv-Fab fusions, bispecific or trispecific, respectively); sc-diabody; kappa(lamda) bodies (scFv-CL fusions); DVD-Ig (dual variable domain antibody, bispecific format); SIP (small immunoprotein, a kind of minibody); SMIP ("small modular immunopharmaceutical" scFv-Fc dimer; DART (ds-stabilized diabody "Dual Affmity ReTargeting"); small antibody mimetics comprising one or more CDRs.

3. The antibody according to claim 2 which is a monoclonal antibody.

4. The antibody of claim 1, wherein the nucleic acid molecule is bound, via a linking reagent, to an acceptor glutamine residue present in the antibody.

5. An antibody comprising an acceptor glutamine functionalized, via a linking reagent, with a nucleic acid molecule comprising an enzymatic cleavable sequence (CL) and a DNA barcode sequence N₁N₂N₃N₄Nₙ wherein N represents a nucleotide and n represents an integer number superior to 4.

6. The antibody of claims 1-5, wherein the linker is covalently bound to the side chain of the acceptor glutamine within the primary sequence of the antibody.

7. The antibody of claims 1-6, wherein the linker is covalently bound to the side chain of an amino acid within the CH2 domain.

8. The antibody of claims 1-7, wherein the acceptor glutamine is within an enzymatic recognition tag fused to the C-terminus of the CH3 domain.

9. The antibody of claims 1-8, wherein the acceptor glutamine is within an enzymatic recognition tag fused to the C-terminus of a Cκ domain.

10. The antibody of claims 9, wherein the acceptor glutamine is within the primary sequence of CH2 at the position that corresponds to the residue Q295 according to Kabat EU numbering.

11. The antibody of claims 1-10, wherein the CH2 domain is free of N-linked glycosylation.

12. The antibody of claims 1-10, wherein the CH2 domain comprises N-linked glycosylation.

13. The antibody of any one of the above claims, wherein the antibody comprises a functionalized acceptor glutamine has Formula IVa:
(Q)-NH-(C)-(CL-(P¹-Z-P²))_{q} Formula IVa
wherein:
- Q is a glutamine residue present in the antibody;
- C is a bond or a linking moiety
- q is an integer selected from among 1, 2, 3 or 4;
- CL is an enzymatic cleavable nucleic acid sequence;
- P¹ is independently absent, or a nucleic acid having at least 10 nucleotides;
- Z is a DNA barcode sequence N₁N₂N₃N₄Nₙ wherein N represents a nucleotide and n represents an integer number superior to 4; and
- - P² is independently absent or a nucleic acid having at least 10 nucleotides.

14. The antibody of any one of the above claims, wherein the antibody comprises a functionalized acceptor glutamine residue having Formula IVb,
(Q)-NH-(C)-((M))_{q} Formula IVb
wherein:
- Q is a glutamine residue present in the antibody;
- C is a bond or a linking moiety
- q is an integer selected from among 1, 2, 3 or 4;
- M is independently: (RR') - C' - (CL'-(P¹-Z-P²)_{q'},
- wherein
- (RR') is an addition product between R and a complementary reactive moiety R';
- C' is a bond or a linking moiety;
- CL' is an enzymatic cleavable nucleic acid sequence;
- P¹ is independently absent, or a nucleic acid having at least 10 nucleotides;
- Z is a DNA barcode sequence N₁N₂N₃N₄Nₙ wherein N represents a nucleotide and n represents an integer number superior to 4;
- P² is independently absent or a nucleic acid having at least 10 nucleotides; and
- q' is an integer selected from among 1, 2, 3 or 4.

15. A linking reagent, or an antibody-conjugated linking reagent having the general Formula Ia:
G-NH-C-(CL-(P¹-Z-P²))_{q} Formula Ia
wherein:
- G is a H, amine protecting group, or upon conjugation, an antibody or antibody fragment attached via an amide bond;
- C is a bond or a linking moiety;
- q is an integer selected from among 1, 2, 3 or 4;
- CL' is independently absent, or an enzymatic cleavable nucleic acid sequence;
- P¹ is independently absent, or a nucleic acid having at least 10 nucleotides;
- Z is a DNA barcode sequence N₁N₂N₃N₄Nₙ wherein N represents a nucleotide and n represents an integer number superior to 4; and
- P² is independently absent, or a nucleic acid having at least 10 nucleotides.

16. The antibody or linking reagent of claims 13-15, wherein C is a substituted or unsubstituted alkyl or heteroalkyl chain, optionally wherein any carbon of the chain is substituted with an alkoxy, hydroxyl, alkylcarbonyloxy, alkyl-S-, thiol, alkyl-C(O)S-, amine, alkylamine, amide, or alkylamide.

17. The antibody or linking reagent of claims 13-15, wherein C comprises a unit: -(C)ₙ-X-L-,
wherein:
(C)ₙ is a substituted or unsubstituted alkyl or heteroalkyl chain, optionally wherein any carbon of the chain is substituted with an alkoxy, hydroxyl, alkylcarbonyloxy, alkyl-S-, thiol, alkyl-C(O)S-, amine, alkylamine, amide, or alkylamide;
n is an integer selected from among the range of 2 to 20;
X is NH, O, S, absent, or a bond;
L is independently absent, a bond or a continuation of a bond, or a carbon comprising framework of 5 to 200 atoms substituted at one or more atoms, optionally, wherein the carbon comprising framework comprises a linear framework of 5 to 30 carbon atoms optionally substituted at one or more atoms, optionally wherein the carbon comprising framework is a linear hydrocarbon, a symmetrically or asymmetrically branched hydrocarbon, monosaccharide, disaccharide, linear or branched oligosaccharide (asymmetrically branched or symmetrically branched), other natural linear or branched oligomers (asymmetrically branched or symmetrically branched), or a dimer, trimer, or higher oligomer (linear, asymmetrically branched or symmetrically branched) resulting from any chain-growth or step-growth polymerization process.

18. The antibody of claims 14 or 16-17 wherein RR' comprises an addition product of a thio-maleimide (or haloacetamide) addition, for example, a *N,S*-disubstituted-3-thio-pyrrolidine-2,5-dione; Staudinger ligation, for example, a *N*,3- or *N*,4-substitued-5-dipenylphosphinoxide-benzoic amide; Huisgen 1,3-cycloaddition (click reaction), for example, a *N*,*S*-disubstituted-3-thio-pyrrolidine-2,5-dione, 1,4-disubstituted-1,2,3-triazole, 3,5-disubstituted-isooxazole, or 3,5-disubstituted-tetrazole; Diels-Alder cycloaddition adduct, for example the 2,4-cycloaddition product between an *O* or *N*-substituted-5-norbornene-2-carboxylic ester or amide, *N*-substituted-5-norbornene-2,3-dicarboxylic imide, *O* or *N-*substituted-7-oxonorbomene-5-carboxylic ester or amide, or *N-*substituted-7-oxonorbornene-5,6-dicarboxylic imide and a 9-substituted anthracene or 3-substituted 1,2,4,5-tetrazine; or any high yield selective amidation or imidization reaction.

19. The antibody of claims 14 or 16-17 wherein RR' comprises a result of the reaction of an alkyne with an azide.

20. The antibody of claim 19 wherein RR' comprises a structure:

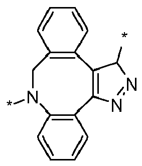

21. A compound having the structure of Formula III, below,
R' - L - (CL-(P¹-Z-P²))_{q} Formula III
wherein:
R' is a reactive group;
L is independently absent, or a carbon comprising framework of 1 to 200 atoms substituted at one or more atoms, optionally, wherein the carbon comprising framework comprises a linear framework of 5 to 30 atoms optionally substituted at one or more atoms, optionally wherein the carbon comprising framework is a linear hydrocarbon, a symmetrically or asymmetrically branched hydrocarbon, monosaccharide, disaccharide, linear or branched oligosaccharide (asymmetrically branched or symmetrically branched), other natural linear or branched oligomers (asymmetrically branched or symmetrically branched), or a dimer, trimer, or higher oligomer (linear, asymmetrically branched or symmetrically branched) resulting from any chain-growth or step-growth polymerization process;
CL is independently absent, or an enzymatic cleavable nucleic acid sequence;
P¹ is independently absent, or a nucleic acid having at least 10 nucleotides;
Z is a DNA barcode sequence N₁N₂N₃N₄Nₙ wherein N represents a nucleotide and n represents an integer number superior to 4;
P² is independently absent or a nucleic acid having at least 10 nucleotides; and
q is an integer selected from among 1, 2, 3 or 4.

22. The antibody of any one of the above claims which is specific for an immune cell regulatrory molecule, a cancer antigen, a viral antigen, a bacterial antigen, or a CD molecule.

23. The antibody of any one of the above claims wherein the enzymatic cleavale sequence CL comprises a restriction enzyme cutting site.

24. The antibody according to claim 23 wherein the enzymatic cleavable sequence is selected from Table B.

25. The antibody of any one of the above claims wherein the DNA barcode comprises , 5, 6, 7, 8, 9, 10, 11, 12, 13, 14 or 15 nucleotides.

26. The antibody of claim 25 wherein the sequence P1 comprises between 10 and 15 nucleotides.

27. The antibody of any one of the above claims wherein the nucleic acid molecule further comprises a sequence P1 of at least 10 nucleotides.

28. The antibody of claims 26 and 24 wherein the nucleic acid molecule has the general formula of: 3'- CL-P1-N₁N₂N₃N₄Nₙ-5' ..

29. The antibody of any one of the above claims wherein the nucleic acod molecule further comprises a second sequence P2 having at least 10 nucleotides.

30. The antibody of claim 29 wherein the sequence P2 comprises between 10 and 15 nucleotides.

31. The antibody of claim 30 wherein the nucleic acid molecule has the general formula of: 3'- CL-P1-N1N2N3N4Nn-P2- 5' wherein
- CL represents an enzymatic cleavable sequence
- P1 represents a first sequence which is able to hybridize to a first forward primer
- N1N2N3N4Nn represents a DNA barcode wherein N represents a nucleotide and n an integer number superior to 4
- P2 represents a second sequence which is able to hybridize to a second reverse primer

32. The antibody of any one of the above claims wherein the nucleic acid molecule comprises the sequence GAATTCCCATCTCATCCCTGCGTGTCTCCGACTCAGNNNNNNNNNATCACCGACT GCCCATAGAGAGG.

33. A method for conjugating a DNA barcode sequence to an antibody, comprising the steps of:
a) providing an antibody having at least one acceptor glutamine residue;
b) reacting said antibody with a linking reagent comprising a primary amine and a compound comprising (i) a nucleic acid molecule comprising an enzymatic cleavable sequence (CL) and a DNA barcode sequence N₁N₂N₃N₄Nₙ wherein N represents a nucleotide and n represents an integer number superior to 4, in the presence of a TGase, under conditions sufficient to obtain an antibody comprising an acceptor glutamine linked to said DNA barcode sequence via a linking reagent.

34. A method for conjugating a DNA barcode sequence to an antibody, comprising the steps of:
a) providing an antibody having at least one acceptor glutamine residue;
b) reacting said antibody with a linking reagent comprising a primary amine and a reactive group (R), in the presence of a TGase, under conditions sufficient to obtain an antibody comprising an acceptor glutamine linked (covalently) to a reactive group (R) via the linking reagent; and
c) reacting the antibody obtained in step b) with a compound comprising (i) a nucleic acid molecule comprising an enzymatic cleavable sequence (CL) and a DNA barcode sequence N₁N₂N₃N₄Nₙ wherein N represents a nucleotide and n represents an integer number superior to 4, and (ii) a reactive group (R') capable of reacting with reactive group R, under conditions sufficient to obtain an antibody comprising an acceptor glutamine linked to said DNA barcode sequence via a linking reagent.

35. The method of claim 33-34, wherein the linking reagent of step (a) is covalently bound to the side chain of the acceptor glutamine within the primary sequence of the antibody.

36. The method of claims 33-34, wherein the linking reagent of step (a) is covalently bound to the side chain of an amino acid within the CH2 domain.

37. The method of claims 33-34, wherein the acceptor glutamine is within an enzymatic recognition tag fused to the C-terminus of the CH3 domain.

38. The method of claims 33-34, wherein the acceptor glutamine is within an enzymatic recognition tag fused to the C-terminus of a Cκ domain.

39. The method of claim 40, wherein the acceptor glutamine is within the primary sequence of CH2 at the position that corresponds to the residue Q295 according to Kabat EU numbering.

40. The method of claims 33-39, wherein the CH2 domain is free of N-linked glycosylation.

41. The method of claims 33-39, wherein the CH2 domain comprises N-linked glycosylation.

42. The method of claim 33-39, wherein the linking reagent comprising a reactive group has the general Formula Ib:
G-NH-(C)-(R)q Formula Ib
wherein:
G is a H, amine protecting group, or upon conjugation, an antibody or antibody fragment attached via an amide bond;
C is a bond or a linking moiety;
q is an integer selected from among 1, 2, 3 or 4; and
R is a reactive moiety.

43. The antibody or linking reagent of claim 32, wherein C comprises a unit: -(C)ₙ-X-L-,
wherein:
(C)ₙ is a substituted or unsubstituted alkyl or heteroalkyl chain, optionally wherein any carbon of the chain is substituted with an alkoxy, hydroxyl, alkylcarbonyloxy, alkyl-S-, thiol, alkyl-C(O)S-, amine, alkylamine, amide, or alkylamide;
n is an integer selected from among the range of 2 to 20;
X is NH, O, S, absent, or a bond;
L is independently absent, a bond or a continuation of a bond, or a carbon comprising framework of 5 to 200 atoms substituted at one or more atoms, optionally, wherein the carbon comprising framework comprises a linear framework of 5 to 30 carbon atoms optionally substituted at one or more atoms, optionally wherein the carbon comprising framework is a linear hydrocarbon, a symmetrically or asymmetrically branched hydrocarbon, monosaccharide, disaccharide, linear or branched oligosaccharide (asymmetrically branched or symmetrically branched), other natural linear or branched oligomers (asymmetrically branched or symmetrically branched), or a dimer, trimer, or higher oligomer (linear, asymmetrically branched or symmetrically branched) resulting from any chain-growth or step-growth polymerization process.

44. The method of claim 33-43, wherein the compound comprising a DNA barcode sequence and a reactive group (R') has the general Formula III:
R' - L - (CL-(P¹-Z-P²))_{q} Formula III
wherein:
R' is a reactive group;
L is independently absent, or a carbon comprising framework of 1 to 200 atoms substituted at one or more atoms, optionally, wherein the carbon comprising framework comprises a linear framework of 5 to 30 atoms optionally substituted at one or more atoms, optionally wherein the carbon comprising framework is a linear hydrocarbon, a symmetrically or asymmetrically branched hydrocarbon, monosaccharide, disaccharide, linear or branched oligosaccharide (asymmetrically branched or symmetrically branched), other natural linear or branched oligomers (asymmetrically branched or symmetrically branched), or a dimer, trimer, or higher oligomer (linear, asymmetrically branched or symmetrically branched) resulting from any chain-growth or step-growth polymerization process;
CL is independently absent, or an enzymatic cleavable nucleic acid sequence;
P¹ is independently absent, or a nucleic acid having at least 10 nucleotides;
Z is a DNA barcode sequence N₁N₂N₃N₄Nₙ wherein N represents a nucleotide and n represents an integer number superior to 4;
P² is independently absent or a nucleic acid having at least 10 nucleotides; and
q' is an integer selected from among 1, 2, 3 or 4.

45. An antibody obtained according to a method of claims 33-44.

46. A method for detecting the presence of at least one antigen in a sample comprising the steps consisting of providing at least one antibody of any one of claims 1-32 or 45 that is specific for the antigen, bringing into contact the sample with an amount of said at least one antibody under conditions effective to allow for binding between the antibody and the antigen, washing the sample to remove unbound antibodies, bringing the sample into contact with the enzyme that is able to cleave the sequence CL, isolating the released DNA barcodes, and sequencing the DNA barcodes wherein the presence and the amount of the barcode indicate the presence and the amount of the antigen in a sample.

47. The method of claim 46 wherein the antigen is borne by a cell.

48. The method of any one of claims 46-47 wherein the sample consists of a heterogeneous cell mixture.

49. The method of claim 48 wherein the heterogeneous cell mixture, is divided randomly or in a certain order into spatially separated single cells into a multiwell plate, a microarray, micro fluidic device, or a slide.

50. The method of claim 49 wherein the microarray comprises microwells each of which is just large enough to fit a single cell.

51. The method of any one of claims 46-50 wherein the cells are previously sorted with a microfluidic sorter, by flow cytometry, or microscopy.

52. The method of any one of claims 46-51 which allows the detection and quantification of a least 5, 10, 30, 50 or even more antigens in a single test.

53. The method of any one of claims 46-52 wherein at least 2; 3; 4; 5; 6; 7; 8; 9; 10; 11; 12; 13; 14; 15; 16; 17; 18; 19; 20; 21; 22; 23; 24; 25; 26; 27; 28; 29; 30; 31; 32; 33; 34; 35; 36; 37; 38; 39; 40; 41; 42; 43; 44; 45; 46; 47; 48; 49; or 50 antibodies are provided, each of said antibody being conjugated to a unique DNA barcode.

54. The method of any one of claims 46-53 wherein the releases DNA barcodes are sequence by deep high throughput sequencing.

55. The method of claim 54 wherein the DNA barcode is amplified before sequencing.

56. A kit comprising an antibody of any one of claims 1-32 or 45, a restriction endonuclease and optionally a couple of primer.
